# EUROPEAN PATENT SPECIFICATION

(11) **EP 1 709 046 B1**
(45) Date of publication and mention of the grant of the patent: **27.07.2011**
(21) Application number: 05702029.9
(22) Date of filing: 27.01.2005
(51) Int. Cl.: C07D 471/04, A61P 29/00, A61K 31/437

(54) **P38 KINASE INHIBITORS**
P38-KINASE-INHIBITOREN
INHIBITEURS DE LA KINASE P38

(30) Priority: 30.01.2004 GB 0402137
(43) Date of publication of application: 11.10.2006
(73) Proprietor: GlaxoSmithKline LLC, Philadelphia, PA 19102 (US)
(72) Inventor: BARKER, Michael David GlaxoSmithKline, Stevenage, Hertfordshire SG1 2NY (GB); HAMBLIN, Julie Nicole GlaxoSmithKline, Stevenage Hertfordshire SG1 2NY (GB); JONES, Katherine Louise GlaxoSmithKline, Stevenage Hertfordshire SG1 2NY (GB); PATEL, Vipulkumar Kantibhai GlaxoSmithKline, Stevenage Hertfordshire SG1 2NY (GB); SWANSON, Stephen GlaxoSmithKline, Stevenage Hertfordshire SG1 2NY (GB); WALKER, Ann Louise GlaxoSmithKline, Stevenage Hertfordshire SG1 2NY (GB)
(74) Representative: Shore, Andrew David
(86) International application number: PCT/GB2005/000274
(87) International publication number: WO 2005/073232

(56) References cited:
- WO-A-03/033502
- WO-A-2004/010995
- BOEHM J C ET AL: "New inhibitors of p38 kinase" EXPERT OPINION ON THERAPEUTIC PATENTS, ASHLEY PUBLICATIONS, GB, vol. 10, no. 1, 2000, pages 25-37, XP002259248 ISSN: 1354-3776

## Description

This invention relates to novel compounds and their use as pharmaceuticals, particularly as p38 kinase inhibitors, for the treatment of conditions or disease states mediated by p38 kinase activity or mediated by cytokines produced by the activity of p38 kinase.

Boehm at al (Expert Opinion of Therapeutic Patents, 2000; vol. 10, no. 1) describes new inhibitors of p38 kinase. Patent application WO03/033502 discloses a series of bicyclic oxopyridine and oxopyrimidine derivatives that are inhibitors of p38 kinase and are said to be useful in the treatment of a number of disorders, such as rheumatoid arthritis.

We have now found a group of novel compounds that are inhibitors of p38 kinase.

According to the invention there is provided a compound of formula (I): 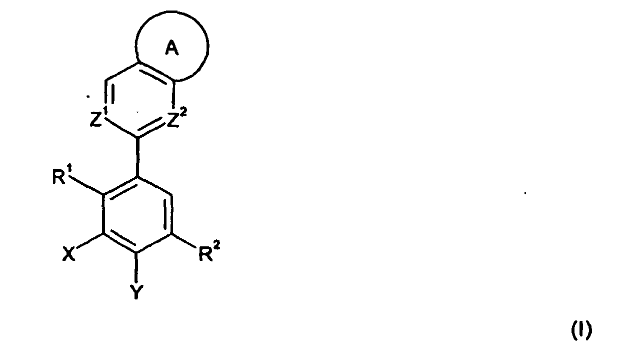 wherein
A is a fused 5-membered heteroaryl ring containing one or two heteroatoms independently selected from oxygen and nitrogen which heteroaryl ring is optionally substituted by up to two substituents independently selected from C₁₋₆alkyl, -(CH₂)ₖ-C₃₋₇cycloalkyl, halogen, cyano, trifluoromethyl, -(CH₂)ₖOR³, -(CH₂)ₖCO₂R³, -(CH₂)ₖNR³R⁴, - (CH₂)ₖCONR³R⁴, -(CH₂)ₖNHCOR³, -(CH₂)ₖSO₂NR³R⁴, -(CH₂)ₖNHSO₂R³, - (CH₂)ₖSO₂(CH₂)ₘR⁵, a 5- or 6-membered heterocyclyl ring containing nitrogen optionally substituted by C₁₋₂alkyl or -(CH₂)ₖCO₂R³, and a 5-membered heteroaryl ring optionally substituted by C₁₋₂alkyl;
A is a fused 5-membered heteroaryl ring containing one or two heteroatoms independently selected from oxygen and nitrogen which heteroaryl ring is substituted by - BR⁶, and
   the heteroaryl ring is optionally further substituted by one substituent selected from -OR⁷, halogen, trifluoromethyl, -CN, -CO₂R⁷ and C₁₋₆alkyl optionally substituted by hydroxy;
A is a fused 5-membered heteroaryl ring containing one or two heteroatoms independently selected from oxygen and nitrogen which heteroaryl ring is substituted by - (CH₂)ₙheterocyclyl wherein the heterocyclyl is a 5- or 6-membered heterocyclic ring containing one or two heteroatoms independently selected from oxygen, sulfur and nitrogen optionally substituted by up to two substituents independently selected from oxo, C₁₋₆alkyl, - (CH₂)ₚphenyl, -OR⁷, -(CH₂)ₚCO₂R⁷, -NR⁷R⁸ and -CONR⁷R⁸, and
   the heteroaryl ring is optionally further substituted by one substituent selected from -OR⁷, halogen, trifluoromethyl, -CN, -CO₂R⁷ and C₁₋₆alkyl optionally substituted by hydroxy; or
A is a fused 5-membered heteroaryl ring containing one or two heteroatoms independently selected from oxygen and nitrogen which heteroaryl ring is substituted by - (CH₂)_{q}aryl or -(CH₂)_{q}heteroaryl wherein the aryl or heteroaryl is optionally substituted by one or more substituents independently selected from oxo, C₁₋₆alkyl, halogen, cyano, trifluoromethyl, -OR⁹, -(CH₂)ᵣCO₂R¹⁰, -NR⁹R¹⁰, -(CH₂)ᵣCONR⁹R¹⁰, -NHCOR⁹, - SO₂NR⁹R¹⁰, -NHSO₂R⁹ and -S(O)_{S}R⁹, and
   the heteroaryl ring is optionally further substituted by one substituent selected from -OR⁷, halogen, trifluoromethyl, -CN, -CO₂R⁷ and C₁₋₆galkyl optionally substituted by hydroxy;
R¹ is selected from methyl and chloro;
R² is selected from -NH-CO-R¹¹ and -CO-NH-(CH₂)ₜ-R^{12;}
R³ is selected from hydrogen, C₁₋₆alkyl optionally substituted by up to two OH groups, -(CH₂)ₖ-C₃₋₇cycloalkyl, -(CH₂)ₖphenyl optionally substituted by R¹³ and/or R¹⁴ and -(CH₂)ₖheteroaryl optionally substituted by R¹³ and/or R¹⁴,
R⁴ is selected from hydrogen and C₁₋₆alkyl, or
R³ and R⁴, together with the nitrogen atom to which they are bound, form a 5- or 6-membered heterocyclic ring optionally containing one additional heteroatom selected from oxygen, sulfur and N-R¹⁵;
R⁵ is selected from C₁₋₆alkyl optionally substituted by up to three halogen atoms, C₂₋₆alkenyl optionally substituted by phenyl, C₃₋₇cycloalkyl, heteroaryl optionally substituted by up to three R¹³ and/or R¹⁴ groups, and phenyl optionally substituted by R¹³ and/or R¹⁴;
R⁶ is a C₃₋₆alkyl group substituted by at least two substituents independently selected from -OR¹⁶, -NR¹⁶R¹⁷, -CO₂R¹⁶, -CONR¹⁶R¹⁷, -NHCOR¹⁶ and -NHSO₂R¹⁶;
R⁷ and R⁸ are each independently selected from hydrogen and C₁₋₆alkyl;
R⁹ is selected from hydrogen, -(CH₂)ᵤ-C₃₋₇cycloalkyl, -(CH₂)ᵤheterocyclyl, - (CH₂)ᵤaryl, and C₁₋₆alkyl optionally substituted by up to two substituents independently selected from -OR¹⁸ and -NR¹⁸R¹⁹,
R¹⁰ is selected from hydrogen and C₁₋₆alkyl, or
R⁹ and R¹⁰, together with the nitrogen atom to which they are bound, form a 5- or 6-membered heterocyclic ring optionally containing one additional heteroatom s elected from oxygen, sulfur and N-R¹⁵;
R¹¹ is s elected from hydrogen, C₁₋₆alkyl, - (CH₂)ₜ-C₃₋₇cycloalkyl, trifluoromethyl, - (CH₂)ᵥheteroaryl optionally substituted by R²⁰ and/or R²¹, and -(CH₂)ᵥphenyl optionally substituted by R²⁰ and/or R²¹;
R¹² is selected from hydrogen, C₁₋₆galkyl, C₃₋₇cycloalkyl, -CONHR²², phenyl optionally substituted by R²⁰ and/or R²¹, and heteroaryl optionally substituted by R²⁰ and/or R²¹;
R¹³ and R¹⁴ are each independently selected from halogen, cyano, trifluoromethyl, nitro, C₁₋₆alkyl, C₁₋₆alkoxy, -CONR²²R²³, -COR²⁴, -CO₂R²⁴, and heteroaryl, or
R¹³ and R¹⁴ are linked to form a fused 5-membered heterocyclyl ring containing one heteroatom selected from oxygen, sulfur and N-R¹⁵, or a fused heteroaryl ring;
R¹⁵ is selected from hydrogen and methyl;
R¹⁶, R¹⁷, R¹⁸ and R¹⁹ a re each independently selected from hydrogen and C₁-₆alkyl;
R²⁰ is selected from C₁₋₆alkyl, C₁₋₆alkoxy, -(CH₂)ₜ-C₃₋₇cycloalkyl, -CONR²²R²³, - NHCOR²³, halogen, -CN, -(CH₂)_{w}NR²⁵R²⁶, trifluoromethyl, phenyl optionally substituted by one or more R²¹ groups, and heteroaryl optionally substituted by one or more R²¹ groups;
R²¹ is selected from C₁₋₆alkyl, C₁₋₆alkoxy, halogen, trifluoromethyl, and - (CH₂)_{w}NR²⁵R²⁶;
R²² and R²³ are each independently selected from hydrogen and C₁₋₆alkyl, or
R22 and R²³, together with the nitrogen atom to which they are bound, form a 5- or 6-membered heterocyclic ring optionally containing one additional heteroatom selected from oxygen, sulfur and N-R¹⁵, wherein the ring may be substituted by up to two C₁₋₆alkyl groups;
R²⁴ is C₁₋₆alkyl;
R²⁵ is selected from hydrogen, C₁₋₆alkyl and -(CH₂)ₜ-C₃₋₇cycloalkyl optionally substituted by C₁₋₆alkyl,
R²⁶ is selected from hydrogen and C₁₋₆alkyl, or
R²⁵ and R²⁶, together with the nitrogen atom to which they are bound, form a 5- or 6-membered heterocyclic ring optionally containing one additional heteroatom selected from oxygen, sulfur and N-R¹⁵;
B is selected from a bond, oxygen, NH and S(O)ₓ;
X and Y are each independently selected from hydrogen, methyl and halogen;
Z¹ is N or N=O and Z² is CH,
Z¹ is CH and Z² is N or N=O, or
Z¹ and Z² are each independently selected from N or N=O;
k, m and w are each independently selected from 0, 1, 2 and 3;
n, q, r, s, t and x are each independently selected from 0, 1 and 2; and
u and v are each independently selected from 0 and 1;
or a pharmaceutically acceptable salt or solvate thereof.

In one embodiment, A includes 5-membered heteroaryl rings containing two heteroatoms independently selected from oxygen and nitrogen, for example rings containing two nitrogen atoms. Examples of suitable A groups include fused isoxazolyl, pyrazolyl and pyrrolyl rings such as those shown below:

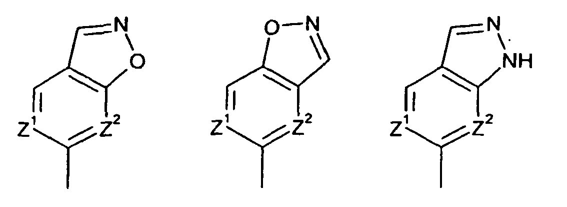

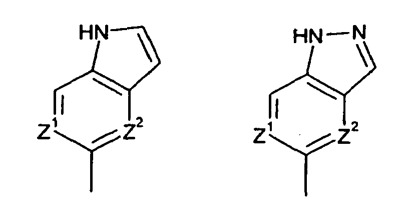

For example, suitable A groups include fused pyrazolyl rings such as those shown below:

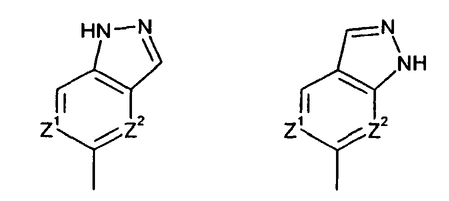

A representative example of an A group is a fused pyrazolyl ring such as that shown below:

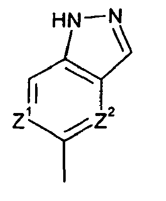

A further representative example of an A group is a fused pyrazolyl ring such as that shown below:

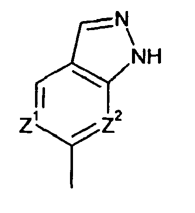

Ring A may be optionally substituted by substituents located on any position on the ring. Preferably, ring A is substituted by one substituent.

A representative example of a compound of formula (I) is wherein A is a fused pyrazolyl ring substituted in position (i), (ii) or (iii), such as position (iii), as shown below:

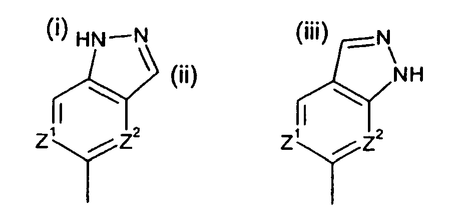

In one embodiment, A is optionally substituted by up to two substituents independently selected from C₁₋₄alkyl, in particular methyl; -(CH₂)ₖ-C₃₋₇cycloalkyl, in particular -(CH₂)ₖ-cyclopropyl; -(CH₂)ₖOR³; -(CH₂)ₖCO₂R³; -(CH₂)ₖNR³R⁴; - (CH₂)ₖCONR³R⁴; -(CH₂)ₖNHCOR³; -(CH₂)ₖSO₂(CH₂)ₘR⁵; and a 5- or 6-membered heterocyclyl ring containing nitrogen, in particular 4-piperidinyl, optionally substituted by C₁₋₂alkyl or -(CH₂)ₖCO₂R³. In a further embodiment, A is optionally substituted by up to two substituents independently selected from C₁₋₄alkyl, in particular methyl; halogen, in particular bromine; -(CH₂)ₖNR³R⁴; -(CH₂)ₖNHCOR³; -(CH₂)ₖNHSO₂R³; and - (CH₂)ₖSO₂(CH₂)ₘR⁵. A representative example of a substituent on A is - (CH₂)ₖSO₂(CH₂)ₘR⁵. Further representative examples of a substituent on A include C₁₋₄alkyl, in particular methyl; halogen, in particular bromine; -(CH₂)ₖNR³R⁴; -(CH₂)ₖNHCOR³; and -(CH₂)ₖNHSO₂R³. For example, A is substituted by -(CH₂)ₖNHCOR³.

In another embodiment, A is substituted by -BR⁶.

In another embodiment, A is substituted by -(CH₂)ₙheterocyclyl wherein the heterocyclyl is a 5- or 6-membered heterocyclic ring containing one or two heteroatoms independently selected from oxygen, sulfur and nitrogen optionally substituted by up to two substituents independently selected from oxo, C₁₋₆alkyl, -(CH₂)ₚphenyl, -OR⁷, - (CH₂)ₚCO₂R⁷, -NR⁷R⁸ and -CONR⁷R⁸. Typically, the heterocyclyl is a 5- or 6-membered heterocyclic ring containing one or two heteroatoms independently selected from oxygen and nitrogen wherein the heterocyclyl is optionally substituted by up to two substituents located on any position on the ring. For example, when the heterocyclyl contains a sulfur atom, the sulfur atom may have up to two oxo substituents. In one embodiment, the heterocyclyl is substituted by -(CH₂)ₙphenyl.

In a further embodiment, A is substituted by -(CH₂)_{q}aryl or -(CH₂)_{q}heteroaryl wherein the aryl or heteroaryl is optionally substituted by one or more substituents independently selected from oxo, C₁₋₆alkyl, halogen, cyano, trifluoromethyl, -OR⁹, -(CH₂)ᵣCO₂R¹⁰, - NR⁹R¹⁰, -(CH₂)ᵣCONR⁹R¹⁰, -NHCOR⁹, -SO₂NR⁹R¹⁰, -NHSO₂R⁹ and -S(O)ₛR⁹. Typically, the -(CH₂)_{q}aryl group is -(CH₂)_{q}phenyl and the -(CH₂)_{q}heteroaryl group is a group wherein the heteroaryl is a 5- or 6-membered heteroaryl ring containing up to two heteroatoms independently selected from oxygen and nitrogen. The -(CH₂)_{q}aryl and - (CH₂)_{q}heteroaryl groups are optionally substituted and the substituents may be located on any position on the aryl or heteroaryl. In one embodiment, the aryl is optionally substituted by one or two substituents independently selected from C₁₋₆alkyl, in particular methyl, halogen, cyano, trifluoromethyl, -OR⁹, -NR⁹R¹⁰, -(CH₂)ᵣCONR⁹R¹⁰ and -S(O)ₛR⁹. In a further embodiment, the aryl is optionally substituted by one or two substituents independently selected from C₁₋₆alkyl, halogen, cyano, -OR⁹ and -(CH₂)ᵣCO₂R¹⁰. Preferably, the heteroaryl is optionally substituted by one or two substituents independently selected from oxo and C₁₋₆alkyl, in particular methyl. A representative example of a substituent on A is -(CH₂)_{q}aryl wherein the aryl is substituted by C₁₋₆alkyl, in particular methyl, or halogen, in particular fluorine, for example -(CH₂)_{q}aryl wherein the aryl is substituted by fluorine. Further representative examples of a substituent on A is -(CH₂)_{q}aryl wherein the aryl is substituted by cyano, -OR⁹ or -(CH₂)ᵣCO₂R¹⁰.

A representative example of R¹ is methyl.

A representative example of R² is -CO-NH-(CH₂)ₜ-R¹².

In one embodiment, R³ selected from hydrogen; C₁₋₆alkyl optionally substituted by up to two OH groups, in particular methyl, ethyl, n-propyl, isopropyl, t-butyl or 2,2-dimethylpropyl optionally substituted by up to two OH groups; -(CH₂)ₖ-C₃₋₇cycloalkyl, in particular -(CH₂)ₖ-cyclopropyl; -(CH₂)ₖphenyl optionally substituted by R¹³ and/or R¹⁴; and -(CH₂)ₖheteroaryl, in particular thiazolyl, optionally substituted by R¹³ and/or R¹⁴. Representative examples of R³ include hydrogen; C₁₋₆alkyl, in particular methyl, ethyl, propyl and isopropyl; -(CH₂)ₖ-C₃₋₇cycloalkyl, in particular -(CH₂)ₖ-cyclopentyl; - (CH₂)ₖphenyl optionally substituted by R¹³ and/or R¹⁴; and -(CH₂)ₖheteroaryl optionally substituted by R¹³ and/or R¹⁴. For example, R³ may be C ₁₋₆alkyl, in particular methyl, ethyl or isopropyl, or -(CH₂)ₖheteroaryl.

In one embodiment, R⁴ is selected from hydrogen and C₁₋₄alkyl such as methyl. A representative example of R⁴ is hydrogen.

Alternatively, R³ and R⁴, together with the nitrogen atom to which they are bound, form a 5- or 6-membered heterocyclic ring optionally containing one additional heteroatom selected from oxygen, sulfur and N-R¹⁵, in particular a pyrrolidinyl, piperidinyl, piperazinyl or 4-methylpiperazinyl, or morpholinyl ring.

In one embodiment, R⁵ is selected from C₁₋₆alkyl optionally substituted by up to three halogen atoms, in particular methyl, ethyl, n-propyl, isopropyl, n-butyl and n-hexyl optionally s ubstituted by up to three halogen atoms; C₂₋₆alkenyl optionally substituted b y phenyl, in particular ethenyl optionally substituted by phenyl; C₃₋₇cycloalkyl, in particular cyclopropyl; heteroaryl optionally substituted by R¹³ and/or R¹⁴, in particular a 5-membered heteroaryl ring containing at least one heteroatom selected from oxyen, nitrogen and sulfur such as furyl, thienyl, isoxazolyl, imidazolyl or pyrazolyl optionally substituted by up to three R¹³ and/or R¹⁴ groups; and phenyl optionally substituted by R¹³ and/or R¹⁴. In a further embodiment, R⁵ is C₁₋₆alkyl optionally substituted by up to three halogen atoms, in particular C₁₋₄alkyl such as n-propyl or isopropyl; C₃₋₇cycloalkyl, in particular cyclopropyl; heteroaryl optionally substituted by R¹³ and/or R¹⁴, in particular a 5-membered heteroaryl ring containing at least one heteroatom selected from oxygen, nitrogen and sulfur such as thienyl; and phenyl optionally substituted by R¹³ and/or R¹⁴. Representative examples of R⁵ include C₁₋₆alkyl optionally substituted by up to three halogen atoms, in particular C₁₋₄alkyl such as n-propyl or isopropyl; heteroaryl optionally substituted by R¹³ and/or R¹⁴, in particular a 5-membered heteroaryl ring containing at least one heteroatom selected from oxygen, nitrogen and sulfur such as thienyl; and phenyl optionally substituted by R¹³ and/or R¹⁴. A further representative example of R⁵ is C₃₋₇cycloalkyl, in particular cyclopropyl.

In one embodiment, R⁶ is a C₃₋₆alkyl group substituted by from two to four substituents, for example two substituents, independently selected from -OR¹⁶, -NR¹⁶R¹⁷ and -CO₂R¹⁶.

In one embodiment, R⁷ and R⁸ are independently selected from hydrogen and C₁₋₄alkyl.

In one embodiment, R⁹ is selected from hydrogen; -(CH₂)ᵤ-C₃₋₇cycloalkyl, in particular -(CH₂)ᵤ-cyclohexyl; -(CH₂)ᵤheterocyclyl, in particular wherein the heterocyclyl is a 5 or 6 membered heterocyclyl containing one heteroatom selected from oxygen, nitrogen and sulfur such a tetrahydrofuran or tetrahydropyran; and C₁₋₆alkyl, in particular C₁₋₄alkyl such as methyl, ethyl, or n-propyl, optionally substituted by up to two substituents independently selected from -OR¹⁸ and -NR¹⁸R¹⁹. A representative example of R⁹ is C₁₋₆alkyl, in particular C₁₋₄alkyl such as methyl.

In one embodiment, R¹⁰ is hydrogen. A representative example of R¹⁰ is C₁₋₆alkyl, in particular C₁₋₄alkyl such as ethyl.

Alternatively, R⁹ and R¹⁰, together with the nitrogen atom to which they are bound, form a 5- or 6-membered heterocyclic ring optionally containing one additional heteroatom selected from oxygen, sulfur and N-R¹⁵, in particular morpholinyl.

In one embodiment, R¹¹ is a -(CH₂)ᵥheteroaryl optionally substituted by R²⁰ and/or R²¹.

In one embodiment, R¹² is selected from C₃₋₇cycloalkyl, phenyl optionally substituted by R²⁰ and/or R²¹, and heteroaryl optionally substituted by R²⁰ and/or R²¹. In a further embodiment, R¹² is selected from C₁₋₆alkyl, C₃₋₇cycloalkyl and heteroaryl optionally substituted by R²⁰ and/or R²¹. A representative example of R¹² is C₃₋₆cycloalkyl, in particular cyclopropyl. Further representative examples of R¹² include C₁₋₆alkyl, in particular C₁₋₄alkyl such as ethyl, and heteroaryl, in particular pyrazolyl, optionally substituted by R²⁰ and/or R²¹.

In one embodiment, R¹³ and R¹⁴ are each independently selected from halogen, in particular chlorine or fluorine; cyano; trifluoromethyl; nitro; C₁₋₄alkyl, in particular methyl, ethyl, n-propyl, isopropyl or n-butyl; C₁₋₄alkoxy, in particular methoxy; -CONR²²R²³; - COR¹⁵ ; -CO₂R¹⁵; and heteroaryl, in particular a 5-membered heteroaryl ring containing up to two heteroatoms independently selected from nitrogen and oxygen, for example isoxazolyl. Representative examples of R¹³ and R¹⁴ include halogen, in particular fluorine; C₁₋₄alkyl, in particular methyl; and C₁₋₄alkoxy, in particular methoxy.

Alternatively, R¹³ and R¹⁴ are linked to form a fused 5-membered heterocyclyl ring containing one heteroatom selected from oxygen, sulfur and N-R¹⁵.

In one embodiment, R¹⁶, R¹⁷, R¹⁸ and R¹⁹ are each independently selected from hydrogen and C₁₋₄alkyl.

In one embodiment, R²⁰ and R²¹ are each independently C₁₋₄alkoxy or - (CH₂)_{w}NR²⁵R²⁶. A representative example of R²⁰ or R²¹ is C₁₋₄alkyl, in particular methyl.

In one embodiment, R²² and R²³ are each independently hydrogen or C₁₋₄alkyl.

In one embodiment, R²⁴ is C₁₋₄alkyl.

In one embodiment, R²⁵ and R²⁶, together with the nitrogen atom to which they are bound, form a 5- or 6-membered heterocyclic ring optionally further containing one additional oxygen atom.

In one embodiment, B is a bond.

In one embodiment, X and Y are each independently selected from hydrogen, chlorine and fluorine. Representative examples of X include hydrogen and fluorine. A representative example of Y is hydrogen.

Z¹ and Z² are each independently selected from N, N=O and CH with the proviso that Z¹ and Z² are not both CH. In one embodiment, Z¹ is N or N=O and Z² is CH or Z¹ is CH and Z² is N. In another embodiment, Z¹ is N or N=O and Z² is CH. In a further embodiment, Z¹ is CH and Z² is N. For example, Z¹ is N and Z² is CH or Z¹ is CH and Z² is N.

A representative example of k and m is 0.

In one embodiment, n and r are independently 1.

In one embodiment, q is 0 or 1. A representative example of q is 0. A further representative example of q is 1.

In one embodiment, u is selected from 0 and 1.

In one embodiment, s is 2.

A representative example of t is 0.

In one embodiment, v and w are independently 0.

It is to be understood that the present invention covers all combinations of the embodiments and the particular and preferred groups described hereinabove. It is also to be understood that the present invention encompasses compounds of formula (I) in which a particular group or parameter, for example R³, R⁴, R⁵, R⁷, R⁸, R⁹, R¹⁰, R¹³, R¹⁴, R¹⁵, R¹⁶, R¹⁷, R¹⁸, R¹⁹, R²¹, R²², R²³, R²⁴, R²⁵, R²⁶, k, m, p, r, s, t, u orw may occur more than once. In such compounds it will be appreciated that each group or parameter is independently selected from the values listed.

Particular compounds according to the invention include those mentioned in the Examples. Specific examples which may be mentioned include:
*N*-cyclopropyl-4-methyl-3-{1-[(1-methylethyl)sulfonyl]-1*H*-pyrazolo[3,4-*c*]pyridin-5-yl}benzamide;
*N*-cyclopropyl-4-methyl-5-[1-(2-thienylsulfonyl)-1*H*-pyrazolo[3,4-*c*]pyridin-5-yl]benzamide;
*N*-cyclopropyl-3-fluoro-4-methyl-5-[1-(2-thienylsulfonyl)-1*H*-pyrazolo[3,4-*c*]pyridin-5-yl]benzamide;
*N*-cyclopropyl-3-[1-(cyclopropylsulfonyl)-1*H*-pyrazolo[3,4-*c*]pyridin-5-yl]-5-fluoro-4-methylbenzamide;
*N*-cyclopropyl-3-fluoro-4-methyl-5-[1-(3-methylphenyl)-1*H*-pyrazolo[3,4-*c*]pyridin-5-yl]benzamide;
*N*-cyclopropyl-4-methyl-5-(1-phenyl-1*H*-pyrazolo[3,4-*c*]pyridin-5-yl)benzamide; and *N*-cyclopropyl-3-[1-(2-fluorophenyl)-1*H*-pyrazolo[3,*4*-*c*]pyridin-5-yl]-4-methylbenzamide; and pharmaceutically acceptable salts or solvates thereof.

Further specific examples which may be mentioned include:
*N*-cyclopropyl-3-fluoro-5-[3-(4-fluorophenyl)-1*H*-pyrazolo[3,4-*b*]pyridin-6-yl]-4-methylbenzamide;
3-fluoro-5-[3-(4-fluorophenyl)-1*H*-pyrazolo[3,4-*b*]pyridin-6-yl]-4-methyl-*N*-(1-methyl-1*H-*pyrazol-5-yl)benzamide;
3-fluoro-5-[3-(4-fluorophenyl)-1*H*-pyrazolo[4,3-*c*]pyridin-6-yl]-4-methyl-*N*-(1-methyl-1*H-*pyrazol-5-yl)benzamide;
3-[3-(acetylamino)-1*H*-pyrazolo[3,4-*b*]pyridin-6-yl]-*N*-cyctopropyl-4-methylbenzamide;
*N*-cyclopropyl-4-methyl-3-{3-[(2-methylpropanoyl)amino]-1*H*-pyrazolo[3,4-*b*]pyridin-6-yl}benzamide;
*N*-cyclopropyl-4-methyl-3-[3-(propanoylamino)-1*H*-pyrazolo[3,4-*b*]pyridin-6-yl]benzamide; and
*N*-(6-{5-[(cyclopropylamino)carbonyl]-2-methylphenyl}-1*H*-pyrazolo[3,4-*b*]pyridin-3-yl)-2-thiophenecarboxamide;
and pharmaceutically acceptable salts or solvates thereof.

As used herein, the term "pharmaceutically acceptable" means a compound which is suitable for pharmaceutical use. Salts and solvates of compounds of the invention which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

The compounds of the present invention may be in the form of and/or may be administered as a pharmaceutically acceptable salt. For a review on suitable salts see Berge et al., J. Pharm. Sci., 1977, 66, 1-19.

Typically, a pharmaceutical acceptable salt may be readily prepared by using a desired acid or base as appropriate. The salt may precipitate from solution and be collected by filtration or may be recovered by evaporation of the solvent.

Salts of the compounds of the present invention may, for example, comprise acid addition salts resulting from reaction of an acid with a nitrogen atom present in a compound of formula (I). Salts encompassed within the term "pharmaceutically acceptable salts" refer to non-toxic salts of the compounds of this invention. Suitable addition s alts a re formed from acids which form non-toxic salts and examples are acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, ethanesulfonate, formate, fumarate, gluceptate, gluconate, glutamate, glycollylarsanilate, hexylresorcinate, hydrabamine, hydrobromide, hydrochloride, hydrogen phosphate, hydroiodide, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, oxaloacetate, pamoate (embonate), palmitate, pantothenate, phosphate/diphosphate, piruvate, polygalacturonate, saccharate, salicylate, stearate, subacetate, succinate, sulfate, tannate, tartrate, teoclate, tosylate, triethiodide, trifluoroacetate and valerate.

Pharmaceutically acceptable base salts include ammonium salts such as a trimethylammonium salt, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Those skilled in the art of organic chemistry will appreciate that many organic compounds can form complexes with solvents in which they are reacted or from which they are precipitated or crystallized. These complexes are known as "solvates". As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include water, methanol, ethanol and acetic acid. Preferably the solvent used is a pharmaceutically acceptable solvent. Examples of suitable pharmaceutically acceptable solvents include water, ethanol and acetic acid. Most preferably the solvent used is water. A complex with water is known as a "hydrate". Solvates of the compounds of the invention are within the scope of the invention.

As used herein, the term "alkyl" refers to straight or branched hydrocarbon chains containing the specified number of carbon atoms. For example, C₁₋₆alkyl means a straight or branched alkyl containing at least 1, and at most 6, carbon atoms. Examples of "alkyl" as used herein include, but are not limited to, methyl, ethyl, n-propyl, n-butyl, n-pentyl, isobutyl, isopropyl and t-butyl. A C₁₋₄alkyl group is preferred, for example methyl, ethyl, isopropyl or t-butyl. The said alkyl groups may be optionally substituted with one or more fluorine atoms for example, trifluoromethyl.

As used herein, the term "alkenyl" refers to straight or branched hydrocarbon chains containing the specified number of carbon atoms and containing at least one double bond. For example, C₂₋₆alkenyl means a straight or branched alkenyl containing at least 2, and at most 6, carbon atoms and containing at least one double bond. Examples of "alkenyl" as used herein include, but are not limited to ethenyl, 2-propenyl, 3-butenyl, 2-butenyl, 2-pentenyl, 3-pentenyl, 3-methyl-2-butenyl, 3-methylbut-2-enyl, 3-hexenyl and 1,1-dimethylbut-2-enyl.

As used herein, the term "alkoxy" refers to a straight or branched chain alkoxy groups containing the specified number of carbon atoms. For example, C₁₋₆alkoxy means a straight or branched alkoxy containing at least 1, and at most 6, carbon atoms. Examples of "alkoxy" as used herein include, but are not limited to methoxy, ethoxy, propoxy, prop-2-oxy, butoxy, but-2-oxy, 2-methylprop-1-oxy, 2-methylprop-2-oxy, pentoxy, or hexyloxy. A C₁₋₄alkoxy group is preferred, for example methoxy or ethoxy.

As used herein, the term "cycloalkyl" refers to a non-aromatic hydrocarbon ring containing the specified n umber of carbon a toms which m ay optionally contain up to one double bond. For example, C₃₋₇cycloalkyl means a non-aromatic ring containing at least three, and at most seven, ring carbon atoms. Examples of "cycloalkyl" as used herein include, but are not limited to, cyclopropyl, cyclobutyl, cyclopentyl, cyclohexyl and cycloheptyl. A C₃₋₆cycloalkyl g roup i s p referred, for example, cyclopropyl, cyclopentyl or cyclohexyl.

As used herein, the term "aryl" refers to an aromatic carbocyclic ring such as phenyl, biphenyl or naphthyl. Preferably the aryl is phenyl.

As used herein, the terms "heteroaryl ring" and "heteroaryl", unless otherwise defined, refer to a monocyclic 5- to 7-membered unsaturated hydrocarbon ring containing at least one heteroatom independently selected from oxygen, nitrogen and sulfur. Preferably, the heteroaryl ring has five or six ring atoms. Examples of heteroaryl rings include, but are not limited to, furyl, thienyl, pyrrolyl, oxazolyl, thiazolyl, isoxazolyl, isothiazolyl, imidazolyl, pyrazolyl, oxadiazolyl, triazolyl, tetrazolyl, thiadiazolyl, pyridyl, pyridazinyl, pyrimidinyl, pyrazinyl and triazinyl. The said ring may be optionally substituted by one or more substituents independently selected from C₁₋₆alkyl and oxy.

As used herein, the terms "heterocyclic ring" or "heterocyclyl", unless otherwise defined refer to a monocyclic 3 - t o 7 -membered saturated hydrocarbon ring containing at least one heteroatom independently selected from oxygen, nitrogen and sulfur. Preferably, the heterocyclyl ring has five or six ring atoms. Examples of heterocyclyl groups include, but are not limited to, pyrrolidinyl, imidazolidinyl, pyrazolidinyl, piperidyl, piperazinyl, morpholino, tetrahydropyranyl, tetrahydrofuranyl, a nd t hiomorpholino. The said ring may be optionally substituted by one or more substituents independently selected from C₁₋₆alkyl and oxy.

As used herein, the terms "halogen" or "halo" refer to the elements fluorine, chlorine, bromine and iodine. Preferred halogens are fluorine, chlorine and bromine. A particularly preferred halogen is fluorine or chlorine.

As used herein, the term "optionally" means that the subsequently described event(s) may or may not occur, and includes both event(s) which occur and events that do not occur.

As used herein, the term "substituted" refers to substitution with the named substituent or substituents, multiple degrees of substitution being allowed unless otherwise stated.

Certain compounds of formula (I) may exist in stereoisomeric forms (e.g. they may contain one or m ore asymmetric carbon atoms or may exhibit cis-trans isomerism). The individual stereoisomers (enantiomers and diastereomers) and mixtures of these are included within the scope of the present invention. The present invention also covers the individual isomers of the compounds represented by formula (I) a s mixtures with isomers thereof in which one or more chiral centres are inverted. Likewise, it is understood that compounds of formula (I) may exist in tautomeric forms other than that shown in the formula and these are also included within the scope of the present invention.

Separation of diastereoisomers or cis and trans isomers may be achieved by conventional techniques, e.g. by fractional crystallisation, chromatography or H.P.L.C. A stereoisomeric mixture of the agent may also be prepared from a corresponding optically pure intermediate or by resolution, such as H.P.L.C. of the corresponding racemate using a suitable chiral support or by fractional crystallisation of the diastereoisomeric salts formed by reaction of the corresponding racemate with a suitable optically active acid or base, as appropriate.

Furthermore, some of the crystalline forms of the compounds of structure (I) may exist as polymorphs, which are included in the present invention.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the working Examples.

A compound of formula (I) may be prepared by reacting a compound of formula (II) 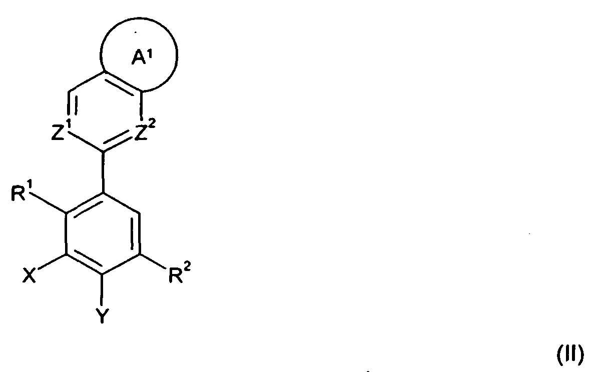 in which R¹, R², X, Y, Z¹ and Z² are as hereinbefore defined and A¹ is an unsubstituted fused 5-membered heteroaryl ring containing one or two heteroatoms independently selected from oxygen and nitrogen, with a suitable reagent such as a halide derivative. For example, when A is substituted by -(CH₂)ₖSO₂(CH₂)ₘR⁵ wherein k is 0, the compound of formula (II) may be reacted with a compound of formula (III)

R⁵(CH₂)ₘSO₂(CH₂)ₖ-Hal (III)

in which R⁵ and m are as hereinbefore defined, k is 0 and Hal is halogen, in particular chlorine,
in, for example, the presence of a base such as sodium hydride and a solvent such as DMF.

A compound of formula (I) may be prepared by reacting a compound of formula (IV) 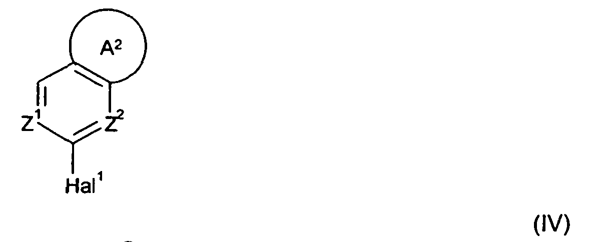 in which Z¹ and Z² are as hereinbefore defined, A² is group convertible to A as hereinbefore defined and Hal¹ is halogen, in particular chlorine,
with a compound of formula (VA) or (VB) 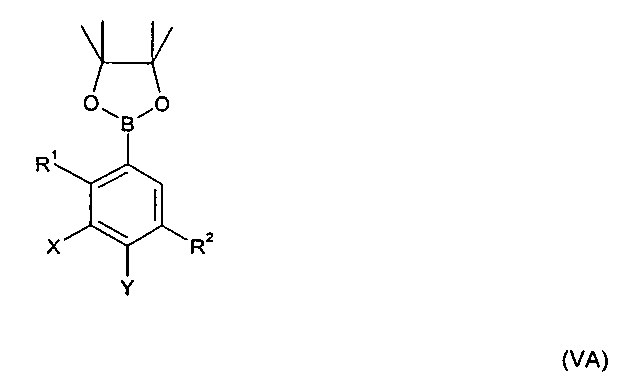 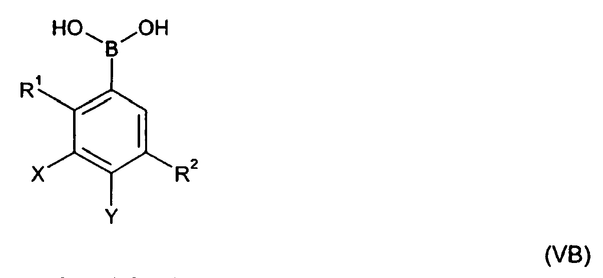 in which R¹ , R², X and Y are as hereinbefore defined,
in the presence of a catalyst, for example tetrakis(triphenylphosphine)palladium.

A compound of formula (VA) may be prepared by, for example, reacting a compound of formula (VI) 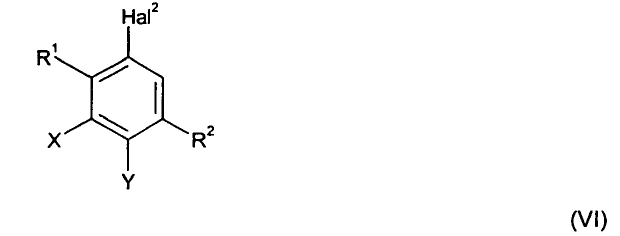 in which R¹, R ², X a nd Y are as hereinbefore defined a nd H al² is halogen, i n p articular iodine,
with bis(pinnacolato)diboron, [1,1'-bis(diphenylphosphino)ferrocene] dichloropalladium (II) complex (PdCl₂(ppdf)) and potassium acetate in a solvent such as DMF.

A compound of formula (VB) may be prepared by, for example, reacting a compound of formula (VI) as hereinbefore defined, with n-butyl lithium and triisopropyl borate in a solvent such as THF.

When R² is -NH-CO-R¹¹, a compound of formula (VI) may be prepared by reacting an amine of formula (VII) 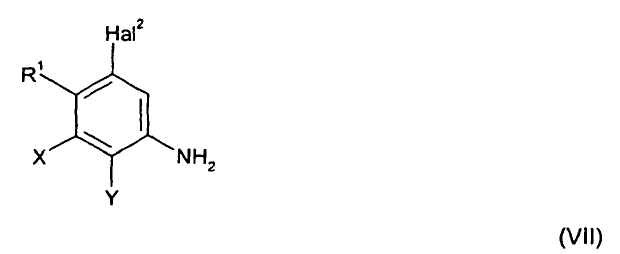 in which R¹, X, Y and Hal² are as hereinbefore defined,
with an acid compound of formula (VIII)

R¹¹CO₂H (VIII)

in which R¹¹ is as hereinbefore defined,
under amide forming conditions.

Suitable amide forming conditions are well known in the art and include adding a base such as DIPEA to a mixture of the amine of formula (VII), the acid of formula (VIII), and HATU in a solvent such as DMF.

Alternatively, when R² is -CO-NH-(CH₂)ₜ-R¹², a compound of formula (VI) may readily be prepared from a corresponding acid compound of formula (IX) 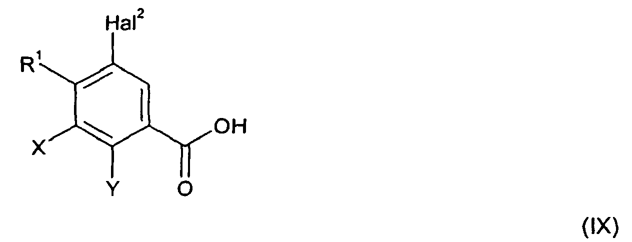 in which R¹, X, Y and Hal² are as hereinbefore defined,
by converting the acid to an activated form of the acid, for example the acid chloride, by treatment with, for example, thionyl chloride, and then reacting the activated acid thus formed with an amine compound of formula (X)

R¹²-(CH₂)ₜ-NH₂ (X)

in which R⁷ is as hereinbefore defined,
under amide forming conditions.

Suitable amide forming conditions are well known i n the art and include treating a solution of the acid of formula (IX), or the activated form thereof, in for example DMF, with an amine of formula (X) in the presence of a base such as triethylamine.

When A is a fused pyrazolyl, another general method for preparing compounds of formula (I) comprises reacting a compound of formula (XI) 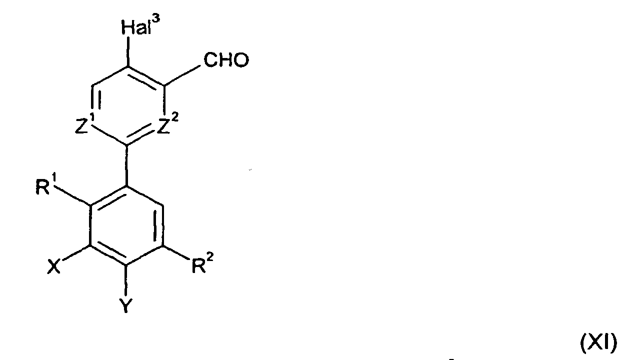 in which R¹, R², X, Y, Z¹ and Z² are as hereinbefore defined and Hal³ is halogen, in particular chlorine, with a hydrazine derivative.

Similarly, when A is a fused pyrazolyl substituted by aryl, another general method for preparing compounds of formula (I) comprises reacting a compound of formula (XII) 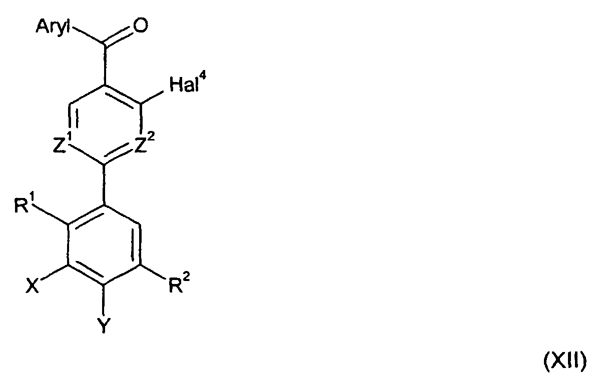 in which R¹, R², X, Y, Z¹ and Z² are as hereinbefore defined and Hal⁴ is halogen, in particular chlorine, with a hydrazine derivative.

Alternatively, a further general method comprises final stage modification of one compound of formula (I) into another compound of formula (I). Suitable f unctional group transformations for converting one compound of formula (I) into another compound of formula (I) are well known in the art and are described in, for instance, Comprehensive Heterocyclic Chemistry II, eds. A. R. Katritzky, C. W. Rees and E. F. V. Scriven (Pergamon Press,1996), Comprehensive Organic Functional Group Transformations, eds. A.R. Katritzky, O. Meth-Cohn and C.W. Rees (Elsevier Science Ltd., Oxford, 1995), Comprehensive Organic Chemistry, eds. D. Barton and W.D. Ollis (Pergamon Press, Oxford, 1979), and Comprehensive Organic Transformations, R.C. Larock (VCH Publishers Inc., New York, 1989).

For example, one general method for preparing the compounds of formula (I) comprises the reactions set out in Scheme 1 below.

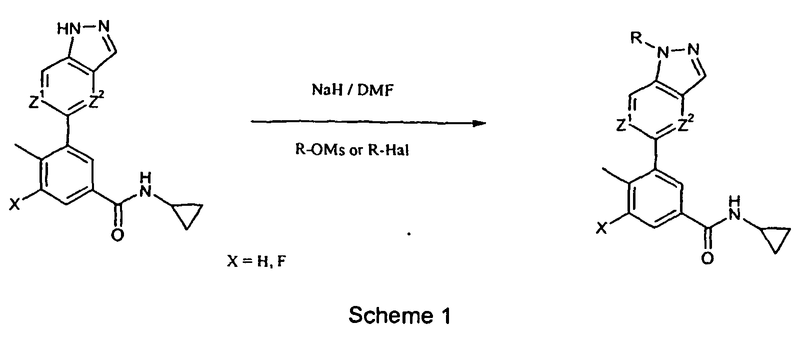

For example, another method for preparing the compounds of formula (I) comprises the reactions set out in Scheme 2 below.

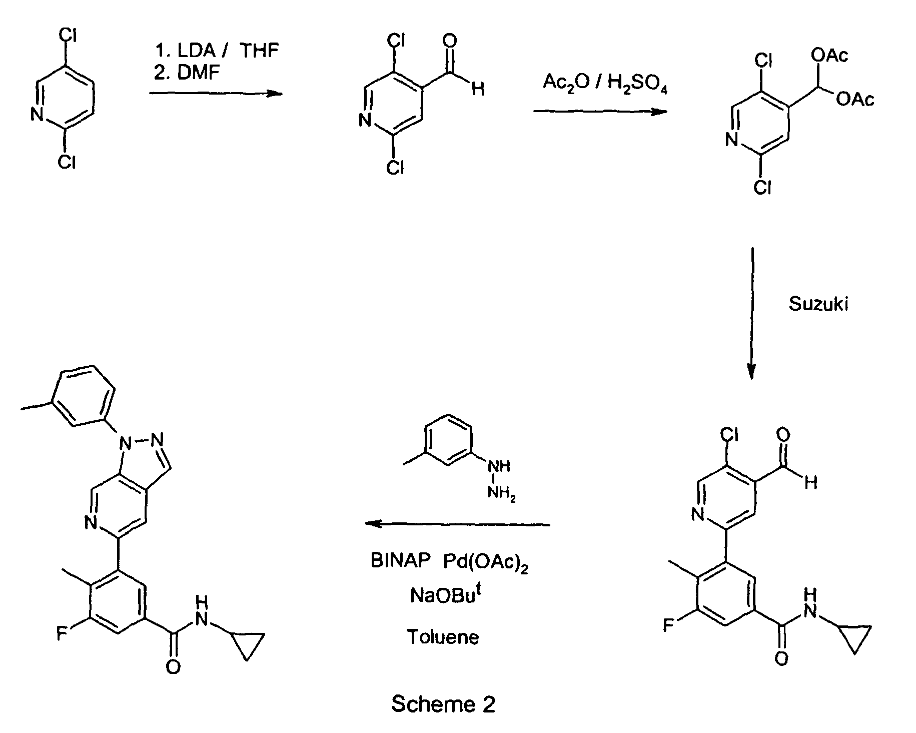

For example, another method for preparing the compounds of formula (I) comprises the reactions set out in Scheme 3 below.

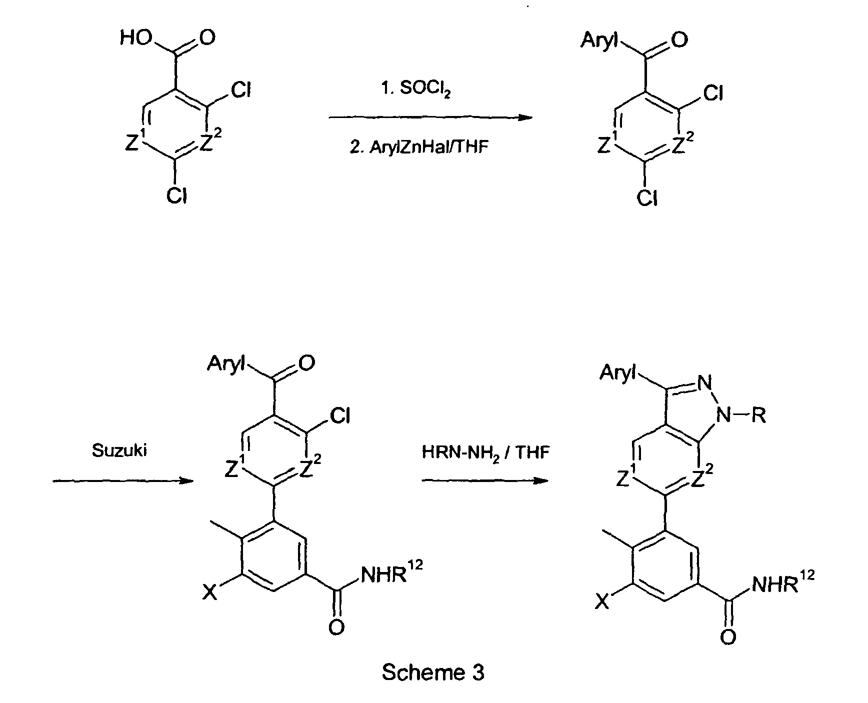

For example, a further method for preparing the compounds of formula (I) comprises the reactions set out in Scheme 4 below.

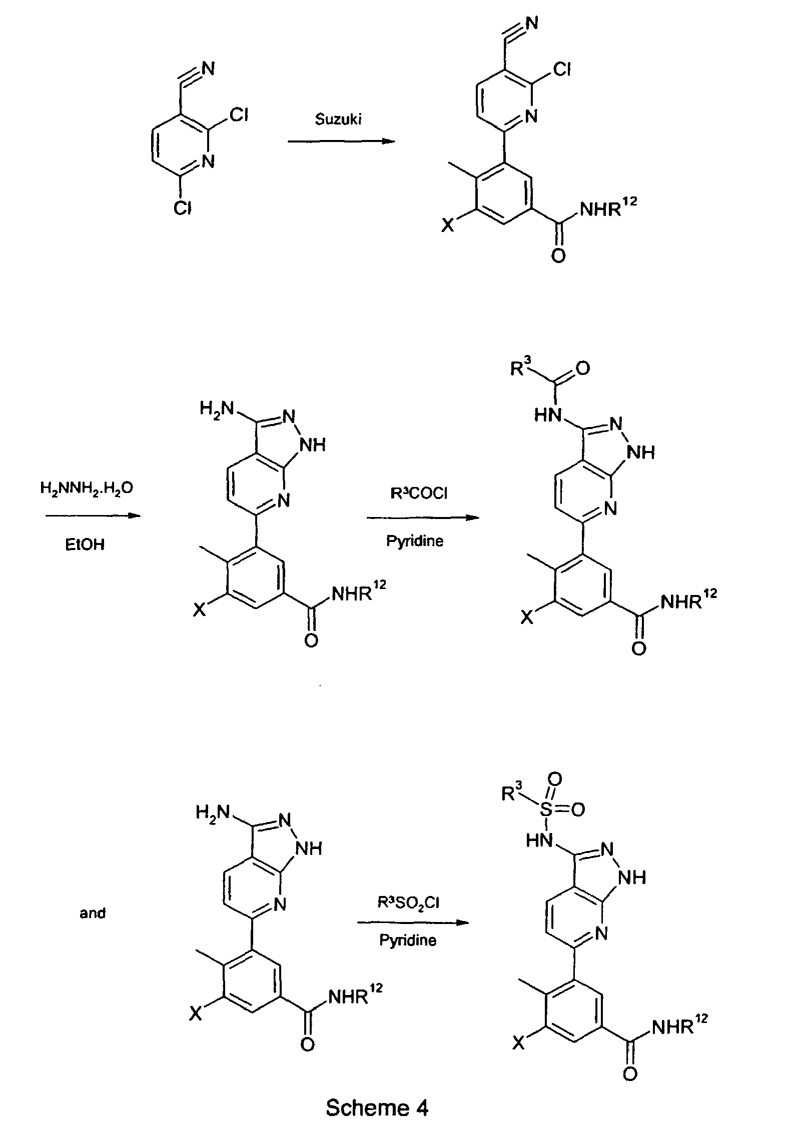

Those skilled in the art will appreciate that in the preparation of the compounds of the invention it may be necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl type protecting groups (e.g. benzyl, trityl, chlorotrityl). Examples of suitable oxygen protecting groups may include for example alky silyl groups, such as trimethylsilyl or tert-butyldimethylsilyl; alkyl ethers such as tetrahydropyranyl or tert-butyl; or esters such as acetate.

Whilst it is possible for the compounds of the present invention to be administered as the raw chemical, the compounds of formula (I) and their pharmaceutically acceptable salts or solvates are conveniently administered in the form of pharmaceutical compositions eg when the agent is in admixture with a suitable pharmaceutical excipient, diluent and/or carrier selected with regard to the intended route of administration and standard pharmaceutical practice.

Thus, in another aspect of the invention, we provide a pharmaceutical composition comprising at least one compound of formula (I) or a pharmaceutically acceptable derivative thereof, in association with one or more pharmaceutically acceptable excipients, diluents and/or carriers. The excipient, diluent or carrier must be "acceptable" in the sense of being compatible with the other ingredients of the formulation and not deletrious to the recipient thereof.

According to a further aspect, the invention provides a pharmaceutical composition comprising, as active ingredient, at least one compound of the invention or a pharmaceutically acceptable derivative thereof, in association one or more pharmaceutically acceptable excipients, diluents and/or carriers for use in therapy, and in particular in the treatment of human or animal subjects suffering from a condition susceptible to amelioration by an inhibitor of p38 kinase.

The present invention also provides a pharmaceutical composition comprising a therapeutically effective amount of the compounds of the present invention and a pharmaceutically acceptable excipient, diluent and/or carrier (including combinations thereof).

There is further provided by the present invention a process of preparing a pharmaceutical composition, which process comprises mixing at least one compound of the invention or a pharmaceutically acceptable salt or solvate thereof, together with a pharmaceutically acceptable excipient, diluent and/or carrier.

The pharmaceutical compositions may be for human or animal usage in human and veterinary medicine and will typically comprise any one or more of a pharmaceutically acceptable excipient, diluent or carrier. Acceptable carriers or diluents for therapetic use are well known in the pharmaceutical art, and are described, for example, in Remington's Pharmaceutical Sciences, Mack Publishing Co. (A. R. Gennaro edit. 1985). The choice of pharmaceutical excipient, diluent or carrier can be selected with regard to the intended route of administration and standard pharmaceutical practice. The pharmaceutical compositions may comprise as- or in addition to - the excipient, diluent or carrier a ny suitable binder(s), lubricant(s), suspending agent(s), coating agent(s) and solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

For some embodiments, the agents of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, beta- and gamma-cyclodextrins are most commonly used and suitable examples are described in WO 91/11172, WO 94/02518 and WO 98/55148.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see WO 02/00196 (SmithKline Beecham).

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

The routes for administration (delivery) include, but are not limited to, one or more of: oral (e. g. as a tablet, capsule, or as an ingestable solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual. It is to be understood that not all of the compounds need be administered by the same route. Likewise, if the composition comprises more than one active component, then those components may be administered by different routes.

The compounds of formula (I) and their pharmaceutically acceptable salts and solvates may be formulated for administration in any suitable manner. They may, for example, be formulated for topical administration or administration by inhalation or, more preferably, for oral, transdermal or parenteral administration. The pharmaceutical composition may be in a form such that it can effect controlled release of the compounds of formula (I) and their pharmaceutically acceptable salts and solvates. In a preferred embodiment, the agents of the present invention are delivered systemically such as orally, buccally or sublingually. A particularly preferred method of administration, and corresponding formulation, is oral administration.

For oral administration, the pharmaceutical composition may take the form of, and be administered as, for example, tablets (including sub-lingual tablets) and capsules (each including timed release and sustained release formulations), ovules, pills, powders, granules, elixirs, tinctures, emulsions, solutions, syrups or suspensions prepared by conventional means with acceptable excipients for immediate-, delayed-, modified-, sustained-, pulsed- or controlled-release applications.

For instance, for oral administration in the form of a tablet or capsule, the active drug component can be combined with an oral, non-toxic pharmaceutically acceptable inert carrier such as ethanol, glycerol, water and the like. The tablets may also contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules can be made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additives such as peppermint oil or saccharin, and the like can also be added.

Where appropriate, dosage unit formulations for oral administration can be microencapsulated. The formulation can also be prepared to prolong or sustain the release as for example by coating or embedding particulate material in polymers, wax or the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of the present invention can also be administered in the form of liposome emulsion delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

The present invention includes pharmaceutical compositions containing 0.1 to 99.5%, more particularly, 0.5 to 90% of a compound of the formula (I) in combination with a pharmaceutically acceptable carrier.

Likewise, the composition may also be administered in nasal, ophthalmic, otic, rectal, topical, intravenous (both bolus and infusion), intraperitoneal, intraarticular, subcutaneous or intramuscular, inhalation or insufflation form, all using forms well known to those of ordinary skill in the pharmaceutical arts.

For transdermal administration, the pharmaceutical composition may be given in the form of a transdermal patch, such as a transdermal iontophoretic patch.

If the compound of the present invention is administered parenterally, then examples of such administration include one or more of: intravenously, intraarterially, intraperitoneally, intrathecally, intraventricularly, intraurethrally, intrasternally, intracranially, intramuscularly or subcutaneously administering the agent; and/or by using infusion techniques. For parenteral administration, the pharmaceutical composition may be given as an injection or a continuous infusion (e.g. intravenously, intravascularly or subcutaneously). The compositions may take such forms as suspensions, solutions or emulsions in oily or aqueous vehicles and may contain formulatory agents such as suspending, stabilizing and/or dispersing agents. F or administration by injection these may take the form of a unit dose presentation or as a multidose presentation preferably with an added p reservative. A Iternatively for parenteral administration the active ingredient may be in powder form for reconstitution with a suitable vehicle. F or parenteral administration, the compound is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The compositions of the present invention may be administered by direct injection.

The compounds of the invention may also be formulated as a depot preparation. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble salts or solvates, for example, as a sparingly soluble salt.

Alternatively the composition may be formulated for topical application, for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water.

Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

For administration by inhalation the compounds according to the invention are conveniently delivered in the form of an aerosol spray presentation from pressurized packs or a nebulizer, with the use of a suitable propellant, e.g. dichlorodifluoromethane, trichlorofluoromethane, dichlorotetrafluoroethane, a hydrofluoroalkane such as tetrafluoroethane or heptafluoropropane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

Alternatively, the compound of the present invention can be administered in the form of a suppository or pessary, or it may be applied topically in the form of a gel, hydrogel, lotion, solution, cream, ointment or dusting powder.

The compounds of the present invention may also be administered by the pulmonary or rectal routes. They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

The pharmaceutical compositions generally are administered in an amount effective for treatment or prophylaxis of a specific condition or conditions. Initial dosing in humans is accompanied by clinical monitoring of symptoms, such symptoms for the selected condition. In general, the compositions are administered in an amount of active agent of at least about 100 µg/kg body weight. In most cases they will be administered in one or more doses in an amount not in excess of about 20 mg/kg body weight per day. Preferably, in most cases, dose is from about 100 µg/kg to about 5 mg/kg body weight, daily. For administration particularly to mammals, and particularly humans, it is expected that the daily dosage level of the active agent will be from 0. 1 mg/kg to 10 mg/kg and typically around 1 mg/kg. It will be appreciated that optimum dosage will be determined by standard methods for each treatment modality and indication, taking into account the indication, its severity, route of administration, complicating conditions and the like. The physician in any event will determine the actual dosage which will be most suitable for an individual and will vary with the activity of the specific compound to be employed, the metabolic stablity and length of action of that compound, age, weight, general health, sex, diet, mode and time of administration, rate of excretion, drug combination, severity of the particular condition and response of the particular individual. The effectiveness of a selected actual dose can readily be determined, for example, by measuring clinical symptoms or standard anti-inflammatory indicia after administration of the selected dose. The above dosages are exemplary of the average case. There can, of course, be individual instances where higher or lower dosage ranges are merited, and such are within the scope of this invention. For conditions or disease states as are treated by the present invention, maintaining consistent daily levels in a subject over an extended period of time, e.g., in a maintenance regime, can be particularly beneficial. For oral and parenteral administration to humans, the daily dosage level of the agent may be in single or divided doses.

In another aspect, the present invention provides a compound of formula (I) or a pharmaceutically acceptable derivative thereof, for use in therapy.

The compounds of the present invention are generally inhibitors of the serine/threonine kinase p38 and are therefore also inhibitors of cytokine production which is mediated by p38 kinase. Within the meaning of the term "inhibitors of the serine/threonine kinase p38" are included those compounds that interfere with the ability of p38 to transfer a phosphate group from ATP to a protein substrate according to the assay described below.

It will be appreciated that the compounds of the invention may be selective for one or more of the isoforms of p38, for example p38α, p38β, p38γ and/or p38δ. In one embodiment, the compounds of the invention selectively inhibit the p38α isoform. In another embodiment, the compounds of the invention selectively inhibit the p38β isoform. In a further embodiment, the compounds of the invention selectively inhibit the p38α and p38β isoforms. Assays for determining the selectivity of compounds for the p38 isoforms are described in, for example, WO 98/61426, WO 00/71535 and WO 02/46158.

It is known that p38 kinase activity can be elevated (locally or throughout the body), p38 kinase can be incorrectly temporally active or expressed, p38 kinase can be expressed or active in a n inappropriate location, p 38 kinase c an b e constitutively expressed, or p38 kinase expression can be erratic; similarly, cytokine production mediated by p38 kinase activity can be occurring at inappropriate times, inappropriate locations, or it can occur at detrimentally high levels.

Accordingly, the present invention provides a compound of formula (I) or a pharmaceutically acceptable derivative thereof for use in the treatment or prophylaxis of a condition or disease state mediated by p38 kinase activity or mediated by cytokines produced by the activity of p38 kinase.

Disclosed is a method for the treatment of a condition or disease state mediated by p38 kinase activity, or mediated by cytokines produced by the activity of p38 kinase, in a subject which comprises administering to said subject a therapeutically effective amount of a compound of formula (I) or a pharmaceutically acceptable derivative thereof. The compound may be administered as a single or polymorphic crystalline form or forms, an amorphous form, a single enantiomer, a racemic mixture, a single stereoisomer, a mixture of stereoisomers, a single diastereoisomer or a mixture of diastereoisomers.

Disclosed is a method of inhibiting cytokine production which is mediated by p38 kinase activity in a subject, e.g. a human, which comprises administering to said subject in need of cytokine production inhibition a therapeutic, or cytokine-inhibiting, amount of a compound of the present invention. The compound may be administered as a single or polymorphic crystalline form or forms, an amorphous form, a single enantiomer, a racemic mixture, a single stereoisomer, a mixture of stereoisomers, a single diastereoisomer or a mixture of diastereoisomers.

The present invention treats these conditions by providing a therapeutically effective amount of a compound of this invention. By "therapeutically effective amount" is meant a symptom-alleviating or symptom-reducing amount, a cytokine-reducing amount, a cytokine-inhibiting amount, a kinase-regulating amount and/or a kinase-inhibiting amount of a compound. Such amounts can be readily determined by standard methods, such as by measuring cytokine levels or observing alleviation of clinical symptoms. For example, the clinician can monitor accepted measurement scores for anti-inflammatory treatments. It will be appreciated that reference to treatment includes acute treatment or prophylaxis as well as the alleviation of established symptoms.

The compounds of the present invention can be administered to any subject in need of inhibition or regulation of p38 kinase or in need of inhibition or regulation of p38 mediated cytokine production. In particular, the compounds may be administered to mammals. Such mammals can include, for example, horses, cows, sheep, pigs, mice, dogs, cats, primates such as chimpanzees, gorillas, rhesus monkeys, and, most preferably, humans.

Thus, the present invention provides methods of treating or reducing symptoms in a human or animal subject suffering from, for example, rheumatoid arthritis, osteoarthritis, asthma, psoriasis, eczema, allergic rhinitis, allergic conjunctivitis, adult respiratory distress syndrome, chronic pulmonary inflammation, chronic obstructive pulmonary disease, chronic heart failure, silicosis, endotoxemia, toxic shock syndrome, inflammatory bowel disease, tuberculosis, atherosclerosis, neurodegenerative disease, Alzheimer's disease, Parkinson's disease, Huntington's disease, amyotrophic lateral sclerosis, epilepsy, multiple sclerosis, aneurism, stroke, irritable bowel syndrome, muscle degeneration, bone resorption diseases, osteoporosis, diabetes, reperfusion injury, graft vs. host reaction, allograft rejections, sepsis, systemic cachexia, cachexia secondary to infection or malignancy, cachexia secondary to aquired immune deficiency syndrome (AIDS), malaria, leprosy, infectious arthritis, leishmaniasis, Lyme disease, glomerulonephritis, gout, psoriatic arthritis, Reiter's syndrome, traumatic arthritis, rubella arthritis, Crohn's disease, ulcerative colitis, acute synovitis, gouty arthritis, spondylitis, and non articular inflammatory conditions, for example, herniated/ruptured/prolapsed intervertebral disk syndrome, bursitis, tendonitis, tenosynovitis, fibromyalgic syndrome and other inflammatory conditions associated with ligamentous sprain and regional musculoskeletal strain, pain, for example that associated with inflammation and/or trauma, osteopetrosis, restenosis, thrombosis, angiogenesis, cancer including breast cancer, colon cancer, lung cancer or prostatic cancer, which comprises administering to said subject a therapeutically effective amount of a compound of formula (I) or a pharmaceutically acceptable derivative thereof.

Disclosed is a method of treatment of a human or animal subject suffering from rheumatoid arthritis, asthma, psoriasis, chronic pulmonary inflammation, chronic obstructive pulmonary disease, chronic heart failure, systemic cachexia, glomerulonephritis, Crohn's disease, neurodegenerative disease, Alzheimer's disease, Parkinson's disease, epilepsy and cancer including breast cancer, colon cancer, lung cancer and prostatic cancer, which comprises administering to said subject a therapeutically effective amount of a compound of formula (I) or a pharmaceutically acceptable derivative thereof.

Disclosed is a method of treatment of a human or animal subject suffering from rheumatoid arthritis, asthma, psoriasis, chronic pulmonary inflammation, chronic obstructive pulmonary disease, chronic heart failure, systemic cachexia, glomerulonephritis, Crohn's disease and cancer including breast cancer, colon cancer, lung cancer and prostatic cancer, which comprises administering to said subject a therapeutically effective amount of a compound of formula (I) or a pharmaceutically acceptable derivative thereof.

Disclosed is a method of treatment of a human or animal subject suffering from rheumatoid arthritis, asthma, chronic pulmonary inflammation, chronic obstructive pulmonary disease, neurodegenerative disease, Alzheimer's disease, Parkinson's disease and epilepsy which comprises administering to said subject a therapeutically effective amount of a compound of formula (I) or a pharmaceutically acceptable derivative thereof.

Disclosed is a method of treatment of a human or animal subject suffering from any type of pain including chronic pain, rapid onset of analgesis, neuromuscular pain, headache, cancer pain, acute and chronic inflammatory pain associated with osteoarthritis and rheumatoid arthritis, post operative inflammatory pain, neuropathic pain, diabetic neuropathy, trigeminal neuralgia, post-hepatic neuralgia, inflammatory neuropathies and migraine pain which comprises administering to said subject a therapeutically effective amount of a compound of formula (I) or a pharmaceutically acceptable derivative thereof.

A further aspect of the invention provides the use of a compound of formula (I), or a pharmaceutically acceptable derivative thereof, in the manufacture of a medicament for use in the treatment of a condition or disease state mediated by p38 kinase activity or mediated by cytokines produced by p38 kinase activity.

The compounds of formula (I) and their salts and solvates may be employed alone or in combination with other therapeutic agents for the treatment of the above-mentioned conditions. The invention thus provides, in a further aspect, a combination comprising a compound of the invention or a pharmaceutically acceptable derivative thereof together with a further therapeutic agent.

In particular, in rheumatoid arthritis therapy, combination with other chemotherapeutic or antibody agents is envisaged. Combination therapies according to the present invention thus comprise the administration of at least one compound of formula (I) or a pharmaceutically acceptable salt or solvate thereof and at least one other pharmaceutically active agent. The compound(s) of formula (I) or pharmaceutically acceptable salt(s) or solvate(s) thereof and the other pharmaceutically active agent(s) may be administered together or separately and, when administered separately, this may occur separately or sequentially in any order. The amounts of the compound(s) of formula (I) or pharmaceutically acceptable salt(s) or solvate(s) thereof and the other pharmaceutically active agent(s) and the relative timings of administration will be selected in order to achieve the desired combined therapeutic effect. Appropriate doses will be readily appreciated by those skilled in the art. It will be appreciated that the amount of a compound of the invention required for treatment will vary with the nature of the condition being treated and the age and condition of the patient and will ultimately be at the discretion of the attendant physician or veterinarian. Examples of other pharmaceutically active agents which may be employed in combination with compounds of formula (I) and their salts and solvates for rheumatoid arthritis therapy include: immunosuppresants such as amtolmetin guacil, mizoribine and rimexolone; anti-TNFα agents such as etanercept, infliximab, diacerein; tyrosine kinase inhibitors such as leflunomide; kallikrein antagonists such as subreum; interleukin 11 agonists such as oprelvekin; interferon beta 1 agonists; hyaluronic acid agonists such as NRD-101 (Aventis); interleukin 1 receptor antagonists such as anakinra; CD8 antagonists such as amiprilose hydrochloride; beta amyloid precursor protein antagonists such as reumacon; matrix metalloprotease inhibitors such as cipemastat and other disease modifying anti-rheumatic drugs (DMARDs) such as methotrexate, sulphasalazine, cyclosporin A, hydroxychoroquine, auranofin, aurothioglucose, gold sodium thiomalate and penicillamine.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention.

The individual components of such combinations may be administered either sequentially or simultaneously in separate or combined pharmaceutical formulations by any convenient route.

When administration is sequential, either the compound of the invention or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

### EXAMPLES

The following Examples are illustrative embodiments of the invention, not limiting the scope of the invention in any way. Reagents are commercially available or are prepared according to procedures in the literature.

*N-*Cyclopropyl-4-methyl-3-(4,4,5,5-tetramethyl-1,3,2-dioxaborolan-2-yl)benzamide and {5-[(cyclopropylamino)carbonyl]-3-fluoro-2-methylphenyl}boronic acid may be prepared by the procedures described in WO 03/068747.

3-Thiophenesulfonyl chloride may be purchased from Maybridge International.

LCMS was conducted on a column (3.3cm x 4.6mm ID, 3µm ABZ+PLUS), at a Flow Rate of 3ml/min, Injection Volume of 5µl, at room temperature and UV Detection Range at 215 to 330nm. Solvent A: 10mM Aqueous ammonium acetate + 0.1% formic acid. Solvent B: 95% Acetonitrile + 0.05% formic acid. Gradient : 0% A/0.7min, 0-100% A/3.5min, 100% A/1.1min, 100-0% A/0.2min.

### Intermediate 1: 3-(5-Amino-4-methyl-2-pyridinyl)-N-cyclopropyl-4-methylbenzamide

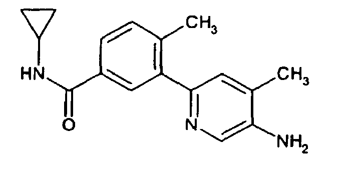

A mixture of 2-chloro-4-methyl-5-nitropyridine (860mg), *N*-cyclopropyl-4-methyl-3-(4,4,5,5-tetramethyl-1,3,2-dioxaborolan-2-yl)benzamide (1.80g), 1M aqueous sodium bicarbonate (32.5ml) and tetrakis(triphenylphosphine)palladium(O) (180mg) in isopropanol (90ml) was stirred at reflux for 76h. A further amount of tetrakis(triphenylphosphine)palladium(0) (100mg) was added and after 15h the reaction mixture was cooled and loaded onto a pre-conditioned (methanol) SCX-2 cartridge. The cartridge was eluted with methanol then 10% aqueous ammonia in methanol. The ammonical fraction was concentrated to give the title compound as a purple gum (949mg).
LC-MS: Rt 1.83min, MH+ 282.

### Intermediate 2: 3-(1-Acetyl-1H-pyrazolo[3,4-c]pyridin-5-yl)-N-cyclopropyl-4-methylbenzamide

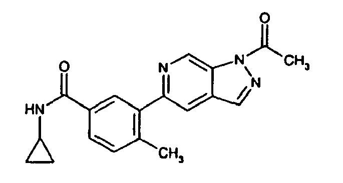

A mixture of 3-(5-amino-4-methyl-2-pyridinyl)-*N-*cyclopropyl-4-methylbenzamide (Intermediate 1, 949mg) in chloroform (20ml) was treated with potassium acetate (331mg) and acetic anhydride (0.623ml). After 20min, t-butylnitrite (0.89ml) and 18-crown-6 (174mg) were added and the reaction mixture was stirred at reflux for 17h. The reaction mixture was cooled, diluted with chloroform and washed with water. The organic layer was separated using a hydrophobic filter tube and concentrated under vacuum. The residue was purified by Biotage chromatography using a cyclohexane/ ethyl acetate gradient to give the title compound as a white foam (129mg).
LC-MS: Rt 2.70min, MH+ 335.

### Intermediate 3: N-Cyclopropyl-4-methyl-3-(1H-pyrazolo[3,4-c]pyridin-5-yl)benzamide

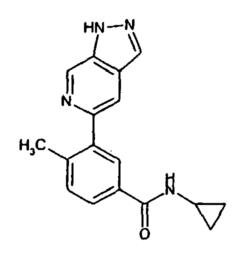

Concentrated hydrochloric acid (100µl) was added to a solution of 3-(1-acetyl-1*H-*pyrazolo[3,4*-c*]pyridin-5-yl)-*N-*cyclopropyl-4-methylbenzamide (Intermediate 2, 126mg) in methanol (5ml) and the reaction mixture was stirred at room temperature for 4h. The methanol was removed under vacuum and the residue was partitioned between chloroform (15ml) and sodium hydroxide (2N, 1ml). The organic phase was washed with water, dried using a hydrophobic filter tube then concentrated under vacuum to the title compound (47.4mg).
LC-MS: Rt 2.33min, MH+ 293.

### Intermediate 4: N-Cyclopropyl-3-fluoro-4-methyl-5-(4-methyl-5-nitropyridin-2-yl)benzamide

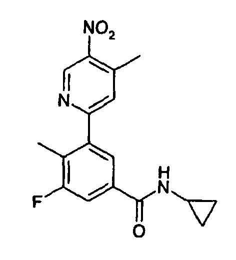

2-Chloro-4-methyl-5-nitropyridine (1.73g), {5-[(cyclopropylamino)carbonyl]-3-fluoro-2-methylphenyl}boronic acid (2.37g), 1 M aqueous sodium hydrogen carbonate solution (65ml) and tetrakis(triphenylphosphine) palladium(0) (360mg) were combined in isopropanol (180ml) and heated at 90°C under nitrogen for 46h. The solvent was evaporated, the residue was partitioned between ethyl acetate and water and the organic layer was dried using a hydrophobic filter tube then concentrated under vacuum. The residue was purified using Biotage chromatography (silca, 2×100g) eluting with 2:1 cyclohexane:ethyl acetate to give the title compound (2.2g).
LC-MS: Rt 3.04min.

### Intermediate 5: 3-(5-Amino-4-methylpyridin-2-yl)-N-cyclopropyl-5-fluoro-4-methylbenzamide

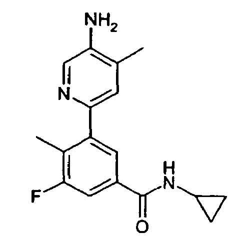

*N-*Cyclopropyl-3-fluoro-4-methyl-5-(4-methyl-5-nitropyridin-2-yl)benzamide (Intermediate 4, 2.2g) in ethanol (200ml) was added to palladium on carbon (10% w/w, 220mg) and stirred under hydrogen at room temperature for 29h. The reaction mixture was filtered through Celite and the solvent was evaporated to give the title compound (2.1g).
LC-MS: Rt 2.01 min.

### Intermediate 6: 3-[5-(Acetylamino)-4-methylpyridin-2-yl]-N-cyclopropyl-5-fluoro-4-methylbenzamide

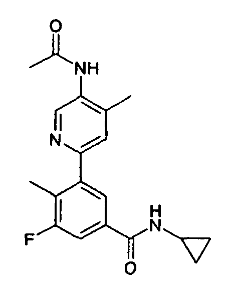

3-(5-Amino-4-methylpyridin-2-yl)-*N-*cyclopropyl-5-fluoro-4-methylbenzamide (Intermediate 5, 180mg), acetic anhydride (113µl) and potassium acetate (82mg) were stirred in chloroform (4ml) under nitrogen at room temperature for 16h. The mixture was partitioned between chloroform and water and the organic layer was separated and dried using a hydrophobic filter tube. The solvent was evaporated to give the title compound as a brown solid (215mg).
LC-MS: Rt 2.43min.

### Intermediate 7: N-Cyclopropyl-3-fluoro-4-methyl-5-(1H-pyrazolo[3,4-c]pyridin-5-yl)benzamide

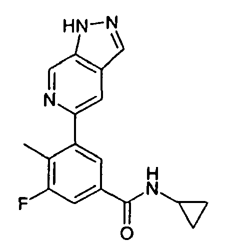

3-[5-(Acetylamino)-4-methylpyridin-2-yl]-*N-*cyclopropyl-5-fluoro-4-methylbenzamide (Intermediate 6, 212mg), t-butyl nitrite (81µl), and 18-crown-6 (33mg) were stirred in chloroform (4ml) under nitrogen at 65°C for 16h. The reaction mixture was partitioned between 1:1 ethyl acetate:chloroform and water, separated and dried using a hydrophobic filter tube and the solvent was evaporated. The residue was purified on an SPE cartridge (silica, 5g) eluting with toluene/ethanol (200:1 to 9:1) to give the title compound as a pale brown solid (66mg).
LC-MS: Rt 2.56min.

### Intermediate 8: 2,5-Dichloro-4-pyridinecarbaldehyde

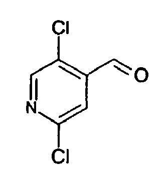

Diisopropylamine (7.5ml) was dissolved in THF (47ml) and the solution was cooled to -35°C. n-Butyl lithium (1.6M in hexanes, 44ml) was added slowly maintaining the temperature below -30°C. After the addition the reaction mixture was cooled to -75°C and a solution of 2,5-dichloropyridine (8.9g) in THF (27ml) was added dropwise. The mixture was stirred at -75°C for a further 30min and a solution of DMF (7.0ml) in THF (14ml) was added dropwise. The reaction mixture was stirred at -75°C for 1.5h then allowed to warm to 10°C over 2.5h. The solution was poured onto a mixture of ice (500ml) and concentrated hydrochloric acid (45ml) and stirred for 15min. The mixture was basified to pH8 with sodium hydroxide (2N), extracted with ether (x3) and the combined organic extracts were washed with brine, dried (magnesium sulfate) and reduced to dryness under vacuum. The resulting oil was applied to a silica column (50g) and eluted with cyclohexane/ethyl acetate (100:0 to 80:20) The product obtained was recrystallised from cyclohexane to give the title compound as beige needles (6.65g).
NMR: [δH d₆-DMSO] 10.20(1H, s), 8.75(1H, s), 7.83(1H, s).

### Intermediate 9: (2,5-Dichloro-4-pyridinyl)methanediyl diacetate

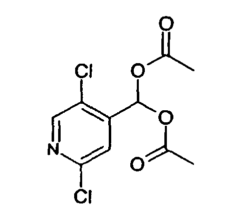

Concentrated sulfuric acid (3 drops) was added to a suspension of 2,5-dichloro-4-pyridinecarbaldehyde (Intermediate 8, 4.0g) in acetic anhydride (25ml) and the mixture was stirred at room temperature for 20h. The acetic anhydride was removed under vacuum to give the title compound as a pale brown oil.
LC-MS: Rt 2.94min, MH+ 278.

### Intermediate 10: 3-(5-Chloro-4-formyl-2-pyridinyl)-N-cyclopropyl-5-fluoro-4-methylbenzamide

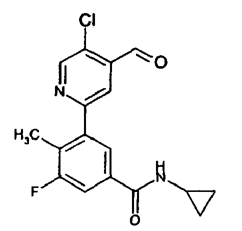

A mixture of (2,5-dichloro-4-pyridinyl)methanediyldiacetate (Intermediate 9 , 1.95g), {5-[(cyclopropylamino)carbonyl]-3-fluoro-2-methylphenyl}boronic acid (1.95g), tetrakis(triphenylphosphine)palladium (0.1g) and aqueous sodiumhydrogen carbonate (1M, 15ml) in isopropanol (30ml) was heated at 85°C for 18h. The reaction mixture was absorbed onto silica and purified on an SPE (silica, 50g) eluting with a cyclohexane/ethyl acetate gradient to give the title compound as an off-white foam (1.1g).
NMR: [δH d₆-DMSO] 10.35(1H, s), 8.99(1H, s), 8.59(1H, b), 7.94(1H, s), 7.77(1H, s), 7.71(1H, d), 2.85(1H, m), 2.25(3H, s), 0.71 (2H, m), 0.58(2H, m).

### Intermediate 11: N-Ethyl-3-fluoro-5-iodo-4-methylbenzamide

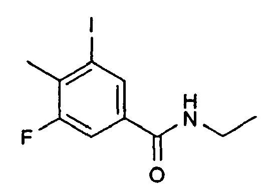

3-Fluoro-5-iodo-4-methylbenzoic acid (intermediate 29, 20g) in thionyl chloride (20ml) was heated at 110°C for 1 h. The excess thionyl chloride was evaporated under vacuum and the residual oil was dissolved in DCM (100ml). Potassium carbonate (21g) was added to the solution followed by the slow addition of ethylamine (2M in THF, 70ml). The reaction was left at room temperature overnight, filtered and the residue was washed with ethyl acetate. The combined filtrate and w ashings w ere r educed to dryness under vacuum a nd the resulting solid was washed with ether/cyclohexane (1:1) to give the title compound as a pale beige solid (18.5g).
NMR: [δH d₆-DMSO] 8.58(1H, b), 8.15(1H, s), 7.64(1H, d), 3.26(2H, quin), 2.33 (3H, s), 1.11 (3H, t).

### Intermediate 12: N-Ethyl-3-fluoro-4-methyl-5-(4,4,5,5-tetramethyl-1,3,2-dioxaborolan-2-yl)benzamide

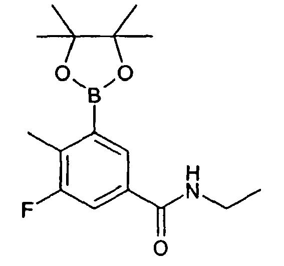

*N-*Ethyl-3-fluoro-5-iodo-4-methylbenzamide (Intermediate 11, 10.9g), bispinnacolcatodiborane (9.9g), Pd(dppf)Cl₂ (600mg) and potassium acetate (17.3g) were mixed in DMF (210ml). The mixture was degassed and then heated at 85°C under nitrogen for 18h. The cooled reaction was absorbed onto silica and applied to a silica column, eluting with an ethyl acetate/cyclohexane gradient (5-25% ethyl acetate). The resultant product was recrystallised from cyclohexane to give the title compound as a white solid (2.83g).
NMR: [δH d₆-DMSO] 8.54(1H, bt), 7.94(1H, s), 7.68(1H, d), 3.27,(2H, quin), 2.42(3H, s), 1.32(12H, s), 1.11 (3H, t).

### Intermediate 13: (4,6-Dichloro-3-pyridiny)[4-(methyloxy)phenyl]methanone

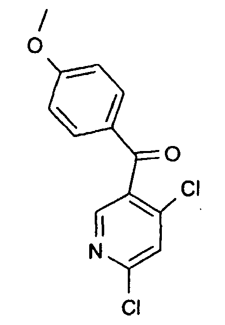

4,6-Dichloro-3-pyridinecarboxylic acid (1.0g) and thionyl chloride (5ml) were heated at 100°C under nitrogen for 4h. Excess thionyl chloride was removed under vacuum and the residue was re-dissolved in anhydrous THF (100ml). Tetrakis(triphenylphosphine)palladium (480mg) and 4-methoxyphenylzinc iodide (0.5M in THF, 33ml) were added to the solution and the mixture was stirred at room temperature for 2h. The solution was treated with aqueous sodium hydrogen carbonate (1M) and extracted with ethyl acetate (x2). The combined organic extracts were washed with brine, dried (magnesium sulphate) and reduced to dryness under vacuum. The residue was absorbed onto silica and applied to a silica column (10g), eluting with an ethyl acetate/cyclohexane gradient (0-100% ethyl acetate). The resultant product was recrystallised from cyclohexane to give the title compound (490mg).
NMR: [δH d₆-DMSO] 8.56(1H, s), 8.02(1H, s), 7.79(2H, d), 7.09(2H, d), 3.87(3H, s).

### Intermediate 14: 3-(4-Chloro-5-{[4-(methyloxy)phenyl]carbonyl}-2-pyridinyl)-N-ethyl-5-fluoro-4-methylbenzamide

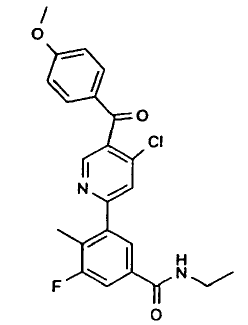

A mixture of (4,6-dichloro-3-pyridinyl)[4-(methyloxy)phenyl]methanone (Intermediate 13, 18mg), *N-*ethyl-3-fluoro-4-methyl-5-(4,4,5,5-tetramethyl-1,3,2-dioxaborolan-2-yl)benzamide (Intermediate 12, 20mg), tetrakis(triphenylphosphine)palladium (1.3mg) and aqueous sodium hydrogen carbonate (1M, 0.2ml) were mixed in isopropanol (0.6ml) and heated at 90°C for 1 h. The cooled reaction was partitioned between ethyl acetate and water and the organic phase was reduced to dryness under vacuum to give impure title compound as a yellow oil (30mg).
LC-MS: Rt 3.39min, MH+ 427.

### Intermediate 15: (2,6-Dichloro-3-pyridinyl)[4-(methyloxy)phenyl]methanone

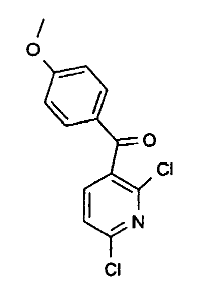

2,6-Dichloronicotinic acid (2g) in thionyl chloride (5ml) was heated at reflux for 2h, then left to cool to room temperature. The excess thionyl chloride was evaporated under vacuum and the resulting yellow oil was dissolved in anhydrous THF (10ml). Tetrakis(triphenylphosphine)palladium (120mg) and 4-methoxyphenylzinc iodide (0.5M, 17ml, Aldrich) were added to the solution which was stirred at room temperature for 20min. The reaction mixture was partitioned between ether and aquous saturated ammonium solution and the organic phase was washed with ammonium chloride and brine. The solution was dried through a hydrophobic filter tube then concentrated under vacuum to give the title compound as an orange oil.
NMR: [δH d₆-DMSO] 8.09(1H, d), 7.77(3H, m), 7.09(2H, d), 3.87(3H, s).

### Intermediate 16: 3-(6-Chloro-5-{[4-(methyloxy)phenyl]carbonyl}-2-pyridinyl)-N-ethyl-5-fluoro-4-methylbenzamide

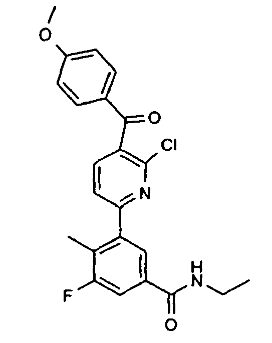

(2,6-Dichloro-3-pyridinyl)[4-(methyloxy)phenyl]methanone (Intermediate 15, 36mg), *N-*ethyl-3-fluoro-4-methyl-5-(4,4,5,5-tetramethyl-1,3,2-dioxaborolan-2-yl)benzamide (Intermediate 12, 40mg), tetrakis(triphenylphosphine)palladium (2.6mg) and aqueous sodium hydrogen carbonate (1M, 0.4ml) were mixed in isopropanol (1.2ml) and heated at 90°C for 2h. The cooled reaction mixture was partitioned between ethyl acetate and water and the organic phase was reduced to dryness under vacuum. The residue was dissolved in ethyl acetate/cyclohexane (1:1) and filtered through a silica column (1g) washing with more ethyl acetate/cyclohexane (1:1). The filtrate was concentrated under vacuum to give the title compound as a yellow oil (40mg).
LC-MS: Rt 3.41min, MH+427.

### Intermediate 17: (4,6-Dichloro-3-pyridinyl)(4-fluorophenyl)methanone

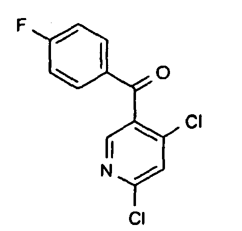

4,6-Dichloro-3-pyridinecarboxylic acid (220mg) in thionyl chloride (1ml) was heated at 100°C for 1.5h. Excess thionyl chloride was removed under vacuum and the residue was dissolved in dry THF (5ml). Tetrakis(triphenylphosphine)palladium (7mg) and 4-fluorophenylzinc bromide (0.5M in THF, 2.6ml) were added to the solution which was stirred at room temperature for 1.5h. The reaction mixture was treated with saturated ammonium chloride solution, extracted with ether (x2) and the organic phase was concentrated under vacuum. The residue was applied to a silica column (5g) and eluted with an ethyl acetate/cyclohexane gradient (0-18% ethyl acetate) to give the title compound as a colourless oil (130mg).
LC-MS: Rt 3.28min, MH+ 270/272/274.

### Intermediate 18: 3-{4-Chloro-5-[(4-fluorophenyl)carbonyl]-2-pyridinyl}-N-ethyl-5-fluoro-4-methylbenzamide

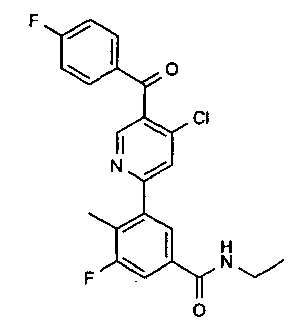

(4,6-Dichloro-3-pyridinyl)(4-fluorophenyl)methanone (Intermediate 17, 49mg), *N-*ethyl-3-fluoro-4-methyl-5-(4,4,5,5-tetramethyl-1,3,2-dioxaborolan-2-yl)benzamide (Intermediate 12, 56mg), tetrakis(triphenylphosphine)palladium (3mg) and aqueous sodium hydrogen carbonate (1M, 0.5ml) were mixed in isopropanol (1.5ml) and heated at 90°C for 1.5h. The solvent was removed under vacuum and the residue was dissolved in ethyl acetate/cyclohexane (1:1). The solution was applied to a silica column (1g) and eluted with more ethyl acetate/cyclohexane (1:1) to give the title compound as a greenish yellow oil (62mg).
LC-MS: Rt 3.43min, MH+ 415.

### Intermediate 19: 4-[(4,6-Dichloro-3-pyridinyl)carbonyl]benzonitrile

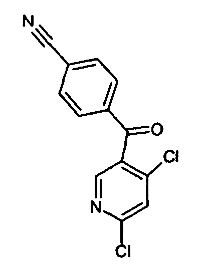

4,6-Dichloro-3-pyridine carboxylic acid (3.2g) in thionyl chloride (15ml) was heated at 100°C for 2h. Excess thionyl chloride was removed under vacuum, the residual oil was dissolved in dry THF (32ml) and tetrakis(triphenylphosphine)palladium (80mg) was added to the solution. An aliquot (2ml) of the resulting mixture were stirred with 4-cyanophenylzinc bromide (0.5M in THF, 2.3ml) for 3h at room temperature. The mixture was treated with aqueous ammonium chloride, extracted with DCM and the organic phase was concentrated under a stream of nitrogen. The residue was dissolved in ethyl acetate/methanol and applied to an aminopropyl SPE cartridge (0.5g) washing with further ethyl acetate/methanol. The combined washings were concentrated under a stream of nitrogen and the residue was re-dissolved in ethyl acetate/cyclohexane (1:1). The solution was applied to a silica column (1g) and eluted with further ethyl acetate/cyclohexane. Precipitated material deposited on top of the column provided the title compound as a cream solid.
LC-MS: Rt 3.07min.

### Intermediate 20: (2,6-Dichloro-3-pyridinyl)(4-fluorophenyl)methanone

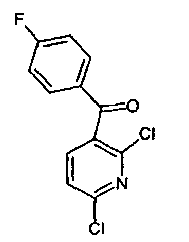

A mixture of 2,6-dichloronicotinic acid (2g) and thionyl chloride (5ml) was heated at 75°C for 1.75hrs and then at 90°C for 0.5hrs. Excess thionyl chloride was removed under vacuum and the residue was dissolved in dry THF (20ml). Tetrakis(triphenylphosphine)palladium (120mg) was added followed by 4-fluorophenylzincbromide (0.5M in THF, 20.8ml) and the mixture was stirred at room temperature for 1h. The reaction mixture was treated with saturated ammonium chloride solution then extracted with ethyl acetate. The organic phase was washed with brine, dried (MgSO₄) and concentrated under vacuum. The residue was applied to a silica column (50g) and eluted with an ethyl acetate/cyclohexane gradient (0-13% ethyl acetate) to give the title compound as a pale yellow oil.
LC-MS: Rt 3.22min, MH+ 270, 272.

### Intermediate 21: 3-{6-Chloro-5-[(4-fluorophenyl)carbonyl]-2-pyridinyl}-N-ethyl-4-methylbenzamide

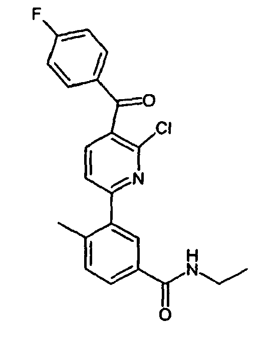

A mixture of (2,6-dichloro-3-pyridinyl)(4-fluorophenyl)methanone (Intermediate 20, 80mg), *N-*ethyl-4-methyl-3-(4,4,5,5-tetramethyl-1,3,2-dioxaborolan-2-yl)benzamide (Intermediate 28, 70mg), tetrakis(triphenylphosphine)palladium (6mg) and aqueous sodium hydrogen carbonate (1M, 0.5ml) in isopropanol (2ml) was degassed then heated at 90°C under nitrogen for 2h. The reaction was absorbed onto silica, applied to a silica column (5g) and eluted with an ethyl acetate/cyclohexane gradient (0-100% ethyl acetate) to give the title compound as a colourless oil (55mg).
LC-MS: Rt 3.36min, MH+ 397, 399.

### Intermediate 22: 3-{6-Chloro-5-[(4-fluorophenyl)carbonyl]-2-pyridinyl}-N-cyclopropyl-5-fluoro-4-methylbenzamide

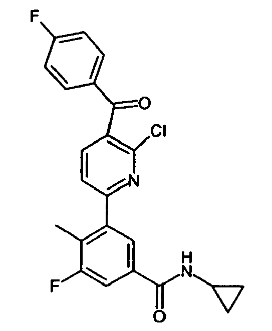

A mixture of (2,6-Dichloro-3-pyridinyl)(4-fluorophenyl)methanone (Intermediate 20, 270mg), {5-[(cyclopropylamino)carbonyl]-3-fluoro-2-methylphenyl}boronic acid (250mg), tetrakis(triphenylphosphine)palladium (23mg) and 1M aqueous sodium hydrogen carbonate (3ml) in isopropanol (9ml) was degassed a nd heated at 90°C for 1.5h. The mixture was concentrated under vacuum and the residue was treated with ethyl acetate and applied to a silica column (1g). The column was washed with ethyl acetate and the solvent was removed under vacuum to give the title compound as a yellow foam.
LC-MS: Rt 3.52min. MH⁺ 427, 429.

### Intermediate 23: 3-Fluoro-5-iodo-4-methyl-N-(1-methyl-1H-pyrazol-5-yl)benzamide

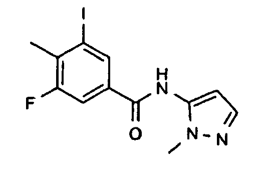

3-Fluoro-5-iodo-4-methylbenzoic acid (Intermediate 29, 6.27g) in thionyl chloride (10ml) was heated at 110°C for 2.5h. The reaction was left to cool to room temperature overnight and the excess thionyl chloride was removed under vacuum. A portion (2.18g) of the crude acid chloride in DCM (10ml) was treated with potassium carbonate (1.7g) followed by a solution of 5-amino-1-methylpyrazole (0.94g) in DCM (10ml). The mixture was stirred overnight then concentrated under vacuum. The residue was washed with water then triturated with cyclohexane to give the title compound as a cream solid (1.44g).
LC-MS: Rt 3.08min, MH+ 360.

### Intermediate 24: 3-Fluoro-4-methyl-N-(1-methyl-1H-pyrazol-5-yl)-5-(4,4,5,5-tetramethyl-1,3,2-dioxaborolan-2-yl)benzamide

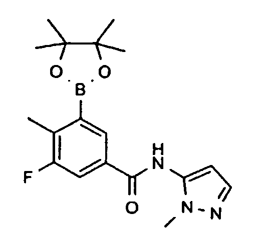

3-Fluoro-5-iodo-4-methyl-*N-*(1-methyl-1*H-*pyrazol-5-yl)benzamide (Intermediate 23, 1.44g) bispinnacolatodiborane (3.05g) Pd(dppf)Cl2 (59mg) and potassium acetate (1.19g) were combined in DMF (34ml), divided between 2 vials then heated by microwave in sealed vessels at 150°C for 15min. The mixtures were combined, absorbed onto silica and applied to silica columns (2 x 100g). The columns were eluted with an ethyl acetate/cyclohexane gradient to give the title compound as an off-white solid (217mg).
LCMS: Rt 3.25min, MH+ 360.

### Intermediate 25: 3-{6-Chloro-5-[(4-fluorophenyl)carbonyl]-2-pyridinyl}-5-fluoro-4-methyl-N-(1-methyl-1H-pyrazol-5-yl)benzamide

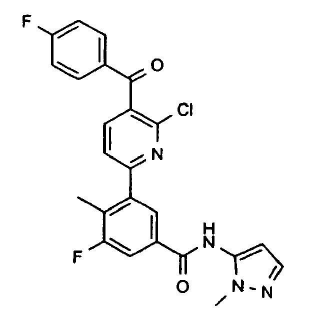

A mixture of (2,6-dichloro-3-pyridinyl)(4-fluorophenyl)methanone (Intermediate 20, 85mg) 3-fluoro-4-methyl-*N-*(1-methyl-1*H-*pyrazol-5-yl)-5-(4,4,5,5-tetramethyl-1,3,2-dioxaborolan-2-yl)benzamide (Intermediate 24, 100mg) tetrakis(triphenylphosphine)palladium (6mg) and 1M aqueous sodiumhydrogen carbonate (0.84ml) in isopropanol (2ml) was degassed and heated at 85°C for 2h. The reaction mixture was absorbed onto silica, applied to a silica column (2g) and eluted with a cyclohexane/ethyl acetate gradient (20-70% ethyl acetate) to give the title compound as a white foam (56mg).
LC-MS: Rt 3.37min, MH+ 467, 469.

### Intermediate 26: 3-{4-Chloro-5-[(4-fluorophenyl)carbonyl]-2-pyridinyl}-5-fluoro-4-methyl-N-(1-methyl-1H-pyrazol-5-yl)benzamide

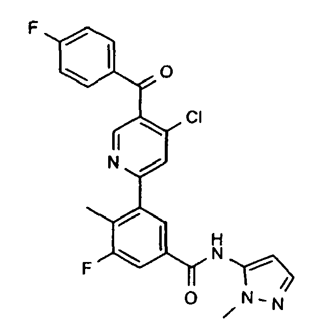

A mixture of (4,6-dichloro-3-pyridinyl)(4-fluorophenyl)methanone (Intermediate 17, 100mg), 3-fluoro-4-methyl-*N-*(1-methyl-1*H-*pyrazol-5-yl)-5-(4,4,5,5-tetramethyl-1,3,2-dioxaborolan-2-yl)benzamide (Intermediate 24, 100mg), tetrakis(triphenylphosphine)palladium (6mg) and aqueous sodium hydrogen carbonate (1M, 0.84ml) in isopropanol (2ml) was heated at 90°C for 1.5h. The reaction mixture was absorbed onto silica, applied to a silica column (5g) and eluted with a cyclohexane/ethyl acetate gradient (4-50% ethyl acetate) to give the title compound as a pale yellow foam.
LC-MS: Rt 3.40min, MH+ 467, 469.

### Intermediate 27: 3-(6-Chloro-5-cyano-2-pyridinyl)-N-cyclopropyl-4-methylbenzamide

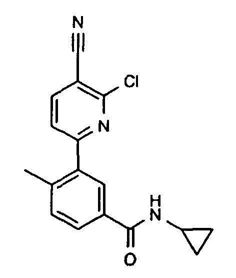

A mixture of *N-*cyclopropyl-4-methyl-3-(4,4,5,5-tetramethyl-1,3,2-dioxaborolan-2-yl)benzamide (860mg), 2,6-dichloro-3-pyridinecarbonitrile (1.51g) tetrakis(triphenylphosphine)palladium(0) (100mg) and aqueous sodium bicarbonate (1M, 15ml) in isopropanol (45ml) was heated at reflux under nitrogen for 3h. On cooling the reaction mixture was concentrated under vacuum and partitioned between ethyl acetate (150ml) and aqueous sodium bicarbonate (1M, 100ml). The aqueous phase was re-extracted with ethyl acetate (100ml) and the combined organic extracts were washed with brine (100ml), dried (Na₂SO₄) and concentrated under vacuum to give a crude product. This was dissolved in dichloromethane and applied to an SPE cartridge (silica, 50g) eluting with an ethyl acetate/DCM gradient (0 to 100%) to give the title compound as a white solid (610mg). LC-MS: Rt 3.04min.

### Intermediate 28: N-Ethyl-4-methyl-3-(4,4,5,5-tetramethyl-1,3,2-dioxaborolan-2-yl)benzamide

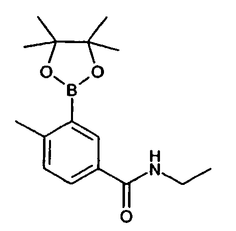

4-Methyl-3-(4,4,5,5-tetramethyl-1,3,2-dioxaborolan-2-yl)benzoic acid (1.3g) 1-(3-dimethylaminopropyl)-3-ethylcarbodiimide hydrochloride (1.44g) and 3*H-*[1,2,3] 1-hydroxy-7-azabenzotriazole (0.077g) was added to a stirred solution of ethylamine in tetrahydrofuran (2M, 5ml) in chloroform (30ml) and the mixture was stirred for 24h. The mixture was poured into water, passed through a hydrophobic filter tube and the organic phase was concentrated under vacuum.. The residual solid was purified on an SCX cartridge eluting with methanol to give the title compound as a pale yellow solid (1.36g).
LC-MS: Rt 3.2min.

### Intermediate 29: 3-Fluoro-5-Iodo-4-methylbenzoic acid

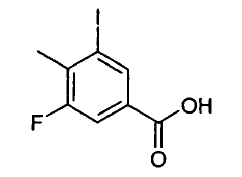

A solution of 3-fluoro-4-methylbenzoic acid (149.7g) in trifluoromethanesulphonic acid (1050ml) at -22°C under nitrogen was treated portionwise over 1.25h with iodosuccinimide (203.5g). The mixture was stirred at -20°C for and further portions of iodosuccinimide were added after 2.5h (46.5g) and 20.5h (30g). The mixture was stirred at -20°C for a further 24h then added slowly to a mixture of aqueous sodium thiosulphate (10%, 1.5L) and ice (3kg). The resultant precipitate was collected by filtration and stirred with ethyl acetate (5L) and aqueous sodium thiosulphate (10%, 1.5L). The organic phase was dried (MgSO₄) and concentrated to -1.5L then left overnight. The precipitate was collected by filtration and further material was obtained through concentration of the filtrate to give the title compound as a white solid (133.9g).
LC-MS: Rt 3.60, MH+ 281.

### Example 1: N-Cyclopropyl-4-methyl-3-{1-[(1-methylethyl)sulfonyl]-1H-pyrazolo[3,4-c]pyridin-5-yl}benzamide

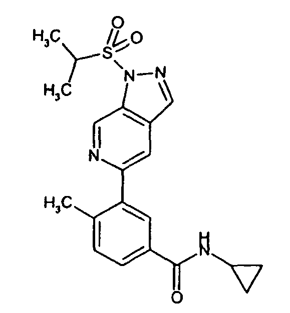

Sodium hydride (60% in mineral oil, 11mg) was added to a solution of *N-*cyclopropyl-4-methyl-3-(1*H-*pyrazolo[3,4*-c*]pyridin-5-yl)benzamide (Intermediate 3, 40mg) in DMF (1.5ml) and the reaction mixture was stirred at room temperature for 5min. A solution of isopropylsulfonylchloride (58.7mg) in DMF (0.25ml) was added to the solution and the mixture was stirred at room temperature for 2h. Water was added to quench the reaction and the mixture was partitioned between water and chloroform. The organic phase was reduced to dryness under vacuum and the residue was purified by preparative HPLC to give the title compound (2.8mg).
LC-MS: Rt 2.90min, MH+ 399.

### Example 2: N-Cyclopropyl-4-methyl-5-[1-(2-thienylsulfonyl)-1H-pyrazolo[3,4-c]pyridin-5-yl]benzamide

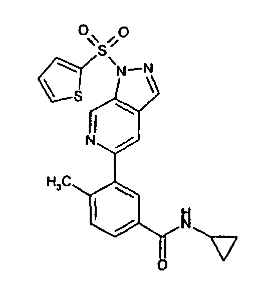

The procedure for Example 1 was followed using sodium hydride (60% in mineral oil, 13mg), *N-*cyclopropyl-4-methyl-3-(1*H-*pyrazolo[3,4*-c*]pyridin-5-yl)benzamide (Intermediate 3, 47mg), 2-thiophenesulfonylchloride (58.7mg) and DMF (2.25ml) to give the title compound (17mg).
LC-MS: Rt 3.09min, MH+ 439.

### General Method 1

*N-*Cyclopropyl-3-fluoro-4-methyl-5-(1*H-*pyrazolo[3,4*-c*]pyridin-5-yl)benzamide (Intermediate 7, 50mg) was dissolved in DMF (2ml). Sodium hydride (60% dispersion in mineral oil, 7mg) and the sulfonyl chloride (1.1eq) were added and the reaction mixture was stirred at room temperature under nitrogen for 18h. Water was added and the mixture was extracted with 1:1 ethyl acetate:chloroform then separated and dried using a hydrophobic filter tube. The solvent was evaporated and residue was purified using (A) an SPE cartridge (silica, 2g) eluting with a methanol/chloroform gradient (B) reverse phase preparative HPLC or (C) Biotage Chromatography (silica, 10g, methanol/chloroform gradient).

### Example 3: N-Cyclopropyl-3-fluoro-4-methyl-5-[1-(2-thienylsulfonyl)-1H-pyrazolo[3,4-c]pyridin-5-yl]benzamide

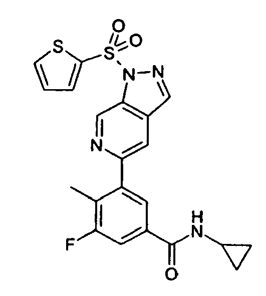

General Method 1 was followed using 2 -thiophenesulfonyl chloride to give the title compound as a colourless gel (7.5mg).
LC-MS: Rt 3.24min, MH⁺ 457.

### Example 4: N-Cyclopropyl-3-fluoro-4-methyl-5-[1-(phenylsulfonyl)-1H-pyrazolo[3,4-c]pyridin-5-yl]benzamide

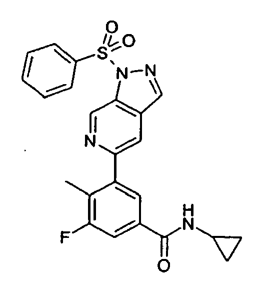

General Method 1 was followed using phenylsulfonyl chloride to give the title compound as a yellow gel (14mg).
LC-MS: Rt 3.34min, MH⁺ 451.

### Example 5: N-Cyclopropyl-3-fluoro-4-methyl-5-[1-(3-thienylsulfonyl)-1H-pyrazolo[3,4-c]pyridin-5-yl]benzamide

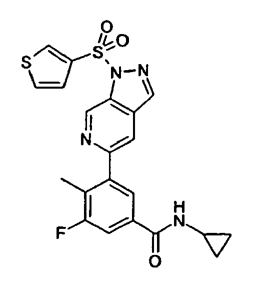

General Method 1 was followed using 3-thiophenesulfonyl chloride to give the title compound as a colourless gel (17.8mg).
LC-MS: Rt 3.27min, MH⁺ 457.

### Example 6: N-Cyclopropyl-3-fluoro-4-methyl-5-[3-(3-thienylsulfonyl)-1H-pyrazolo[3,4-c]pyridin-5-yl]benzamide

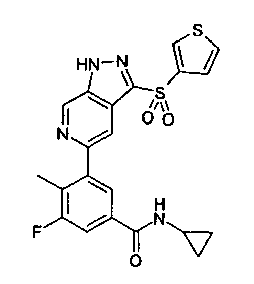

General Method 1 w as followed u sing 3 -thiophenesulfonyl chloride to give the title compound as a colourless gel (2.8mg).
LC-MS: Rt 3.34min, MH⁺ 457.

### Example 7: N-Cyclopropyl-3-fluoro-4-methyl-5-[1-(propylsulfonyl)-1H-pyrazolo[3,4-c]pyridin-5-yl]benzamide

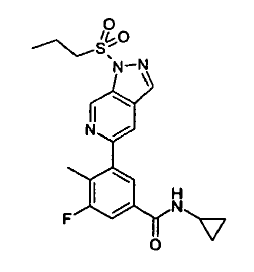

General Method 1 was followed using n-propylsulfonyl chloride to give the title compound as a colourless gel (3.3mg).
LC-MS: Rt 3.10min, MH⁺ 417.

### Example 8: N-Cyclopropyl-3-fluoro-4-methyl-5-{1-[(1-methylethyl)sulfonyl]-1H-pyrazolo[3,4-c]pyridin-5-yl}benzamide

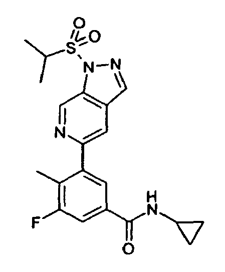

General Method 1 was followed using isopropylsulfonyl chloride to give the title compound as a colourless gel (13.3mg).
LC-MS: Rt 3.06min, MH⁺ 417.

### Example 9: N-Cyclopropyl-3-[1-(cyclopropylsulfonyl)-1H-pyrazolo[3,4-c]pyridin-5-yl]-5-fluoro-4-methylbenzamide

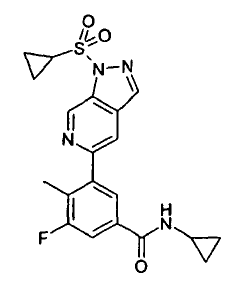

General Method 1 was followed using cyclopropylsulfonyl chloride to give the title compound (6.4mg).
LC-MS: Rt 3.02min, MH⁺ 415.

### Example 10: N-Cyclopropyl-3-fluoro-4-methyl-5-[1-(3-methylphenyl)-1H-pyrazolo[3,4-c]pyridin-5-yl]benzamide

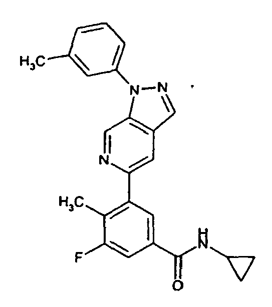

A mixture of 3-(5-chloro-4-formyl-2-pyridinyl)-*N-*cyclopropyl-5-fluoro-4-methylbenzamide (Intermediate 10, 33mg), (3-methylphenyl)hydrazine (2 drops), sodium t-butoxide (13mg), palladium (II) acetate (1mg) and +/- BINAP (3mg) in toluene (1ml) was heated in a sealed vessel at 100°C for 6h. The reaction mixture was concentrated and applied directly to an SPE cartridge (silica, 10g) and eluted with a cyclohexane/ethylacetate gradient. The crude product obtained was further purified by preparative HPLC to give the title compound as a yellow glass (5mg).
LC-MS: Rt 3.39min, MH+ 401.

### Example 11: N-Cyclopropyl-4-methyl-5-(1-phenyl-1H-pyrazolo[3,4-c]pyridin-5-yl)benzamide

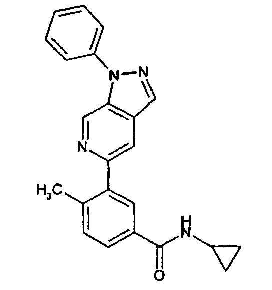

The procedure for Example 10 was followed using 3-(5-chloro-4-formyl-2-pyridinyl)-4-methyl-*N-*(1-methylpropyl)benzamide (Intermediate 10, 100mg), palladium II acetate (2.5mg), (+/-)BINAP (5mg), phenylhydrazine (0.038ml) and sodium t-butoxide (46mg) in toluene (1.6ml) to give the title compound (35.5mg).
LC-MS: Rt 3.16min, MH+ 369.

### Example 12: N-cyclopropyl-3-fluoro-4-methyl-5-[1-(3-methylphenyl)-6-oxido-1H-pyrazolo[3,4-c]pyridin-5-yl]benzamide

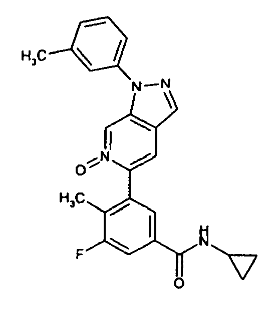

m-CPBA (15mg) was added to a solution of *N-*cyclopropyl-3-fluoro-4-methyl-5-[1-(3-methylphenyl)-1*H-*pyrazolo[3,4*-c*]pyridin-5-yl]benzamide (Example 10, 5mg) in chloroform (1ml) at 60°C. The reaction was maintained at 60°C for 1.25h, allowed to cool and diluted with methanol. The solution was eluted through an SPE cartridge (aminopropyl, 1g) and the solvent was removed under vacuum to give the title compound as a yellow glass (3.2mg).
LC-MS: Rt 2.93min, MH+ 417.

### Example 13: N-Cyclopropyl-3-[1-(2-fluorophenyl)-1H-pyrazolo[3 4-c]pyridin-5-yl]-4-methylbenzamide

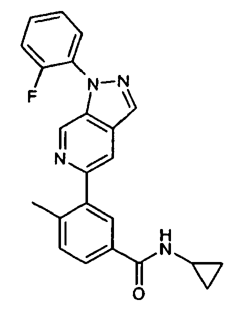

The procedure for Example 10 was followed using 3-(5-chloro-4-formyl-2-pyridinyl)-4-methyl-*N-*(1-methylpropyl)benzamide (Intermediate 10, 100mg), palladium II acetate (1mg), (+/-)BINAP (3mg), 2-fluorophenylhydrazine (0.63mg) and sodium t-butoxide (83mg) in toluene (3.5ml) to give the title compound as a bronze coloured glass (17mg).
LC-MS: Rt 3.05min, MH+ 387.

### Example 14: N-Cyclopropyl-4-methyl-3-(6-oxido-1-phenyl-1H-pyrazolo[3,4-c]pyridin-5-yl)benzamide

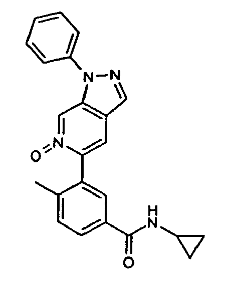

The procedure for Example 12 was followed using *N-*cyclopropyl-4-methyl-5-(1-phenyl-1*H-*pyrazolo[3,4*-c*]pyridin-5-yl)benzamide (Example 11, 38mg), m-CPBA (30mg) and chloroform (5ml) to give the title compound as a yellow solid (33mg).
LC-MS: Rt 2.65min, MH+ 385.

### Example 15: N-Cyclopropyl-3-[1-(2-fluorophenyl)-6-oxido-1H-pyrazolo[3,4-c]pyridin-5-yl]-4-methylbenzamide

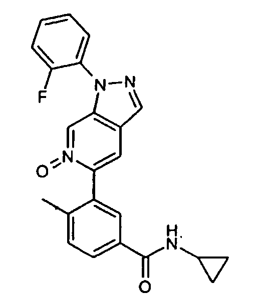

The procedure for Example 12 was followed using *N-*cyclopropyl-3-[1-(2-fluorophenyl)-1*H-*pyrazolo[3,4*-c*]pyridin-5-yl]-4-methylbenzamide (Example 13, 8mg), m-CPBA (6mg) and chloroform (2ml) to give the title compound as an off-white solid (8mg).
LC-MS: Rt 2.59min, MH+ 403.

### Example 16: N-Ethyl-3-fluoro-4-methyl-5-{3-[4-(methyloxy)phenyl]-1H-pyrazolo[4,3-c]pyridin-6-yl}benzamide

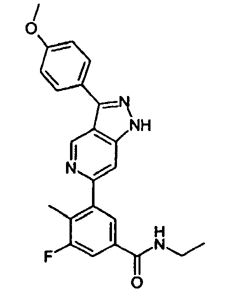

3-(4-Chloro-5-{[4-(methyloxy)phenyl]carbonyl}-2-pyridinyl)-*N-*ethyl-5-fluoro-4-methylbenzamide (Intermediate 14, 30mg) and hydrazine hydrate (0.2ml) were heated at 60°C in THF (1.5ml) for 1.5h. A further 0.2ml of hydrazine hydrate was added and heating continued for 0.5h. The resulting solution was partitioned between ethyl acetate and water and the organic phase reduced to dryness under vacuum. The residue was applied to a silica column (5g) and eluted with an ethyl acetate/cyclohexane gradient (0-100% ethyl acetate) followed by methanol/ethyl acetate (1:9). The resultant product was triturated with ether to give the title compound as a cream solid.
LC-MS: Rt 2.97min, MH+ 405.

### Example 17: N-Ethyl-3-fluoro-4-methyl-5-{3-[4-(methyloxy)phenyl]-1H-pyrazolo[3,4-b]pyridin-6-yl}benzamide

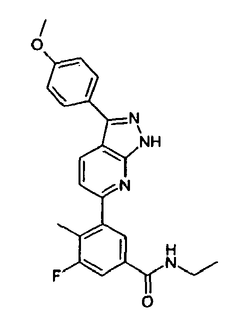

3-(6-Chloro-5-{[4-(methyloxy)phenyl]carbonyl}-2-pyridinyl)-*N-*ethyl-5-fluoro-4-methylbenzamide (Intermediate 16, 40mg) and hydrazine hydrate (0.5ml) were mixed in THF (1ml) and heated at 60°C for 1h. The reaction was left at room temperature overnight and then heated at 60°C for a further 5.5h. The cooled reaction was partitioned between DCM and water. The organic phase was dried through a hydrophobic filter tube and reduced to dryness under vacuum. The residue was triturated with ether/ethyl acetate to give the title compound as a white solid (13mg).
LC-MS: Rt 3.32min, MH+ 405.

### Example 18: N-Ethyl-3-fluoro-5-[3-(4-fluorophenyl)-1H-pyrazolo[4,3-c]pyridin-6-yl]-4-methylbenzamide

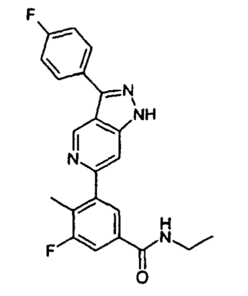

3-{4-Chloro-5-[(4-fluorophenyl)carbonyl]-2-pyridinyl}-*N-*ethyl-5-fluoro-4-methylbenzamide (Intermediate 18, 62mg) and hydrazine hydrate (0.2ml) in THF (1.5ml) were heated at 60°C for 1.75h then left at room temperature overnight. The reaction mixture was partitioned between dichloromethane and water and the organic phase was dried through a hydrophobic filter tube and concentrated under vacuum The residue was triturated with ether/ethyl acetate to give the title compound as an off-white solid (30mg).
LC-MS: Rt 3.05min, MH+ 393.

### Example 19: Ethyl 4-(6-{5-[(ethylamino)carbonyl]-2-methylphenyl}-1H-pyrazolo[3,4-b]pyridin-3-yl)benzoate

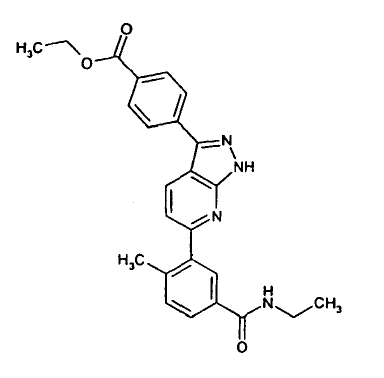

2,6-Dichloronicotinic acid (3.4g, Aldrich) in thionyl chloride (15ml) was heated at 100°C for 2h. The excess thionyl chloride was removed under vacuum, the residual oil was dissolved in dry THF (36ml) and tetrakis(triphenylphosphine)palladium (90mg) was added to the solution. An aliquot (2ml) of the resulting mixture was treated with (4-ethoxycarbonyl)phenylzinc iodide, 0.5M in T HF, 2.3ml, A Idrich) and stirred for 3h at room temperature. The reaction mixture was treated with aqueous ammonium chloride, extracted with DCM and the organic layer was filtered through a silica column (2g). The solvent was evaporated and the residues was dissolved in ethyl acetate/methanol and passed through an aminopropyl SPE cartridge (1g) washing with further ethyl acetate/methanol. The combined filtrate and washings were reduced to dryness under a stream of nitrogen to give the crude ketone intermediate which was used without further purification.

The ketone was mixed with *N-*ethyl-4-methyl-3-(4,4,5,5-tetramethyl-1,3,2-dioxaborolan-2-yl)benzamide (Intermediate 28, 20.6mg), tetrakis(triphenylphosphine)palladium (-1 mg), aqueous sodium hydrogen carbonate (1M, 0.21ml) and isopropanol (1ml) then heated at 90°C under nitrogen for 1.5h. The reaction was allowed to cool, diluted with ethyl acetate, and the solution was filtered through a silica column (0.5g). The filtrate was reduced to dryness under a stream of nitrogen and the residue was used without further purification.

The crude intermediate was dissolved in in THF (1 ml), hydrazine hydrate (0.1 ml) was added and the solution was heated at 60°C for 4h. The reaction mixture was allowed to cool, partitioned between chloroform and water and the organic phase was applied to an SCX SPE cartridge (1g). The cartridge was eluted with methanol followed by a solution of ammonia (0.88) in methanol (10%). The solvent was evaporated and the crude residue was purified on a silica column (1g) eluting sequentially with cyclohexane/ether, ether, ethyl acetate and methanol. The resultant product was triturated with ether to give the title compound as a cream solid.
LC-MS: Rt 3.42min, MH+ 429.

### Example 20: 3-[3-(4-Chloro-3-fluorophenyl)-1H-pyrazolo[3,4-b]pyridin-6-yl]-N-ethyl-4-methylbenzamide

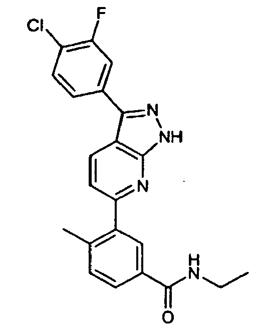

The title compound was prepared in an analogous manner to Example 19, replacing (4-(ethoxycarbonyl)phenylzinc iodide with 4-chloro-3-fluorophenylzinc iodide to give a cream solid.
LC-MS: Rt 3.56min, MH+ 409.

### Example 21: N-Ethyl-3-[3-(3-fluorophenyl)-1H-pyrazolo[3,4-b]pyridin-6-yl]-4-methylbenzamide

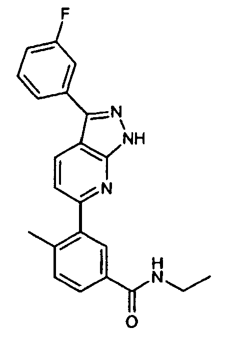

The title compound was prepared in an analogous manner to Example 19, replacing (4-(ethoxycarbonyl)phenylzinc iodide with 3-fluorophenylzinc iodide to give a cream solid. LC-MS: Rt 3.33min, MH+ 375.

### Example 22: 3-[3-(4-Cyanophenyl)-1H-pyrazolo[3,4-b]pyridin-6-yl]-N-ethyl-4-methylbenzamide

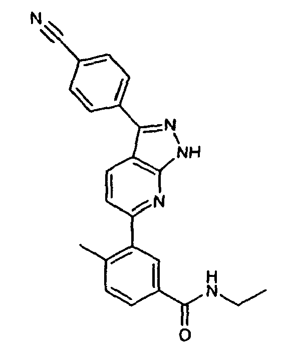

The title compound was prepared in an analogous manner to Example 19, replacing (4-(ethoxycarbonyl)phenylzinc iodide with 4-cyanophenylzinc iodide to give a cream solid.
LC-MS: Rt 3.15min, MH+ 382.

### Example 23: N-Ethyl-4-methyl-3-{3-[3-(methyloxy)phenyl]-1H-pyrazolo[3,4-b]pyridin-6-yl}benzamide

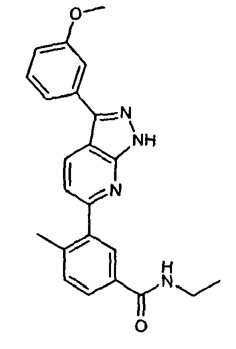

The title compound was prepared in an analogous manner to Example 19, replacing (4-(ethoxycarbonyl)phenylzinc iodide with 3-methoxyphenylzinc iodide to give a cream solid to give a cream solid.
LC-MS: Rt 3.87min, MH+ 387.

### Example 24: N-Ethyl-3-[3-(3-fluoro-4-methylphenyl)-1H-pyrazolo[3,4-b]pyridin-6-yl]-4-methylbenzamide

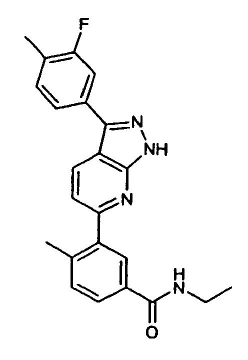

The title compound was prepared in an analogous manner to Example 19, replacing (4-(ethoxycarbonyl)phenylzinc iodide with 3-fluoro-4-methylphenylzinc iodide to give a cream solid.
LC-MS: Rt 3.44min, MH+ 389.

### Example 25: 3-[3-(4-Chlorophenyl)-1H-pyrazolo[3,4-b]pyridin-6-yl]-N-ethyl-4-methylbenzamide

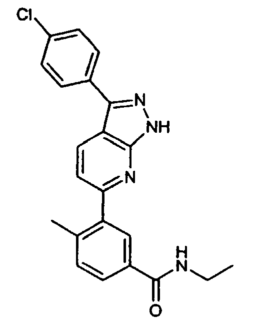

The title compound was prepared in an analogous manner to Example 19, replacing (4-(ethoxycarbonyl)phenylzinc iodide with 4-chlorophenylzinc iodide.
LC-MS: Rt 3.47min, MH+ 391.

### Example 26: 3-{3-[(3,4-Difluorophenyl)methyl]-1H-pyrazolo[4,3-c]pyridin-6-yl}-N-ethyl-4-methylbenzamide

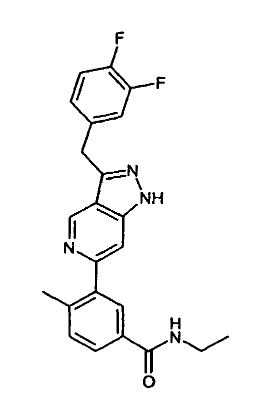

4,6-Dichloro-3-pyridine carboxylic acid (3.2g) in thionyl chloride (15ml) was heated at 100°C for 2h. The excess thionyl chloride was removed under vacuum, the residual oil was dissolved in dry THF (32ml) and tetrakis(triphenylphosphine)palladium (80mg) was added to the solution. An aliquot (2ml) of the resulting mixture was treated with 3,4-difluorobenzylzinc iodide (0.5M in THF, 2.3ml) then stirred for 3h at room temperature. The mixture was treated with aqueous ammonium chloride, extracted with DCM and the organic phase was concentrated under a stream of nitrogen. The residue was dissolved in ethyl acetate/methanol and filtered through an aminopropyl SPE cartridge (0.5g) washing with further ethyl acetate/methanol. The combined washings were concentrated under a stream of nitrogen and the residue was re-dissolved in ethyl acetate/cyclohexane (1:1). The solution was applied to a silica column (1g), eluting with more ethyl acetate/cyclohexane. The solvent was removed under a stream of nitrogen and the crude ketone intermediate was used without further purification.

The ketone was mixed with *N-*ethyl-4-methyl-3-(4,4,5,5-tetramethyl-1,3,2-dioxaborolan-2-yl)benzamide (Intermediate 28, 20.6mg), tetrakis(triphenylphosphine)palladium (∼1mg), aqueous sodium hydrogen carbonate (1M, 0.21 ml) and isopropanol (1ml) then heated at 90°C under nitrogen for 1.5h. The reaction was allowed to cool, diluted with ethyl acetate, and the solution was filtered through a silica column (0.5g). The filtrate was reduced to dryness under a stream of nitrogen and the residue was used without further purification.

The crude intermediate was dissolved in in THF (1ml), hydrazine hydrate (0.1ml) was added and the solution was heated at 60°C for 4h. The reaction mixture was allowed to cool, partitioned between chloroform and water and the organic phase was applied to an SCX SPE cartridge (1g). The cartridge was eluted with methanol followed by a solution of ammonia (0.88) in methanol (10%). The solvent was evaporated and the crude residue was purified by reverse phase preparative HPLC to give the title compound as a cream solid.
LC-MS: Rt 2.62min, MH+ 407.

### Example 27: 3-[3-(4-Chloro-3-fluorophenyl)-1H-pyrazolo[4,3-c]pyridin-6-yl]-N-ethyl-4-methylbenzamide

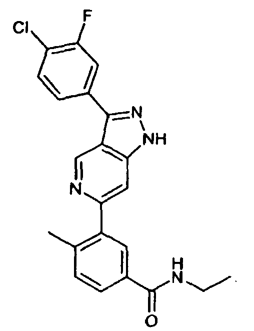

The compound was prepared in an analogous manner to Example 26, replacing 3,4-difluorobenzylzinc iodide with 4-chloro-3-fluorophenylzinc iodide. The crude product from the SCX cartridge was purified by column chromatography on silica, eluting with a cyclohexane/ethyl acetate gradient followed by methanol. The resultant product was triturated with ether to give the title compound as a cream solid.
LC-MS: Rt 3.20min, MH+ 409.

### Example 28: 3-{3-[(3-Chlorophenyl)methyl]-1H-pyrazolo[4,3-c]pyridin-6-yl}-N-ethyl-4-methylbenzamide

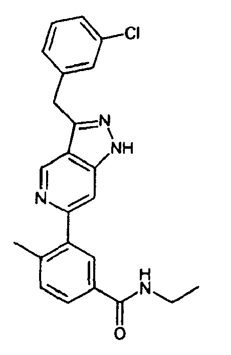

The compound was prepared in an analogous manner to Example 26, replacing 3,4-difluorobenzylzinc iodide with 3-chlorobenzylzinc iodide. Purification by reverse phase preparative HPLC gave the title compound as a cream solid.
LC-MS: Rt 2.71 min, MH+ 405, 407.

### Example 29: N-Ethyl-3-{3-[(4-fluorophenyl)methyl]-1H-pyrazolo[4,3-c]pyridin-6-yl}-4-methylbenzamide

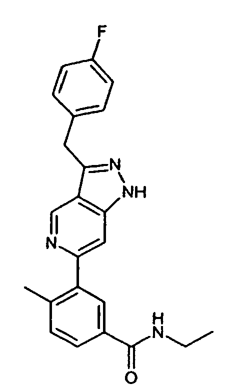

The compound was prepared in an analogous manner to Example 26, replacing 3,4-difluorobenzylzinc iodide with 4-fluorobenzylzinc iodide. Purification by reverse phase preparative HPLC gave the title compound as a cream solid.
LC-MS: Rt 2.51 min, MH+ 389.

### Example 30: N-Ethyl-4-methyl-3-{3-[3-(methyloxy)phenyl]-1H-pyrazolo[4,3-c]pyridin-6-yl}benzamide

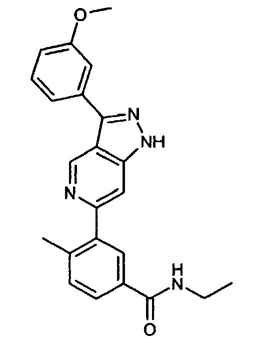

The compound was prepared in an analogous manner to Example 26, replacing 3,4-difluorobenzylzinc iodide with 3-methoxyphenylzinc iodide. The crude product from the SCX cartridge was triturated with ether to give the title compound as a cream solid.
LC-MS: Rt 2.66min, MH+ 387.

### Example 31: 3-[3-(4-Chlorophenyl)-1H-pyrazolo[4,3-c]pyridin-6-yl]-N-ethyl-4-methylbenzamide

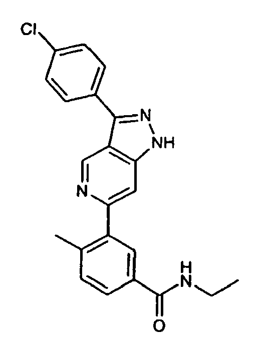

The compound was prepared in an analogous manner to Example 26, replacing 3,4-difluorobenzylzinc iodide with 4-chlorophenylzinc iodide. Purification by reverse phase preparative HPLC gave the title compound as an off-white solid.
LC-MS: Rt 3.04min, MH+ 391, 393.

### Example 32: N-Ethyl-3-[3-(3-fluoro-4-methylphenyl)-1H-pyrazolo[4,3-c]pyridin-6-yl]-4-methylbenzamide

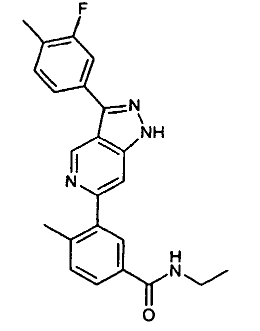

The compound was prepared in an analogous manner to Example 26, replacing 3,4-difluorobenzylzinc iodide with 3-fluoro-4-methylphenylzinc iodide. Purification by reverse phase preparative HPLC gave the title compound as an off-white solid.
LC-MS: Rt 3.04min, MH+ 389.

### Example 33: N-Ethyl-3-[3-(3-fluorophenyl)-1H-pyrazolo[4,3-c]pyridin-6-yl]-4-methylbenzamide

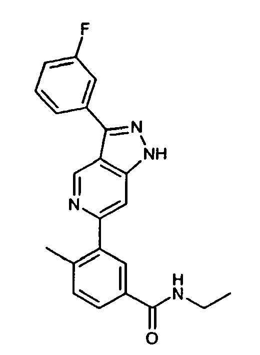

The compound was prepared in an analogous manner to Example 26, replacing 3,4-difluorobenzylzinc iodide with 3-fluorophenylzinc iodide. Purification by reverse phase preparative HPLC gave the title compound as an off-white solid.
LC-MS: Rt 2.75min, MH+ 375.

### Example 34: 3-[3-(4-Cyanophenyl)-1H-pyrazolo[4,3-c]pyridin-6-yl]-N-ethyl-4-methylbenzamide

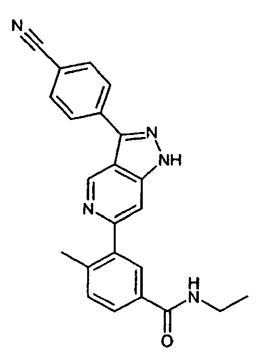

4-[(4,6-Dichloro-3-pyridinyl)carbonyl]benzonitrile (Intermediate 19, 25mg), *N-*ethyl-4-methyl-3-(4,4,5,5-tetramethyl-1,3,2-dioxaborolan-2-yl)benzamide (Intermediate 28, 20.6mg), tetrakis(triphenylphosphine)palladium (~1mg), aqueous sodium hydrogen carbonate (1M, 0.21 ml) and isopropanol (1ml) were mixed and heated at 90°C under nitrogen for 1.5h. The reaction was allowed to cool, diluted with ethyl acetate and the solution was filtered through a silica column (0.5g). The filtrate was concentrated under a stream of nitrogen and the residue was dissolved in THF (1ml). Hydrazine hydrate (0.1ml) was added and the resulting solution was heated at 60°C for 4h. The reaction was allowed to cool, partitioned between chloroform and water and the organic phase was applied to an SCX SPE cartridge (1g) and washed with methanol. Further elution with aqueous ammonia (0.88) in methanol (10%) gave the crude product which was purified by reverse phase HPLC to give the title compound as an off-white solid.
LC-MS: Rt 2.78min, MH+ 382.

### Example 35: N-Ethyl-3-[3-(4-fluorophenyl)-1H-pyrazolo[3,4-b]pyridin-6-yl]-4-methylbenzamide

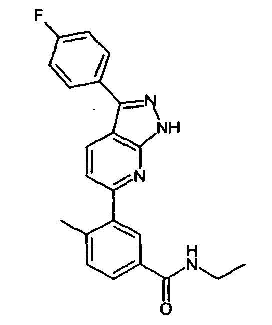

A mixture of 3-{6-chloro-5-[(4-fluorophenyl)carbonyl]-2-pyridinyl}-*N-*ethyl-4-methylbenzamide (Intermediate 21, 55mg) and hydrazine hydrate (0.2ml) was heated in THF (4ml) at 60°C for 6h. Further hydrazine hydrate (0.1ml) was added and heating was continued for 18h. The reaction was partitioned between chloroform and water and the organic phase was dried through a hydrophobic filter tube then concentrated under vacuum. The residue was absorbed onto silica, applied to a silica column (5g) and eluted with an ethyl acetate/cyclohexane gradient (0-100% ethyl acetate). The resultant product was triturated with ether to the title compound as an off-white solid (15mg).
LC-MS: Rt 3.29min, MH+ 375.

### Example 36: N-Cyclopropyl-3-fluoro-5-[3-(4-fluorophenyl)-1H-pyrazolo[3,4-b]pyridin-6-yl]-4-methylbenzamide

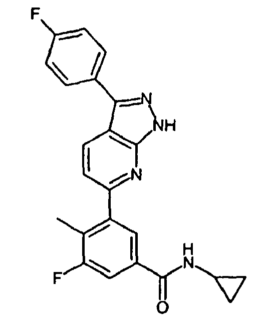

3-{6-Chloro-5-[(4-fluorophenyl)carbonyl]-2-pyridinyl}-*N-*cyclopropyl-5-fluoro-4-methylbenzamide (Intermediate 22, 400mg) and hydrazine hydrate (0.5ml) in THF (5ml) were heated at 60°C for 4.5h. The resulting solution was allowed to cool, diluted with water and extracted with chloroform (x3). The combined organic extracts were dried (MgSO₄) and concentrated under vacuum. The yellow residue was dissolved in ether, applied to a silica column and eluted with ether. The resultant product was triturated with ether to give the title compound as a cream solid (117mg).
LC-MS: Rt 3.39min, MH+ 405.

### Example 37: 3-Fluoro-5-[3-(4-fluorophenyl)-1H-pyrazolo[3,4-b]pyridin-6-yl]-4-methyl-N-(1-methyl-1H-pyrazol-5-yl)benzamide

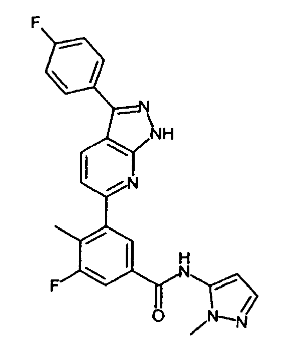

3-{6-Chloro-5-[(4-fluorophenyl)carbonyl]-2-pyridinyl}-5-fluoro-4-methyl-*N-*(1-methyl-1*H-*pyrazol-5-yl)benzamide (Intermediate 25, 56mg) and hydrazine hydrate (0.2ml) in THF (1ml) were heated at 60°C for 4h. The resulting solution was allowed to cool, diluted with water and extracted with chloroform. The organic extracts were dried through a hydrophobic filter tube and concentrated under vacuum. The residue was triturated with methanol/ether to give the title compound as a pale yellow solid (30mg).
LC-MS: Rt 3.34min, MH+ 445.

### Example 38: 3-Fluoro-5-[3-(4-fluorophenyl)-1-methyl-1H-pyrazolo[3,4-b]pyridin-6-yl]-4-methyl-N-(1-methyl-1H-pyrazol-5-yl)benzamide

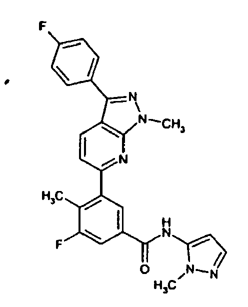

3-{6-Chloro-5-[(4-fluorophenyl)carbonyl]-2-pyridinyl}-5-fluoro-4-methyl-*N-*(1-methyl-1*H-*pyrazol-5-yl)benzamide (Intermediate 25, 32mg) and methyl hydrazine (0.2ml) in THF (1ml) were heated at 60°C for 6h. The resulting solution was allowed to cool diluted with water and extracted with ethyl acetate/chloroform. The organic extracts were dried through a hydrophobic filter tube and concentrated under vacuum. The residue was dissolved in ethyl acetate, applied to a silica column and eluted with ethyl acetate. The resultant product was triturated with ether to give the title compound as an off-white solid.
LC-MS: Rt 3.62min, MH+ 459.

### Example 39: 3-Fluoro-5-[3-(4-fluorophenyl)-1H-pyrazolo[4,3-c]pyridin-6-yl]-4-methyl-N-(1-methyl-1H-pyrazol-5-yl)benzamide

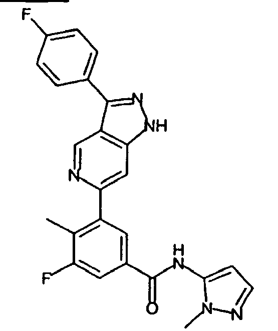

3-{4-Chloro-5-[(4-fluorophenyl)carbonyl]-2-pyridinyl}-5-fluoro-4-methyl-*N-*(1-methyl-1*H-*pyrazol-5-yl)benzamide (Intermediate 26, 50mg) and hydrazine hydrate (0.2ml) in THF (1ml) were heated at 60°C for 18h. The resulting solution was allowed to cool diluted with water and extracted with chloroform. The organic extracts were dried through a hydrophobic filter tube and concentrated under vacuum. The residue was triturated with ether to give the title compound as a pale pink solid (35mg).
LC-MS:,Rt 3.16min, MH+ 445.

### Example 40: 3-Fluoro-5-[3-(4-fluorophenyl)-1-methyl-1H-pyrazolo[4,3-c]pyridin-6-yl]-4-methyl-N-(1-methyl-1H-pyrazol-5-yl)benzamide

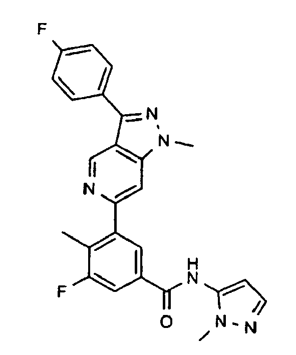

3-{4-Chloro-5-[(4-fluorophenyl)carbonyl]-2-pyridinyl}-5-fluoro-4-methyl-*N-*(1-methyl-1*H-*pyrazol-5-yl)benzamide (Intermediate 26, 48mg) and methyl hydrazine (0.2ml) in THF (1ml) were heated at 60°C for 6h. The resulting solution was allowed to cool diluted with water and extracted with ethyl acetate/chloroform. The organic extracts were dried through a hydrophobic filter tube and concentrated under vacuum. The residue was dissolved in ethyl acetate, applied to a silica column and eluted with ethyl acetate. The resultant product was triturated with ether to give the title compound as a white solid.
LC-MS: Rt 3.29min, MH+ 459.

### Example 41: 3-(3-Amino-1H-pyrazolo[3,4-b]pyridin-6-yl)-N-cyclopropyl-4-methylbenzamide

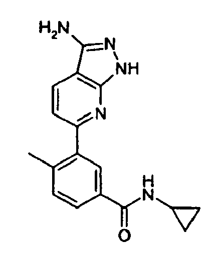

A solution of 3-(6-chloro-5-cyano-2-pyridinyl)-*N-*cyclopropyl-4-methylbenzamide (Intermediate 27, 610mg) in ethanol (7ml) was treated with hydrazine hydrate (0.22ml) then heated at 70°C for 20h. The cooled reaction mixture was partitioned between chloroform (150ml) and water (100ml). The aqueous layer was re-extracted with chloroform (2 X 50ml) and the combined organic extracts were washed with brine (100ml), dried (Na₂SO₄) and concentrated under vacuum. The crude product was triturated in diethyl ether (50ml) to give the title compound as a pale yellow solid (420mg).
LC-MS: Rt 2.40min MH⁺ 308.

### Example 42: 3-[3-(Acetylamino)-1H-pyrazolo[3,4-b]pyridin-6-yl]-N-cyclopropyl-4-methylbenzamide

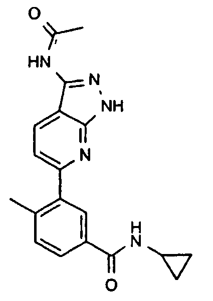

A solution of 3-(3-amino-1*H-*pyrazolo[3,4*-b*]pyridin-6-yl)-*N-*cyclopropyl-4-methylbenzamide (Example 41, 23mg) in pyridine (1ml) was treated with acetyl chloride (7mg) then stirred at room temperature for 1.5h. The pyridine was removed under a stream of nitrogen and the residue was purified by reverse phase preparative HPLC to give the title compound as an off-white solid (10.5mg).

LC-MS: Rt 2.48min, MH+ 350.

### Example 43: N-Cyclopropyl-3-(3-{[(4-fluorophenyl)carbonyl]amino}-1H-pyrazolo[3,4-b]pyridin-6-yl)-4-methylbenzamide

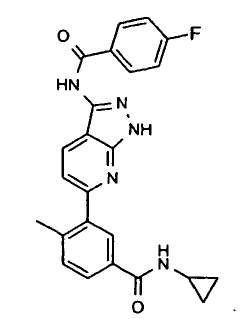

The procedure for Example 42 was followed using 3-(3-amino-1*H-*pyrazolo[3,4-*b*]pyridin-6-yl)-*N-*cyclopropyl-4-methylbenzamide (Example 41, 23mg) 4-fluorobenzoyl chloride (15.5mg) and pyridine (1ml) to give the title compound as an off-white solid (14mg).
LC-MS: Rt 3.09min, MH+ 430.

### Example 44: N-Cyclopropyl-4-methyl-3-{3-[(2-methylpropanoyl)amino]-1H-pyrazolo[3,4-b]pyridin-6-yl}benzamid

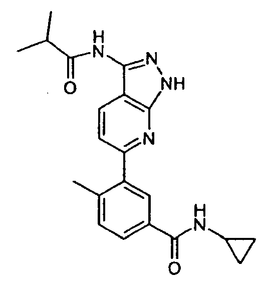

The procedure for Example 42 was followed using 3-(3-amino-1*H-*pyrazolo[3,4-*b*]pyridin-6-yl)-*N-*cyclopropyl-4-methylbenzamide (Example 41, 22mg) isobutyrl chloride (66mg) and pyridine (1ml) to give the title compund as a white solid (8.6 mg).
Example 44: LC-MS: Rt 2.67min, MH+ 378.

### Example 45: 3-{3-[(Cyclopentylcarbonyl)amino]-1H-pyrazolo[3,4-b]pyridin-6-yl}-N-cyclopropyl-4-methylbenzamide

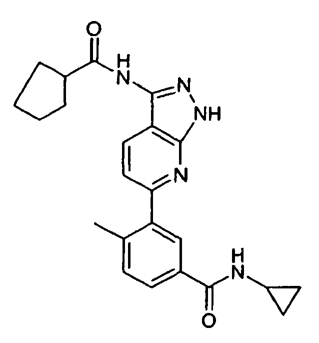

The procedure for Example 42 was followed using 3-(3-amino-1*H-*pyrazolo[3,4-*b*]pyridin-6-yl)-*N-*cyclopropyl-4-methylbenzamide (Example 41, 20mg) cyclopentanecarbonyl chloride (9.4µl) and pyridine (1ml) to give the title compound (1.8 mg).
LC-MS: Rt 2.90min, MH+ 404.

### Example 46: N-Cyclopropyl-4-methyl-3-[3-(propanoylamino)-1H-pyrazolo[3,4-b]pyridin-6-yl]benzamide

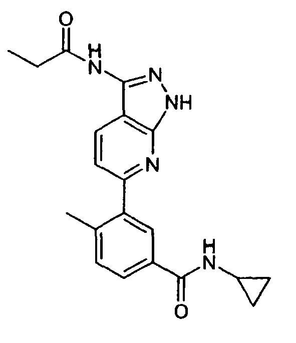

The procedure for Example 42 was followed using 3-(3-amino-1*H-*pyrazolo[3,4-*b*]pyridin-6-yl)-*N-*cyclopropyl-4-methylbenzamide (Example 41, 22mg) propionyl chloride (7.5µl) and pyridine (1ml) to give the title compound (2.0 mg).
LC-MS: Rt 2.56min, MH+ 364.

### Example 47: N-Cyclopropyl-4-methyl-3-(3-{[(4-methylphenyl)carbonyl]amino}-1H-pyrazolo[3,4,b]pyridin-6-yl)benzamide

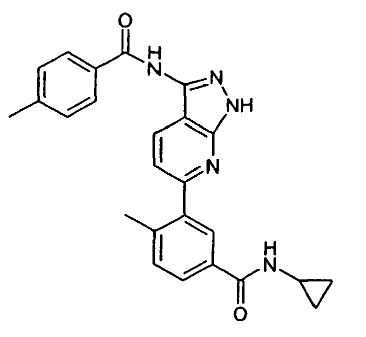

The procedure for Example 42 was followed using 3-(3-amino-1*H-*pyrazolo[3,4-*b*]pyridin-6-yl)-*N-*cyclopropyl-4-methylbenzamide (Example 41, 27mg) p-toluoyl chloride (28µl) and pyridine (1ml) to give the title compound (13.9 mg).
LC-MS: Rt 3.06min, MH+ 426.

### Example 48: N-Cyclopropyl-4-methyl-3-[3-({[4-(methyloxy)phenyl]carbonyl}amino)-1H-pyrazolo[3,4-b]pyridin-6-yl]benzamide

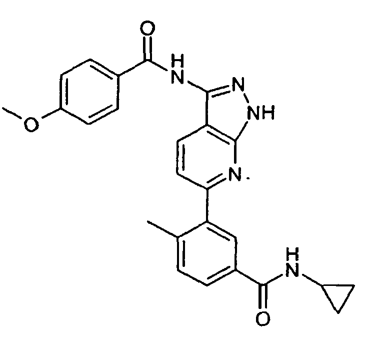

The procedure for Example 42 was followed using 3-(3-amino-1*H-*pyrazolo[3,4-*b*]pyridin-6-yl)-*N-*cyclopropyl-4-methylbenzamide (Example 41, 25mg) p-anisoyl chloride (34µl) and pyridine (1ml) to give the title compound (16.1 mg).
LC-MS: Rt 2.94, MH+ 442.

### Example 49: N-(6-{5-[(Cyclopropylamino)carbonyl]-2-methylphenyl}-1H-pyrazolo[3,4-b]pyridin-3-yl)-2-thiophenecarboxamide

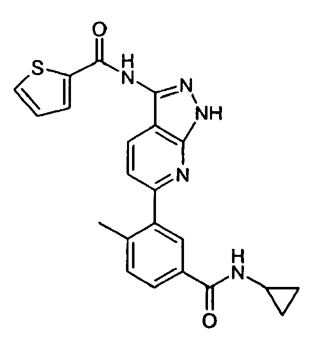

The procedure for Example 42 was followed using 3-(3-amino-1*H-*pyrazolo[3,4-*b*]pyridin-6-yl)-*N-*cyclopropyl-4-methylbenzamide (Example 41, 20mg) thiophene-2-carbonyl chloride (32µl) and pyridine (1ml) to give the title compound (7.1 mg).
LC-MS: Rt 2.88min, MH+ 418.

### Example 50: N-Cyclopropyl-4-methyl-3-{3-[(methylsulfonyl)amino]-1H-pyrazolo[3,4-b]pyridin-6-yl}benzamide

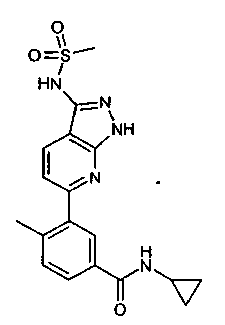

A solution of 3-(3-amino-1*H-*pyrazolo[3,4*-b*]pyridin-6-yl)-*N-*cyclopropyl-4-methylbenzamide (Example 41, 23mg) in pyridine (1ml) was treated with methanesulphonyl chloride (9.5mg) then stirred at room temperature for 1.5h. The pyridine was removed under a stream of nitrogen and the residue was purified by reverse phase preparative HPLC to give the title compound as an off-white solid (6.1 mg).
LC-MS: Rt 2.56min, MH+ 386.

### Example 51: N-Cyclopropyl-3-(3-{[(4-fluorophenyl)sulfonyl]amino}-1H-pyrazolo[3,4-b]pyridin-6-yl)-4-methylbenzamide

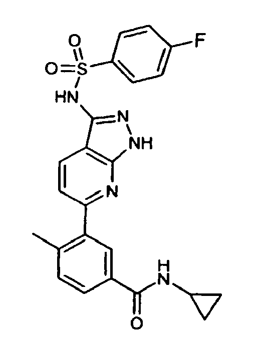

The procedure for Example 50 was followed using 3-(3-amino-1*H-*pyrazolo[3,4-*b*]pyridin-6-yl)-*N-*cyclopropyl-4-methylbenzamide (Example 41, 20mg) 4-fluorobenzenesulphonyl chloride (16mg) and pyridine (1ml) to give the title compound as an off-white solid (12.5 mg).
LC-MS: Rt 3.01 min, MH+ 466.

### Example 52: N-Cyclopropyl-3-{3-[(ethylsulfonyl)amino]-1H-pyrazolo[3,4-b]pyridin-6-yl}-4-methylbenzamide

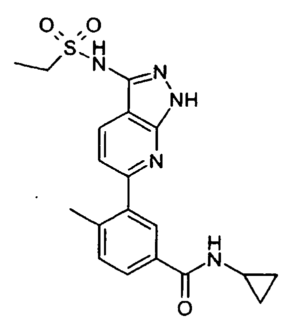

The procedure for Example 50 was followed using 3-(3-amino-1*H-*pyrazolo[3,4-*b*]pyridin-6-yl)-*N-*cyclopropyl-4-methylbenzamide (Example 41, 25mg), ethanesulphonyl chloride (6.8µl) and pyridine (1ml) to give the title compound (9.5mg).
LC-MS: Rt 2.56min, MH+ 400.

### Example 53: N-Cyclopropyl-4-methyl-3-{3-[(propylsulfonyl)amino]-1H-pyrazolo[3,4-b]pyridin-6-yl}benzamide

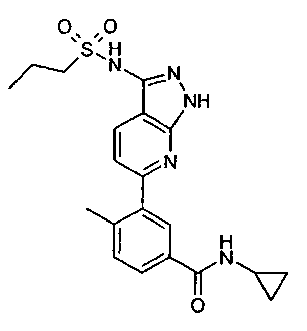

The procedure for Example 50 was followed using 3-(3-amino-1*H-*pyrazolo[3,4-*b*]pyridin-6-yl)-*N-*cyclopropyl-4-methylbenzamide (Example 41, 21 mg), 1-propanesulphonyl chloride (16µl) and pyridine (1ml) to give the title compound (8.5mg).
LC-MS: Rt 2.68min, MH+ 414.

### Example 54: N-Cyclopropyl-4-methyl-3-{3-[(3-thienylsulfonyl)amino]-1H-pyrazolo[3,4-b]pyridin-6-yl}benzamide

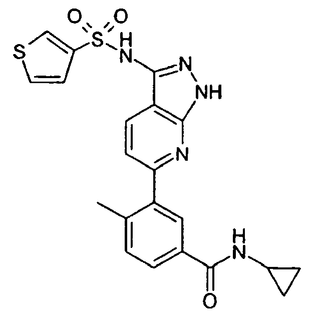

The procedure for Example 50 was followed using 3-(3-amino-1*H-*pyrazolo[3,4-*b*]pyridin-6-yl)-*N-*cyclopropyl-4-methylbenzamide (Example 41, 24mg), 3-thiophenesulfonyl chloride (39mg) and pyridine (1ml) to give the title compound (16mg).
LC-MS: Rt 2.81 min, MH+ 454.

### Example 55: N-Cyclopronyl-3-(3-{[(3,5-dimethyl-4-isoxazolyl)sulfonyl]amino}-1H-pyrazolo[3,4,b]pyridin-6-yl)-4-methylbenzamide

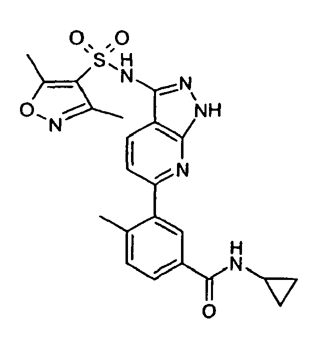

The procedure for Example 50 was followed using 3-(3-amino-1*H-*pyrazolo[3,4-*b*]pyridin-6-yl)-*N-*cyclopropyl-4-methylbenzamide (Example 41, 21 mg), 3,5-dimethyl-4-isoxazolesulfonyl chloride (15mg) and pyridine (1ml) to give the title compound (12.2mg).
LC-MS: Rt 2.85min, MH+ 467.

### Example 56: N-Cyclopropyl-4-methyl-3-{3-[(2-thienylsulfonyl)amino]-1H-pyrazolo[3,4-b]pyridin-6-yl}benzamide

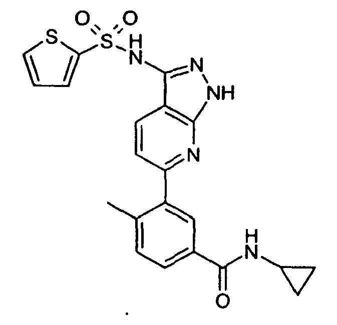

The procedure for Example 50 was followed using 3-(3-amino-1*H-*pyrazolo[3,4-*b*]pyridin-6-yl)-*N-*cyclopropyl-4-methylbenzamide (Example 41, 21 mg), 2-thiophenesulphonyl chloride (20mg) and pyridine (1ml) to give the title compound (13mg).
LC-MS: Rt 2.85min, MH+ 454.

### Example 57: N-Cyclopropyl-4-methyl-3-{3-[(1-methylethyl)amino]-1H-pyrazolo[3,4-b]pyridin-6-yl}benzamide

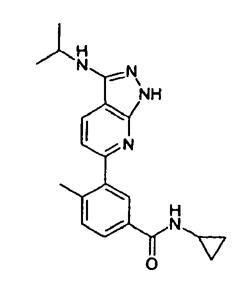

A mixture of 3-(3-amino-1*H-*pyrazolo[3,4*-b*]pyridin-6-yl)-*N-*cyclopropyl-4-methylbenzamide (Example 41, 30mg) acetone (0.22ml) sodium triacetoxyborohydride (126mg) and acetic acid (22µl) in DMF (1ml) was stirred at room temperature for 84h. The mixture was concentrated under vacuum and the residue was purified by reverse phase preparative HPLC to give the title compound as a yellow solid (5.2mg).
LC-MS: Rt 2.70min, MH+ 350.

### Example 58: 3-(3-Bromo-1H-pyrazolo[3,4-b]pyridin-6-yl)-N-cyclopropyl-4-methylbenzamide

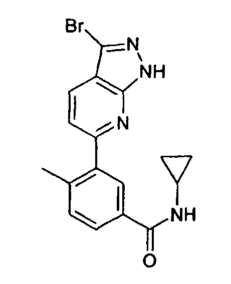

To a suspension of 3-(3-amino-1*H-*pyrazolo[3,4*-b*]pyridin-6-yl)-*N-*cyclopropyl-4-methylbenzamide (Example 41, 65mg) in water (2ml) was added concentrated sulphuric acid (0.14ml). The resulting orange suspension was cooled to 0°C and a solution of sodium nitrite (35mg) in water (0.3ml) was added dropwise. The mixture was stirred at 0°C for 40 min. then hydrobromic acid (48%, 0.4ml) was added dropwise. The mixture was stirred for 30min at 0°C, warmed to room temperature, then heated with a heat gun until effervescence ceased. The solid was collected by filtration and purified by reverse phase preparative HPLC to give the title compound as a pale brown solid (12mg).
LC-MS: Rt 3.08min MH⁺ 371,373.

### Abbreviations

- Ac₂O: Acetic anhydride
- BINAP: 2,2'-Bis(diphenylphosphino)1-,1'-binaphthyl
- DCM: Dichloromethane
- DIPEA: N,N-Diisopropylethylamine
- DMF: Dimethylformamide
- DMSO: Dimethylsulfoxide
- EtOH: Ethanol
- h: Hours
- Hal: Halogen
- HATU: O-(7-Azabenzotriazol-1-yl)-N,N,N',N'-tetramethyluronium hexafluorophosphate
- LDA: Lithium diisopropylamide
- m-CPBA: 3-Chloroperbenzoic acid
- min: Minutes
- Ms: Mesyl
- NaOBu^{t}: Sodium t-butoxide
- PdCl₂dppf: [1,1'-bis(diphenylphosphino)ferrocene]dichloropalladium (II) complex with dichloromethane (1:1)
- Pd(OAc)₂: Palladium (II) acetate
- Rt: Retention Time
- SPE: Solid phase extraction
- THF: Tetrahydrofuran

### BIOLOGICAL EXAMPLES

The activity of compounds of formula (I) as p38 inhibitors may be determined by the following *in vitro* assays:

### Fluorescence anisotropy kinase binding assay 1

The kinase enzyme, fluorescent ligand and a variable concentration of test compound are incubated together to reach thermodynamic equilibrium under conditions such that in the absence of test compound the fluorescent ligand is significantly (>50%) enzyme bound and in the presence of a sufficient concentration (>10x Kᵢ) of a potent inhibitor the anisotropy of the unbound fluorescent ligand is measurably different from the bound value.

The concentration of kinase enzyme should preferably be ≥ 1 x K_{f}. The concentration of fluorescent ligand required will depend on the instrumentation used, and the fluorescent and physicochemical properties. The concentration used must be lower than the concentration of kinase enzyme, and preferably less than half the kinase enzyme concentration. A typical protocol is:

All components dissolved in Buffer of final composition 62.5 mM HEPES, pH 7.5, 1.25 mM CHAPS, 1.25 mM DTT, 12.5 mM MgCl₂ 3.3% DMSO.
p38 Enzyme concentration: 12 nM
Fluorescent ligand concentration: 5 nM
Test compound concentration: 0.1 nM - 100 µM
Components incubated in 30 µl final volume in NUNC 384 well black microtitre plate until equilibrium reached (5-30 mins)
Fluorescence anisotropy read in LJL Acquest.
Definitions:
Kᵢ = dissociation constant for inhibitor binding
K_{f} = dissociation constant for fluorescent ligand binding

The fluorescent ligand is the following compound: 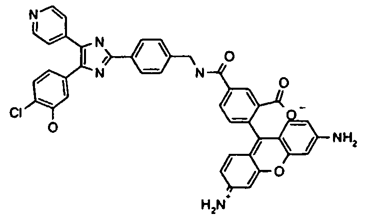 which is derived from 5-[2-(4-aminomethylphenyl)-5-pyridin-4-yl-1H-imidazol-4-yl]-2-chlorophenol and rhodamine green.

### Fluorescence anisotropy kinase binding assay 2 (macro volume assay)

The kinase enzyme, fluorescent ligand and a variable concentration of test compound are incubated together to reach thermodynamic equilibrium under conditions such that in the absence of test compound the fluorescent ligand is significantly (>50%) enzyme bound and in the presence of a sufficient concentration (>10 × *K*i) of a potent inhibitor the anisotropy of the unbound fluorescent ligand is measurably different from the bound value.

The concentration of kinase enzyme should preferably be 2 x *K*f. The concentration of fluorescent ligand required will depend on the instrumentation used, and the fluorescent and physicochemical properties. The concentration used must be lower than the concentration of kinase enzyme, and preferably less than half the kinase enzyme concentration.

The fluorescent ligand is the following compound: 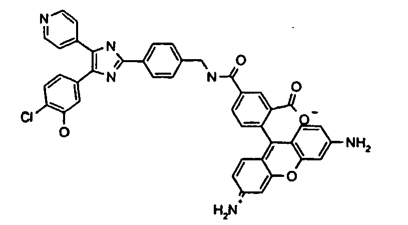 which is derived from 5-[2-(4-aminomethylphenyl)-5-pyridin-4-yl-1H-imidazol-4-yl]-2-chlorophenol and rhodamine green.

Recombinant human p38α was expressed as a GST-tagged protein. To activate this protein, 3.5 µM unactivated p38α was incubated in 50 mM Tris-HCl pH 7.5, 0.1 mM EGTA, 0.1% 2-mercaptoethanol, 0.1mM sodium vanadate, 10mM MgAc, 0.1mM ATP with 200nM MBP-MKK6 DD at 30 degrees for 30 mins. Following activation p38α was re-purified and the activity assessed using a standard filter-binding assay.

Protocol: All components are dissolved in buffer of composition 62.5 mM HEPES, pH 7.5, 1.25 mM CHAPS, 1 mM DTT, 12.5 mM MgCl₂ with final concentrations of 12nM p38α and 5nM fluorescent ligand. 30µl of this reaction mixture is added to wells containing 1 µl of various concentrations of test compound (0.28 nM - 16.6 µM final) or DMSO vehicle (3% final) in NUNC 384 well black microtitre plate and equilibrated for 30-60 mins at room temperature. Fluorescence anisotropy is read in Molecular Devices Acquest (excitation 485nm/emission 535nm).

Definitions:
*K*i = dissociation constant for inhibitor binding
*K*f = dissociation constant for fluorescent ligand binding

### Fluorescence anisotropy kinase binding assay 3 (micro volume assay)

The kinase enzyme, fluorescent ligand and a variable concentration of test compound are incubated together to reach thermodynamic equilibrium under conditions such that in the absence of test compound the fluorescent ligand is significantly (>50%) enzyme bound and in the presence of a sufficient concentration (>10 x *K*i) of a potent inhibitor the anisotropy of the unbound fluorescent ligand is measurably different from the bound value.

The concentration of kinase enzyme should preferably be 2 x *K*f. The concentration of fluorescent ligand required will depend on the instrumentation used, and the fluorescent and physicochemical properties. The concentration used must be lower than the concentration of kinase enzyme, and preferably less than half the kinase enzyme concentration.

The fluorescent ligand is the following compound: 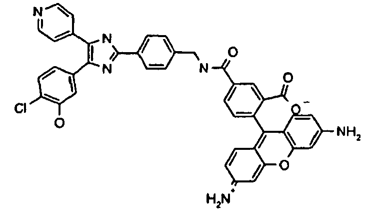 which is derived from 5-[2-(4-aminomethylphenyl)-5-pyridin-4-yl-1H-imidazol-4-yl]-2-chlorophenol and rhodamine green.

Recombinant human p38α was expressed as a GST-tagged protein. To activate this protein, 3.5 µM unactivated p38α was incubated in 50 mM Tris-HCl pH 7.5, 0.1 mM EGTA, 0.1% 2-mercaptoethanol, 0.1mM sodium vanadate, 10mM MgAc. 0.1mM ATP with 200nM MBP-MKK6 DD at 30 degrees for 30 mins. Following activation p38α was re-purified and the activity assessed using a standard filter-binding assay.

Protocol: All components are dissolved in buffer of composition 62.5 mM HEPES, pH 7.5, 1.25 mM CHAPS, 1 mM DTT, 12.5 mM MgCl₂ with final concentrations of 12nM p38α and 5nM fluorescent ligand. 6µl of this reaction mixture is added to wells containing 0.2 µl of various concentrations of test compound (0.28 nM - 16.6 µM final) or DMSO vehicle (3% final) in Greiner 384 well black low volume microtitre plate and equilibrated for 30-60 mins at room temperature. Fluorescence anisotropy is read in Molecular Devices Acquest (excitation 485nm/emission 535nm).

Definitions:
*K*i = dissociation constant for inhibitor binding
*K*f = dissociation constant for fluorescent ligand binding

### Results

The compounds described in the Examples were tested in at least one of the assays described above and had either IC₅₀ values of <10 µM or pKᵢ values of >6.

## Claims

1. A compound of formula (I): 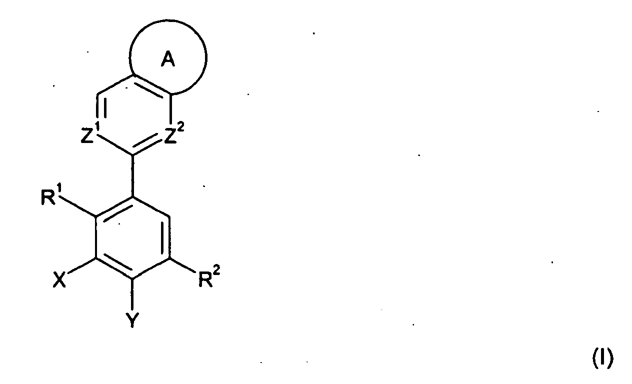 wherein
A is a fused 5-membered heteroaryl ring containing one or two heteroatoms independently selected from oxygen and nitrogen which heteroaryl ring is optionally substituted by up to two substituents independently selected from C₁₋₆alkyl, -(CH₂)ₖ-C₃₋₇cycloalkyl, halogen, cyano, trifluoromethyl, -(CH₂)ₖOR³, -(CH₂)ₖCO₂R³, -(CH₂)ₖNR³R⁴, - (CH₂)ₖCONR³R⁴, -(CH₂)ₖNHCOR³, -(CH₂)ₖSO₂NR³R⁴, -(CH₂)ₖNHSO₂R³,-(CH₂)ₖSO₂(CH₂)ₘR^{S}, a 5- or 6-membered heterocyclyl ring containing nitrogen optionally substituted by C₁₋₂alkyl or -(CH₂)ₖCO₂R³, and a 5-membered heteroaryl ring optionally substituted by C₁₋₂alkyl;
A is a fused 5-membered heteroaryl ring containing one or two heteroatoms independently selected from oxygen and nitrogen which heteroaryl ring is substituted by - BR⁶, and
the heteroaryl ring is optionally further substituted by one substituent selected from -OR⁷, halogen, trifluoromethyl, -CN, -CO₂R⁷ and C₁₋₆alkyl optionally substituted by hydroxy;
A is a fused 5-membered heteroaryl ring containing one or two heteroatoms independently selected from oxygen and nitrogen which heteroaryl ring is substituted by - (CH₂)ₙheterocyclyl wherein the heterocyclyl is a 5- or 6-membered heterocyclic ring containing one or two heteroatoms independently selected from oxygen, sulfur and nitrogen optionally substituted by up.to two substituents independently selected from oxo, C₁₋₆alkyl, - (CH₂)ₚphenyl, -OR⁷, -(CH₂)ₚCO₂R⁷, -NR⁷R⁸ and -CONR⁷R⁸, and
the heteroaryl ring is optionally further substituted by one substituent selected from -OR⁷, halogen, trifluoromethyl, -CN, -CO₂R⁷ and C₁₋₆alkyl optionally substituted by hydroxy; or
A is a fused 5-membered heteroaryl ring containing one or two heteroatoms independently selected from oxygen and nitrogen which heteroaryl ring is substituted by - (CH₂)_{q}aryl or -(CH₂)_{q}heteroaryl wherein the aryl or heteroaryl is optionally substituted by one or more substituents independently selected from oxo, C₁₋₆alkyl, - halogen, cyano, trifluoromethyl, -OR⁹, -(CH₂)ᵣCO₂R¹⁰, -NR⁹R¹⁰, -(CH₂)ᵣCONR⁹R¹⁰, -NHCOR9, - SO₂NR⁹R¹⁰, -NHSO₂R⁹ and -S(O)ₛR⁹, and
the heteroaryl, ring is optionally further substituted by one substituent selected from -OR⁷, halogen, trifluoromethyl, -CN, -CO₂R⁷ and C₁₋₆alkyl optionally substituted by hydroxy;
R¹ is selected from methyl and chloro;
R² is selected from -NH-CO-R¹¹ and -CO-NH-(CH₂)ₜR¹²;
R³ is selected from hydrogen, C₁₋₆alkyl optionally substituted by up to two OH groups, -(CH₂)ₖ-C₃₋₇cycloalkyl, -(CH₂)ₖphenyl optionally substituted by R¹³ and/or R¹⁴ and -(CH₂)ₖheteroaryl optionally substituted by R¹³ and/or R¹⁴,
R⁴ is selected from hydrogen and C₁₋₆alkyl, or
R³ and R⁴, together with the nitrogen atom to which they are bound, form a 5- or 6-membered heterocyclic ring optionally containing one additional heteroatom selected from oxygen, sulfur and N-R¹⁵;
R⁵ is selected from C₁₋₆alkyl optionally substituted by up to three halogen atoms, C₂₋₆alkenyl optionally substituted by phenyl, C₃₋₇cycloalkyl, heteroaryl optionally substituted by up to three R¹³ and/or R¹⁴ groups, and phenyl optionally substituted by R¹³ and/or R¹⁴;
R⁶ is a C₃₋₆alkyl group substituted by at least two substituents independently selected from -OR¹⁶, -NR¹⁶R¹⁷, -CO₂R¹⁶, -CONR¹⁶R¹⁷ -NHCOR¹⁶ and -NHSO₂R¹⁶;
R⁷ and R⁸ are each independently selected from hydrogen and C₁₋₆alkyl;
R⁹ is selected from hydrogen, -(CH₂)ᵤ-C₃₋₇cycloalkyl, -(CH₂)ᵤheterocyclyl, - (CH₂)ᵤaryl, and C₁₋₆alkyl optionally substituted by up to two substituents independently selected from -OR¹⁸ and -NR¹⁸R¹⁹,
R¹⁰ is selected from hydrogen and C₁₋₆alkyl, or
R⁹ and R¹⁰, together with the nitrogen atom to which they are bound, form a 5- or 6-membered heterocyclic ring optionally containing one additional heteroatom selected from oxygen, sulfur and N-R¹⁵;
R¹¹ is selected from hydrogen, C₁₋₆alkyl, -(CH₂)ₜ-C₃₋₇cycloalkyl, trifluoromethyl, - (CH₂)ᵥheteroaryl optionally substituted by R²⁰ and/or R²¹, and -(CH₂)ᵥPhenyl optionally substituted by R²⁰ and/or R²¹;
R¹² is selected from hydrogen, C₁₋₆alkyl, C₃₋₇cycloalkyl, -CONHR²², phenyl optionally substituted by R²⁰ and/or R²¹, and heteroaryl optionally substituted by R²⁰ and/or R²¹;
R¹³ and R¹⁴ are each independently selected from halogen, cyano, trifluoromethyl, nitro, C₁₋₆alkyl, C₁₋₆alkoxy, -CONR²²R²³, -COR²⁴, -CO₂R²⁴, and heteroaryl, or
R¹³ and R¹⁴ are linked to form a fused 5-membered heterocyclyl ring containing one heteroatom selected from oxygen, sulfur and N-R¹⁵, or a fused heteroaryl ring;
R¹⁵ is selected from hydrogen and methyl;
R¹⁶, R¹⁷, R¹⁸ and R19 are each independently selected from hydrogen and C₁₋₆alkyl;
R²⁰ is selected from C₁₋₆alkyl, C₁₋₆alkoxy, -(CH₂)ₜ-C₃₋₇cycloalkyl, -CONR²²R²³, - NHCOR²³, halogen, -CN, -(CH₂)_{w}NR²⁵R²⁶, trifluoromethyl, phenyl optionally substituted by one or more R²¹ groups, and heteroaryl optionally substituted by one or more R²¹ groups;
R²¹ is selected from C₁₋₆alkyl, C₁₋₆alkoxy, halogen, trifluoromethyl, and - (CH2)_{w}NR²⁵R²⁶;
R²² and R²³ are each independently selected from hydrogen and C₁₋₆alkyl, or
R²² and R²³, together with the nitrogen atom to which they are bound, form a 5- or 6-membered heterocyclic ring optionally containing one additional heteroatom selected from oxygen, sulfur and N-R¹⁵, wherein the ring may be substituted by up to two C₁₋₆allyl groups;
R²⁴ is C₁₋₆alkyl;
R²⁵ is selected from hydrogen, C₁₋₆alkyl and -(CH₂)ₜ-C₃₋₇cycloalkyl optionally substituted by C₁₋₆alkyl,
R²⁶ is selected from hydrogen and C₁₋₆alkyl, or
R²⁵ and R²⁶, together with the nitrogen atom to which they are bound, form a 5- or 6-membered heterocyclic ring optionally containing one additional heteroatom selected from oxygen, sulfur and N-R¹⁵;
B is selected from a bond, oxygen, NH and S(O)ₓ;
X and Y are each independently selected from hydrogen, methyl and halogen;
Z¹ is N or N=O and Z² is CH,
Z¹ is CH and Z² is N or N=O, or
Z¹ and 2² are each independently selected from N or N=O;
k, m and w are each independently selected from 0, 1, 2 and 3;
n, q, r, s, t and x are each independently selected from 0, 1 and 2; and
u and v are each independently selected from 0 and 1;
or a pharmaceutically salt or solvate thereof.

2. A compound according to claim 1 wherein A is a 5-membered heteroaryl ring containing two heteroatoms independently selected from oxygen and nitrogen.

3. A compound according to claim 1 or claim 2 wherein A is substituted by up to two substituents independently selected from C₁₋₄alkyl, halogen, -(CH₂)ₖNR³R⁴, - (CH₂)ₖNHCOR³, -(CH₂)ₖNHSO₂R³ and -(CH₂)ₖSO₂(CH₂)mR⁵, or A is substituted by - (CH₂)_{q}aryl wherein the aryl is optionally substituted by one or two substituents independently selected from C₁₋₆alkyl, halogen, cyano, -OR⁹ and -(CH₂)ᵣCO₂R¹⁰.

4. A compound according to any one of the preceding claims wherein A is substituted by -(CH₂)kSO₂(CH₂)ₘR⁵ or -(CH₂)_{q}aryl wherein the aryl is substituted by C₁₋₆alkyl or halogen.

5. A compound according to any one of the preceding claims wherein R¹ is methyl.

6. A compound according to any one of the preceding claims wherein R² is -CO-NH-(CH₂)ₜ-R¹².

7. A compound according to any one of the preceding claims wherein X is hydrogen or fluorine.

8. A compound according to claim 1 selected from:
*N*-cyclopropyl-4-methyl-3-{1-[(1-methylethyl)sulfonyl]-1*H*-pyrazolo[3,4-*c*]pyridin-5-yl}benzamide;
*N*-cyclopropyl-4-methyl-5-[1-(2-thienylsulfonyl)-1*H*-pyrazolo[3,4-*c*]pyridin-5-yl]benzamide;
*N-*cyclopropyl-3-fluoro-4-methyl-5-[1-(2-thienylsulfonyl)-1*H-*pyrazolo[3,4*-c*]pyridin-5-yl]benzamide;
*N*-cyclopropyl-3-[1-(cyclopropylsulfonyl)-1*H*-pyrazolo[3,4-*c*]pyridin-5-yl]-5-fluoro-4-methylbenzamide;
*N-*cyclopropyl-3-fluoro-4-methyl-5-[1-(3-methylphenyl)-1*H-*pyrazolo[3,4*-c*]pyridin-5-yl]benzamide;
*N*-cyclopropyl-4-methyl-5-(1-phenyl-1*H*-pyrazolo[3,4-*c*]pyridin-5-yl)benzamide;
*N*-cyclopropyl-3-[1-(2-fluorophenyl)-1*H*-pyrazolo[3,4-*c*]pyridin-5-yl]-4-methylbenzamide;
*N*-cyclopropyl-3-fluoro-5-[3-(4-fluorophenyl)-1*H*-pyrazolo[3,4-*b*]pyridin-6-yl]-4-methylbenzamide;
3-fluoro-5-[3-(4-fluorophenyl)-1*H*-pyrazolo[3,4-*b*]pyridin-6-yl]-4-methyl-*N*-(1-methy-1*H-*pyrazol-5-yl)benzamide;
3-fluoro-5-[3-(4-fluorophenyl)-1*H*-pyrazolo[4,3-*c*]pyridin-6-yl]-4-methyl-*N*-(1 -methyl-1*H-*pyrazol-5-yl)benzamide;
3-[3-(acetylamino)-1*H-*pyrazolo[3,4*-b*]pyridin-6-yl]-*N*-cyclopropyl-4-methylbenzamide;
*N*-cyclopropyl-4-methyl-3-{3-[(2-methylpropanoyl)amino]-1*H*-pyrazolo[3,4-*b*]pyridin-6-yl}benzamide;
*N*-cyclopropyl-4-methyl-3-[3-(propanoylamino)-1*H*-pyrazolo[3,4-*b*]pyridin-6-yl]benzamide;
and
*N*-(6-{5-[(cyclopropylamino)carbonyl]-2-methylphenyl}-1*H*-pyrazolo[3,4-*b*]pyridin-3-yl)-2-thiophenecarboxamide;
or a pharmaceutically acceptable salt or solvate thereof.

9. A pharmaceutical composition comprising at least one compound as claimed in any one of claims 1 to 8, or a pharmaceutically acceptable salt or solvate thereof, in association with one or more pharmaceutically acceptable excipients, diluents and/or carriers.

10. A compound according to any one of claims 1 to 8, or a pharmaceutically acceptable salt or solvate thereof, for use in therapy.

11. A compound as claimed in any one of claims 1 to 8, or a pharmaceutically acceptable salt or solvate thereof, for use in the treatment or prophylaxis of a condition or disease state mediated by p38 kinase activity or mediated by cytokines produced by the activity of p38 kinase.

12. Use of a compound as claimed in any one of claims 1 to 8, or a pharmaceutically acceptable salt or solvate thereof, in the manufacture of a medicament for use in the treatment of a condition or disease state mediated by p38 kinase activity or mediated by cytokines produced by the activity of p38 kinase.

13. A process for preparing a compound of formula (I) as claimed in any one of claims 1 to 8, or a pharmaceutically acceptable salt or solvate thereof, which comprises
(a) reacting a compound of formula (II) 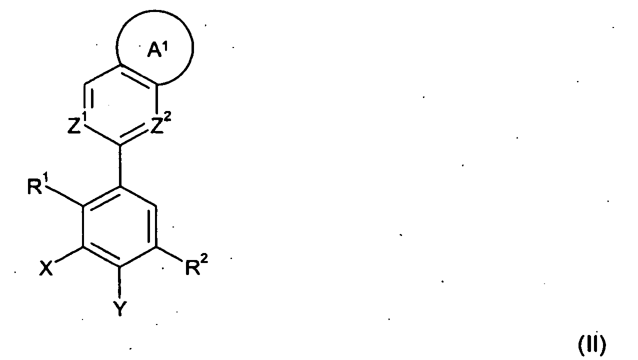 in which R¹, R², X, Y, Z¹ and Z² are as defined in claim 1 and A¹ is an unsubstituted fused 5-membered heteroaryl ring containing one or two heteroatoms independently selected from oxygen and nitrogen with a halide derivative,
in the presence of a base;
(b) when A is a fused pyrazolyl, reacting a compound of formula (XI) 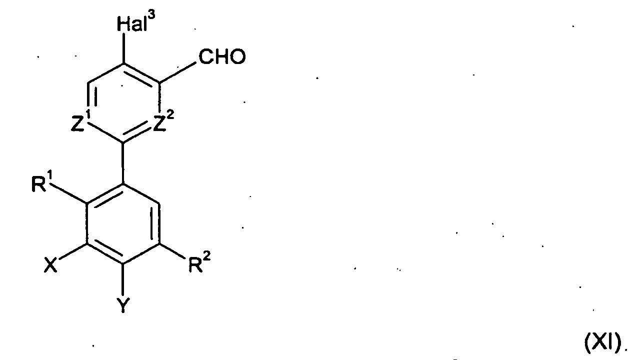 in which R¹, R², X, Y, Z¹ and Z² are as hereinbefore defined and Hal³ is halogen, in particular chlorine, with a hydrazine derivative;
(c) when A is a fused pyrazolyl substituted by aryl, reacting a compound of formula (XII) 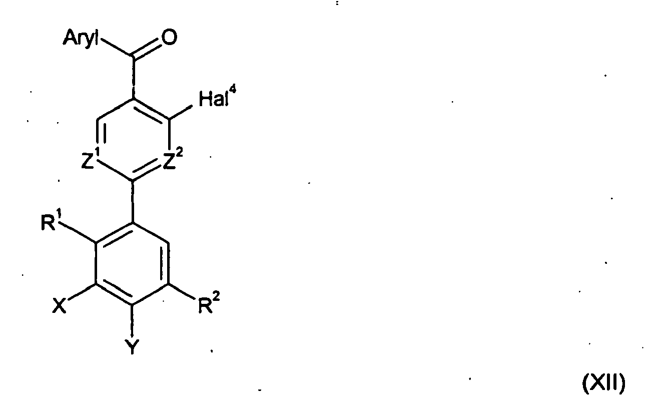 in which R¹, R², X, Y, Z¹ and Z² are as hereinbefore defined and Hal⁴ is halogen, in particular chlorine, with a hydrazine derivative; or
(d) final stage modification of one compound of formula (I) as defined in claim 1 to give another compound of formula (I) as defined in claim 1.

## Patentansprüche

1. Verbindung der Formel (I): 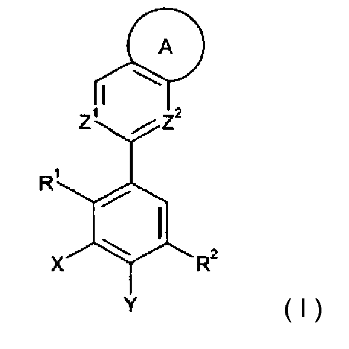 worin :
A ein kondensierter 5-gliedriger Heteroarylring mit einem oder zwei Heteroatomen ist, die unabhängig voneinander aus Sauerstoff oder Stickstoff ausgewählt sind, wobei der Heteroarylring wahlweise mit bis zu zwei Substituenten substituiert ist, die unabhängig voneinander aus C₁₋₆-Alkyl, -(CH₂)ₖ-C₃₋₇-Cycloalkyl, Halogen, Cyano, Trifluormethyl, -(CH₂)ₖOR³, - (C_{H}2) ₖCO₂R³, - (CH₂)ₖNR³R⁴, - (CH₂) ₖCONR³R⁴, -(CH₂)ₖNHCOR³, -(CH₂)ₖSO₂NR³R⁴, -(CH₂)ₖNHSO₂R³, -(CH₂)ₖSO₂(CH₂)ₘR⁵, einem 5- oder 6-gliedrigen, heterocyclischen, stickstoffhaltigen Ring, der wahlweise mit C₁₋₂-Alkyl oder -(CH₂)ₖCO₂R³ substituiert ist, und einem 5-gliedrigen Heteroarylring, der wahlweise mit C₁₋₂-Alkyl substituiert ist, ausgewählt sind;
A ein kondensierter 5-gliedriger Heteroarylring mit einem oder zwei Heteroatomen ist, die unabhängig voneinander aus Sauerstoff und Stickstoff ausgewählt sind, wobei der Heteroarylring mit -BR⁶ substituiert ist, und
der Heteroarylring wahlweise weiter mit einem Substituenten, ausgewählt aus -OR⁷, Halogen, Trifluormethyl, -CN, -CO₂R⁷ und C₁₋₆-Alkyl, das wahlweise mit Hydroxy substituiert ist, substituiert ist;
A ein kondensierter 5-gliedriger Heteroarylring mit einem oder zwei Heteroatomen ist, die unabhängig voneinander aus Sauerstoff und Stickstoff ausgewählt sind, wobei der Heteroarylring mit (CH₂)ₙ⁻ Heterocyclyl substituiert ist, wobei das Heterocyclyl ein 5- oder 6-gliedriger heterocyclischer Ring mit einem oder zwei Heteroatomen ist, die unabhängig voneinander aus Sauerstoff, Schwefel und Stickstoff ausgewählt sind, wahlweise substituiert mit bis zu zwei Substituenten, die unabhängig voneinander aus Oxo, C₁₋₆-Alkyl, -(CH₂)ₚ-Phenyl, -OR⁷, -(CH₂)ₚCO₂R⁷, -NR⁷R⁸ und -CONR⁷R⁸ ausgewählt sind, und der Heteroarylring wahlweise weiter mit einem Substituenten, ausgewählt aus -OR⁷, Halogen, Trifluormethyl, -CN, -CO₂R⁷ und C₁₋₆-Alkyl, wahlweise substituiert mit Hydroxy, substituiert ist; oder
A ein kondensierter 5-gliedriger Heteroarylring mit einem oder zwei Heteroatomen ist, die unabhängig voneinander aus Sauerstoff und Stickstoff ausgewählt sind, wobei der Heteroarylring mit -(CH₂)_{q}-Aryl oder -(CH₂)_{q}-Heteroaryl substituiert ist, wobei das Aryl oder Heteroaryl wahlweise mit einem oder mehreren Substituenten substituiert ist, die unabhängig voneinander aus Oxo, C₁₋₆-Alkyl, Halogen, Cyano, Trifluormethyl, -OR⁹, -(CH₂)ᵣCO₂R¹⁰, -NR⁹R¹⁰, -(CH₂)ᵣCONR⁹R¹⁰, -NHCOR⁹, -SO₂NR⁹R¹⁰, -NHSO₂R⁹ und -S (O) ₛR⁹ ausgewählt sind, und
der Heteroarylring wahlweise weiter mit einem Substituenten, ausgewählt aus -OR⁷, Halogen, Trifluormethyl, -CN, -C_{2O}R⁷ und C₁₋₆-Alkyl, das wahlweise mit Hydroxy substituiert ist, substituiert ist;
R¹ aus Methyl und Chlor ausgewählt ist;
R² aus -NH-CO-R¹¹ und -CO-NH-(CH₂)ₜ-R¹² ausgewählt ist;
R³ aus Wasserstoff, C₁₋₆-Alkyl, wahlweise substituiert mit bis zu zwei OH-Gruppen, -(CH₂)ₖ-C₃₋₇-Cycloalkyl, - (CH₂)ₖ-Phenyl, wahlweise substituiert mit R¹³ und/oder R¹⁴, und -(CH₂)ₖ-Heteroaryl, wahlweise substituiert mit R¹³ und/oder R¹⁴, ausgewählt ist;
R⁴ aus Wasserstoff und C₁₋₆-Alkyl ausgewählt ist oder
R³ und R⁴ zusammen mit dem Stickstoffatom, an das sie gebunden sind, einen 5- oder 6-gliedrigen heterocyclischen Ring mit wahlweise einem zusätzlichen Heteroatom, ausgewählt aus Sauerstoff, Schwefel und N-R¹⁵, bilden;
R⁵ aus C₁₋₆-Alkyl, wahlweise mit bis zu drei Halogenatomen substituiert, C₂₋₆-Alkenyl, wahlweise substituiert mit Phenyl, C₃₋₇-Cycloalkyl, Heteroaryl, wahlweise substituiert mit bis zu drei R¹³- und/oder R¹⁴-Gruppen, und Phenyl, wahlweise substituiert mit R¹³ und/oder R¹⁴, ausgewählt ist;
R⁶ eine C₃₋₆-Alkylgruppe ist, substituiert mit mindestens zwei Substituenten, die unabhängig voneinander ausgewählt sind aus -OR¹⁶, -NR¹⁶R¹⁷, -CO₂R¹⁶, -CONR¹⁶R¹⁷, -NHCOR¹⁶ und -NHSO₂R¹⁶;
R⁷ und R⁸ jeweils unabhängig voneinander aus Wasserstoff und C₁₋₆-Alkyl ausgewählt sind;
R⁹ aus Wasserstoff, -(CH₂)ᵤ-C₃₋₇-Cycloalkyl, -(CH₂)ᵤ-Heterocyclyl, -(CH₂)ᵤ-Aryl und C₁₋₆-Alkyl, wahlweise substituiert mit bis zu zwei Substituenten, unabhängig voneinander ausgewählt aus -OR¹⁸ und -NR¹⁸R¹⁹, ausgewählt ist;
R¹⁰ aus Wasserstoff und C₁₋₆-Alkyl ausgewählt ist oder
R⁹ und R¹⁰ zusammen mit dem Stickstoffatom, an das sie gebunden sind, einen 5- oder 6-gliedrigen heterocyclischen Ring, wahlweise mit einem zusätzlichen Heteroatom, ausgewählt aus Sauerstoff, Schwefel und N-R¹⁵, bilden;
R¹¹ aus Wasserstoff, C₁₋₆-Alkyl, -(CH₂)ₜ-C₃₋₇-Cycloalkyl, Trifluormethyl, -(CH₂)ᵥ-Heteroaryl, wahlweise substituiert mit R²⁰ und/oder R²¹, und -(CH₂)ᵥ-Phenyl, wahlweise substituiert mit R²⁰ und/oder R²¹, ausgewählt ist;
R¹² aus Wasserstoff, C₁₋₆-Alkyl, C₃₋₇-Cycloalkyl, -CONHR²², Phenyl, wahlweise substituiert mit R²⁰ und/oder R²¹, und Heteroaryl, wahlweise substituiert mit R²⁰ und/oder R²¹, ausgewählt ist;
R¹³ und R¹⁴ jeweils unabhängig voneinander aus Halogen, Cyano, Trifluormethyl, Nitro, C₁₋₆-Alkyl, C₁₋₆-Alkoxy, -CONR²²R²³, -COR²⁴, -CO₂R²⁴ und Heteroaryl ausgewählt sind oder
R¹³ und R¹⁴ unter Bildung eines kondensierten 5-gliedrigen Heterocyclylrings mit einem Heteroatom, ausgewählt aus Sauerstoff, Schwefel und N-R¹⁵, oder eines kondensierten Heteroarylrings verknüpft sind;
R¹⁵ aus Wasserstoff und Methyl ausgewählt ist;
R¹⁶, R¹⁷, R¹⁸ und R¹⁹ jeweils unabhängig voneinander aus Wasserstoff und C₁₋₆-Alkyl ausgewählt sind;
R²⁰ aus C₁₋₆₋Alkyl, C₁₋₆-Alkoxy, -(CH₂)ₜ-C₃₋₇-Cycloalkyl, -CONR²²R²³, -NHCOR²³, Halogen, -CN, -(CH₂)_{w}NR²⁵R²⁶, Trifluormethyl, Phenyl, wahlweise substituiert mit einer oder mehreren R²¹-Gruppen, und Heteroaryl, wahlweise substituiert mit einer oder mehreren R²¹-Gruppen, ausgewählt ist;
R²¹ aus C₁₋₆-Alkyl, C₁₋₆-Alkoxy, Halogen, Trifluormethyl und -(CH₂)_{w}NR²⁵R²⁶ ausgewählt ist;
R²² und R²³ jeweils unabhängig voneinander aus Wasserstoff und C₁₋₆-Alkyl ausgewählt sind oder
R²² und R²³ zusammen mit dem Stickstoffatom, an das sie gebunden sind, einen 5- oder 6-gliedrigen heterocyclischen Ring, wahlweise mit einem zusätzlichen Heteroatom, ausgewählt aus Sauerstoff, Schwefel und N-R¹⁵, bilden, wobei der Ring mit bis zu zwei C₁₋₆-Alkylgruppen substituiert sein kann;
R²⁴ C₁₋₆-Alkyl ist;
R²⁵ aus Wasserstoff, C₁₋₆-Alkyl und -(CH₂)ₜ-C₃₋₇-Cycloalkyl, wahlweise substituiert mit C₁₋₆-Alkyl, ausgewählt ist;
R²⁶ aus Wasserstoff und C₁₋₆-Alkyl ausgewählt ist oder
R²⁵ und R²⁶ zusammen mit dem Stickstoffatom, an das sie gebunden sind, einen 5- oder 6-gliedrigen heterocyclischen Ring, wahlweise mit einem zusätzlichen Heteroatom, ausgewählt aus Sauerstoff, Schwefel und N-R¹⁵, bilden;
B aus einer Bindung, Sauerstoff, NH und S(O)ₓ ausgewählt ist;
X und Y jeweils unabhängig voneinander aus Wasserstoff, Methyl und Halogen ausgewählt sind;
Z¹ N oder N=O ist und Z² CH ist,
Z¹ CH ist und Z² N oder N=O ist oder
Z¹ und Z² jeweils unabhängig voneinander aus N oder N=O ausgewählt sind;
k, m und w jeweils unabhängig voneinander aus 0, 1, 2 und 3 ausgewählt sind;
n, q, r, s, t und x jeweils unabhängig voneinander aus 0, 1 und 2 ausgewählt sind; und
u und v jeweils unabhängig voneinander aus 0 und 1 ausgewählt sind;
oder ein pharmazeutisch akzeptables Salz oder Solvat davon.

2. Verbindung gemäss Anspruch 1, worin A ein 5-gliedriger Heteroarylring mit zwei Heteroatomen, unabhängig voneinander ausgewählt aus Sauerstoff und Stickstoff, ist.

3. Verbindung gemäss Anspruch 1 oder Anspruch 2, worin A mit bis zu zwei Substituenten, unabhängig voneinander ausgewählt aus C₁₋₄-Alkyl, Halogen, -(CH₂) ₖNR³R⁴, - (CH₂) ₖNHCOR³, - (CH₂) ₖNHSO₂R³ und - (CH₂)ₖSO₂(CH₂)ₘR⁵, substituiert ist oder A mit -(CH₂)_{q}-Aryl substituiert ist, wobei das Aryl wahlweise mit einem oder zwei Substituenten, unabhängig voneinander ausgewählt aus C₁₋₆-Alkyl, Halogen, Cyano, -OR⁹ und -(CH₂)ᵣCO₂R¹⁰, substituiert ist.

4. Verbindung gemäss einem der vorhergehenden Ansprüche, worin A mit - (CH₂)ₖSO₂(CH²)ₘR⁵ oder - (CH₂)_{q}-Aryl substituiert ist, wobei das Aryl mit C₁₋₆-Alkyl oder Halogen substituiert ist.

5. Verbindung gemäss einem der vorhergehenden Ansprüche, worin R¹ Methyl ist.

6. Verbindung gemäss einem der vorhergehenden Ansprüche, worin R² -CO-NH-(CH₂)ₜ-R¹² ist.

7. Verbindung gemäss einem der vorhergehenden Ansprüche, worin X Wasserstoff oder Fluor ist.

8. Verbindung gemäss Anspruch 1, ausgewählt aus:
N-Cyclopropyl-4-methyl-3-{1-[(1-methylethyl)-sulfonyl]-1H-pyrazolo[3,4-c]pyridin-5-yl}benzamid;
N-Cyclopropyl-4-methyl-5-[1-(2-thienylsulfonyl)-1H-pyrazolo[3,4-c]pyridin-5-yl]benzamid;
N-Cyclopropyl-3-fluor-4-methyl-5-[1-(2-thienyl-sulfonyl)-1H-pyrazolo[3,4-c]pyridin-5-yl]benzamid;
N-Cyclopropyl-3-[1-(cyclopropylsulfonyl)-1H-pyrazolo-[3,4-c]pyridin-5-yl]-5-fluor-4-methylbenzamid;
N-Cyclopropyl-3-fluor-4-methyl-5-[1-(3-methylphenyl)-1H-pyrazolo[3,4-c]pyridin-5-yl]benzamid;
N-Cyclopropyl-4-methyl-5-(1-phenyl-1H-pyrazolo-[3,4-c]pyridin-5-yl)benzamid;
N-Cyclopropyl-3-[1-(2-fluorphenyl)-1H-pyrazolo-[3,4-c]pyridin-5-yl]-4-methylbenzamid;
N-Cyclopropyl-3-fluor-5-[3-(4-fluorphenyl)-1H-pyrazolo[3,4-b]pyridin-6-yl]-4-methylbenzamid;
3-Fluor-5-[3-(4-fluorphenyl)-1H-pyrazolo[3,4-b]-pyridin-6-yl]-4-methyl-N-(1-methyl-1H-pyrazolo-5-yl)benzamid;
3-Fluor-5-[3-(4-fluorphenyl)-1H-pyrazolo[4,3-c]-pyridin-6-yl]-4-methyl-N-(1-methyl-1H-pyrazolo-5-yl)benzamid;
3-[3-(Acetylamino)-1H-pyrazolo[3,4-b]pyridin-6-yl]-N-cyclopropyl-4-methylbenzamid;
N-Cyclopropyl-4-methyl-3-{3-[(2-methylpropanoyl)-amino]-1H-pyrazolo[3,4-b]pyridin-6-yl}benzamid;
N-Cyclopropyl-4-methyl-3-[3-(propanoylamino)-1H-pyrazolo[3,4-b]pyridin-6-yl]benzamid; und N-(6-{5-[(Cyclopropylamino)carbonyl]-2-methylphenyl}-1H-pyrazolo[3,4-b]pyridin-3-yl)-2-thiophencarboxamid;
oder ein pharmazeutisch akzeptables Salz oder Solvat davon.

9. Pharmazeutische Zusammensetzung, umfassend mindestens eine Verbindung gemäss einem der Ansprüche 1 bis 8 oder ein pharmazeutisch akzeptables Salz oder Solvat davon in Verbindung mit einem oder mehreren pharmazeutisch akzeptablen Hilfsstoff(en), Verdünnungsmittel (n) und/oder Träger(n).

10. Verbindung gemäss einem der Ansprüche 1 bis 8 oder ein pharmazeutisch akzeptables Salz oder Solvat davon zur therapeutischen Anwendung.

11. Verbindung gemäss einem der Ansprüche 1 bis 8 oder ein pharmazeutisch akzeptables Salz oder Solvat davon zur Verwendung bei der Behandlung oder Prophylaxe eines Leidens oder Krankheitszustands, vermittelt durch p38-Kinaseaktivität oder vermittelt durch Cytokine, die durch die Aktivität der p38-Kinase produziert werden.

12. Verwendung einer Verbindung gemäss einem der Ansprüche 1 bis 8 oder eines pharmazeutisch akzeptablen Salzes oder Solvats davon zur Herstellung eines Arzneimittels zur Verwendung bei der Behandlung eines Leidens oder eines Krankheitszustands, vermittelt durch p38-Kinaseaktivität oder vermittelt durch Cytokine, die durch die Aktivität der p38-Kinase produziert werden.

13. Verfahren zur Herstellung einer Verbindung der Formel (I) gemäss einem der Ansprüche 1 bis 8 oder eines pharmazeutisch akzeptablen Salzes oder Solvats davon, das umfasst:
(a) Umsetzen einer Verbindung der Formel (II): 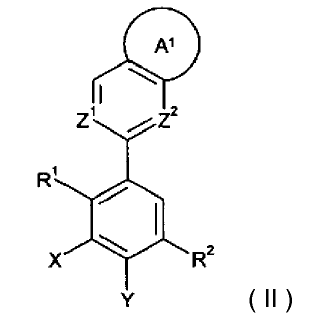 worin R¹, R², X, Y, Z¹ und Z² wie in Anspruch 1 definiert sind und A¹ ein unsubstituierter, kondensierter, 5-gliedriger Heteroarylring ist, der ein oder zwei Heteroatome, unabhängig voneinander ausgewählt aus Sauerstoff und Stickstoff, enthält, mit einem Halogenidderivat,
in Gegenwart einer Base;
(b) wenn A kondensiertes Pyrazolyl ist, Umsetzen einer Verbindung der Formel (XI): 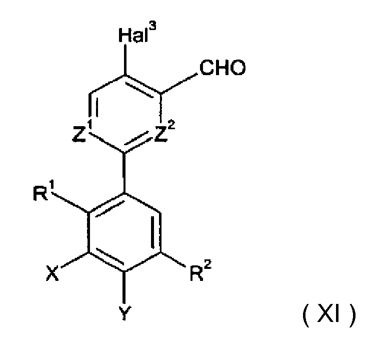 worin R¹, R², X, Y, Z¹ und Z² wie zuvor definiert sind und Hal³ Halogen, insbesondere Chlor, ist, mit einem Hydrazinderivat;
(c) wenn A kondensiertes Pyrazolyl, substituiert mit Aryl, ist, Umsetzen einer Verbindung der Formel (XII) : 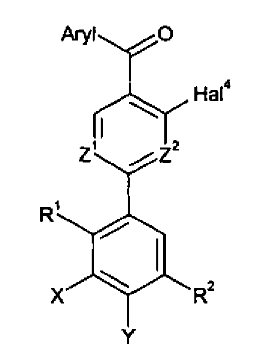 worin R¹, R², X, Y, Z¹ und Z² wie vorstehend definiert sind und Hal⁴ Halogen, insbesondere Chlor, ist, mit einem Hydrazinderivat; oder
(d) als Endstufe Modifizieren einer Verbindung der Formel (I) wie in Anspruch 1 definiert, um eine andere Verbindung der Formel (I), wie in Anspruch 1 definiert, zu erhalten.

## Revendications

1. Composé de formule (I) : 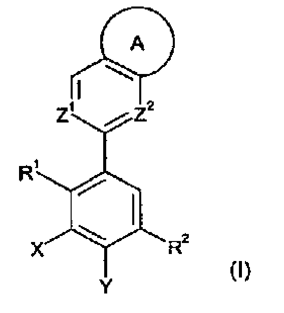 dans laquelle
A est un cycle hétéroaryle à 5 chaînons condensé contenant un ou deux hétéroatomes indépendamment choisis parmi l'oxygène et l'azote, lequel cycle hétéroaryle est facultativement substitué par jusqu'à deux substituants indépendamment choisis parmi un groupe alkyle en C_{1 à 6}, - (CH₂)ₖ-cycloalkyle en C_{3 à 7}, un atome d'halogène, un groupe cyano, trifluorométhyle, - (CH₂)ₖOR³, - (CH₂)ₖCO₂R³, - (CH₂)ₖNR³R⁴, - (CH₂)ₖCONR³R⁴, - (CH₂)ₖNHCOR³, - (CH₂)ₖSO₂NR³R⁴, - (CH₂)ₖNHSO₂R³, - (CH₂)ₖSO₂(CH₂)ₘR⁵, un cycle hétérocyclyle à 5 ou 6 chaînons contenant de l'azote facultativement substitué par un groupe alkyle en C_{1 à 2} ou - (CH₂) ₖCO₂R³, et un cycle hétéroaryle à 5 chaînons facultativement substitué par un groupe alkyle en C_{1 à 2} ;
A est un cycle hétéroaryle à 5 chaînons condensé contenant un ou deux hétéroatomes indépendamment choisis parmi l'oxygène et l'azote, lequel cycle hétéroaryle est substitué par -BR⁶, et
le cycle hétéroaryle est en outre facultativement substitué par un substituant choisi parmi -OR⁷, un atome d'halogène, un groupe trifluorométhyle, -CN, -CO₂R⁷ et alkyle en C_{1 à 6} facultativement substitué par un groupe hydroxy ;
A est un cycle hétéroaryle à 5 chaînons condensé contenant un ou deux hétéroatomes indépendamment choisis parmi l'oxygène et l'azote, lequel cycle hétéroaryle est substitué par un groupe - (CH₂)ₙ hétérocyclyle dans lequel l'hétérocyclyle est un cycle hétérocyclique à 5 ou 6 chaînons contenant un ou deux hétéroatomes indépendamment choisis parmi l'oxygène, le soufre et l'azote, facultativement substitué par jusqu'à deux substituants indépendamment choisis parmi un groupe oxo, alkyle en C_{1 à 6}, -(CH₂)ₚ phényle, -OR⁷, -(CH₂)ₚCO₂R⁷, -NR⁷R⁸ et -CONR⁷R⁸, et
le cycle hétéroaryle est en outre facultativement substitué par un substituant choisi parmi -OR⁷, un atome d'halogène, un groupe trifluorométhyle, -CN, -CO₂R⁷ et alkyle en C_{1 à 6} facultativement substitué par un groupe hydroxy ; ou
A est un cycle hétéroaryle à 5 chaînons condensé contenant un ou deux hétéroatomes indépendamment choisis parmi l'oxygène et l'azote, lequel cycle hétéroaryle est substitué par -(CH₂)_{q}aryle ou - (CH₂)_{q}hétéroaryle où le groupe aryle ou hétéroaryle est facultativement substitué par un ou plusieurs substituants indépendamment choisis parmi un groupe oxo, alkyle en C_{1 à 6}, un atome d'halogène, un groupe cyano, trifluorométhyle, -OR⁹, - (CH₂)ᵣCO₂R¹⁰, -NR⁹R¹⁰, - (CH₂)ᵣCONR⁹R¹⁰, -NHCOR⁹, -SO₂NR⁹R¹⁰, -NHSO₂R⁹ et -S(O)ₛR⁹, et
le cycle hétéroaryle est en outre facultativement substitué par un substituant choisi parmi -OR⁷, un atome d'halogène, un groupe trifluorométhyle, -CN, -CO₂R⁷ et alkyle en C_{1 à 6} facultativement substitué par un groupe hydroxy ;
R¹ est choisi parmi les groupes méthyle et chloro ;
R² est choisi parmi les groupes -NH-CO-R¹¹ et -CO-NH- (CH₂)ₜ-R¹² ;
R³ est choisi parmi un atome d'hydrogène, un groupe alkyle en C_{1 à 6} facultativement substitué par jusqu'à deux groupes OH, un groupe -(CH₂)ₖ-Cycloalkyle en C_{3 à 7}, - (CH₂)ₖphényle facultativement substitué par R¹³ et/ou R¹⁴ et - (CH₂)ₖhétéroaryle facultativement substitué par R¹³ et/ou R¹⁴,
R⁴ est choisi parmi un atome d'hydrogène et un groupe alkyle en C_{1 à 6}, ou
R³ et R⁴ conjointement avec l'atome d'azote auquel ils sont liés, forment un cycle hétérocyclique à 5 ou 6 chaînons contenant facultativement un hétéroatome additionnel choisi parmi l'oxygène, le soufre et N-R¹⁵ ;
R⁵ est choisi parmi un groupe alkyle en C_{1 à 6} facultativement substitué par jusqu'à trois atomes d'halogène, alcényle en C_{2 à 6} facultativement substitué par un groupe phényle, cycloalkyle en C_{3 à 7}, hétéroaryle facultativement substitué par jusqu'à trois groupes R¹³ et/ou R¹⁴, et phényle facultativement substitué par R¹³ et/ou R¹⁴ ;
R⁶ est un groupe alkyle en C_{3 à 6} substitué par au moins deux substituants indépendamment choisis parmi -OR¹⁶, -NR¹⁶R¹⁷, -CO₂R¹⁶, -CONR¹⁶R¹⁷, -NHCOR¹⁶ et -NHSO₂R¹⁶ ;
R⁷ et R⁸ sont chacun indépendamment choisis parmi un atome d'hydrogène et un groupe alkyle en C _{1 à 6} ;
R⁹ est choisi parmi un atome d'hydrogène, un groupe - (CH₂)ᵤ-Cycloalkyle en C_{3 à 7}, - (CH₂)ᵤhétérocyclyle, - (CH₂)ᵤaryle et alkyle en C_{1 à 6} facultativement substitué par jusqu'à deux substituants indépendamment choisis parmi -OR¹⁸ et -NR¹⁸R¹⁹,
R¹⁰ est choisi parmi un atome d'hydrogène et un groupe alkyle en C_{1 à 6}, ou
R⁹ et R¹⁰, conjointement avec l'atome d'azote auquel ils sont liés, forment un cycle hétérocyclique à 5 ou 6 chaînons contenant facultativement un hétéroatome additionnel choisi parmi l'oxygène, le soufre et N-R¹⁵ ;
R¹¹ est choisi parmi un atome d'hydrogène, un groupe alkyle en C_{1 à 6}, - (CH₂)ₜ-cycloalkyle en C_{3 à} **₇,** trifluorométhyle, - (CH₂)ᵥhétéroaryle facultativement substitué par R²⁰ et/ou R²¹, et - (CH₂)ᵥphényle facultativement substitué par R²⁰ et/ou R²¹ ;
R¹² est choisi parmi un atome d'hydrogène, un groupe alkyle en C_{1 à 6,} cycloalkyle en C_{3 à 7}, -CONHR²², phényle facultativement substitué par R²⁰ et/ou R²¹, et hétéroaryle facultativement substitué par R²⁰ et/ou R²¹ ;
R¹³ et R¹⁴ sont chacun indépendamment choisis parmi un atome d'halogène, un groupe cyano, trifluorométhyle, nitro, alkyle en C_{1 à 6}, alcoxy en C_{1 à 6}, -CONR²²R²³, COR²⁴, -CO₂R²⁴ et hétéroaryle, ou
R¹³ et R¹⁴ sont liés pour former un cycle hétérocyclyle à 5 chaînons condensé contenant un hétéroatome choisi parmi l'oxygène, le soufre et N-R¹⁵, ou un cycle hétéroaryle condensé ;
R¹⁵ est choisi parmi un atome d'hydrogène et un groupe méthyle ;
R¹⁶, R¹⁷, R¹⁸ et R¹⁹ sont chacun indépendamment choisis parmi un atome d'hydrogène et un groupe alkyle en C_{1 à 6} ;
R²⁰ est choisi parmi un groupe alkyle en C_{1 à 6}, alcoxy en C_{1 à 6}, - (CH₂)ₜ-cycloalkyle en C_{3 à 7}, -CONR²²R²³, -NHCOR²³, un atome d'halogène, un groupe -CN, - (CH₂)_{w}NR²⁵R²⁶, trifluorométhyle, phényle facultativement substitué par un ou plusieurs groupes R²¹, et hétéroaryle facultativement substitué par un ou plusieurs groupes R²¹ ;
R²¹ est choisi parmi un groupe alkyle en C_{1 à 6,} alcoxy en C_{1 à 6}, un atome d'halogène, un groupe trifluorométhyle et -(CH₂)_{w}NR²⁵R²⁶ ;
R²² et R²³ sont chacun indépendamment choisis parmi un atome d'hydrogène et un groupe alkyle en C_{1 à 6}, ou
R²² et R²³, conjointement avec l'atome d'azote auquel ils sont liés, forment un cycle hétérocyclique à 5 ou 6 chaînons contenant facultativement un hétéroatome additionnel choisi parmi un atome d'oxygène, de soufre et N-R¹⁵, où le cycle peut être substitué par jusqu'à deux groupes alkyle en C _{1 à 6} ;
R²⁴ est un groupe alkyle en C_{1 à 6} ;
R²⁵ est choisi parmi un atome d'hydrogène, un groupe alkyle en C_{1 à 6} et - (CH₂)ₜ-cycloalkyle en C_{3 à 7} facultativement substitué par un groupe alkyle en C_{1 à 6},
R²⁶ est choisi parmi un atome d'hydrogène et un groupe alkyle en C_{1 à 6}, ou
R²⁵ et R²⁶, conjointement avec l'atome d'azote auquel ils sont liés, forment un cycle hétérocyclique à 5 ou 6 chaînons contenant facultativement un hétéroatome additionnel choisi parmi l'oxygène, le soufre et N-R¹⁵ ;
B est choisi parmi une liaison, un atome d'oxygène, NH et S (O)ₓ ;
X et Y sont chacun indépendamment choisis parmi un atome d'hydrogène, un groupe méthyle et un atome d'halogène ;
Z¹ est N ou N=O et Z² est CH,
Z¹ est CH et Z² est N ou N=O, ou
Z¹ et Z² sont chacun indépendamment choisis parmi N ou N=O ;
k, m et w sont chacun indépendamment choisis parmi 0, 1, 2 et 3 ;
n, q, r, s, t et x sont chacun indépendamment choisis parmi 0, 1 et 2 ; et
u et v sont chacun indépendamment choisis parmi 0 et 1 ;
ou sel ou solvate pharmaceutiquement acceptable de celui-ci.

2. Composé selon la revendication 1, dans lequel A est un cycle hétéroaryle à 5 chaînons contenant deux hétéroatomes indépendamment choisis parmi l'oxygène et l'azote.

3. Composé selon la revendication 1 ou 2, dans lequel A est substitué par jusqu'à deux substituants indépendamment choisis parmi un groupe alkyle en C_{1 à 4}, un atome d'halogène, - (CH₂)ₖNR³R⁴, - (CH₂) ₖNHCOR³, - (CH₂)ₖNHS₂OR³ et - (CH₂)ₖSO₂ (CH₂)ₘR⁵, ou A est substitué par un groupe - (CH₂)_{q}aryle dans lequel le groupe aryle est facultativement substitué par un ou deux substituants indépendamment choisis parmi un groupe alkyle en C_{1 à 6}, un atome d'halogène, un groupe cyano, -OR⁹ et - (CH2)ᵣCO₂R¹⁰.

4. Composé selon l'une quelconque des revendications précédentes, dans lequel A est substitué par un groupe -(CH₂)ₖSO₂(CH₂)ₘR⁵ ou - (CH₂) _{q}aryle dans lequel l'aryle est substitué par un groupe alkyle en C_{1 à 6} ou un atome d'halogène.

5. Composé selon l'une quelconque des revendications précédentes, dans lequel R¹ est un groupe méthyle.

6. Composé selon l'une quelconque des revendications précédentes, dans lequel R² est -CO-NH-(CH₂)ₜ-R¹².

7. Composé selon l'une quelconque des revendications précédentes, dans lequel X est un atome d'oxygène ou de fluor.

8. Composé selon la revendication 1 choisi parmi :
le *N-*cyclopropyl-4-méthyl-3-{1-[(1-méthyléthyl)sulfonyl]-1*H-*pyrazolo[3,4-c]pyridin-5-yl}benzamide ;
le *N-*cyclopropyl-4-méthyl-5-[1-(2-thiénylsulfonyl)-1*H-*pyrazolo[3,4-c]pyridin-5-yl]benzamide ;
le *N*-cyclopropyl-3-fluoro-4-méthyl-5-[1-(2-thiénylsulfonyl)-1*H-*pyrazolo[3,4-c]pyridin-5-yl]benzamide ;
le *N*-cyclopropyl-3-[1-(cyclopropylsulfonyl)-1*H-*pyrazolo[3,4-c]pyridin-5-yl]-5-fluoro-4-méthylbenzamide ;
le *N*-cyclopropyl-3-fluoro-4-méthyl-5-[1-(3-méthylphényl)-1*H*-pyrazolo[3,4-c]pyridin-5-yl]benzamide ;
le *N*-cyclopropyl-4-méthyl-5-(1-phényl-1*H-*pyrazolo[3,4-c]pyridin-5-yl)benzamide ;
le *N*-cyclopropyl-3-[1-(2-fluorophényl)-1*H-*pyrazolo[3,4-c]pyridin-5-yl]-4-méthylbenzamide ;
le *N*-cyclopropyl-3-fluoro-5-[3-(4-fluorophényl)-1*H*-pyrazolo[3,4-b]pyridin-6-yl]-4-méthylbenzamide ;
le 3-fluoro-5-[3-(4-fluorophényl)-1*H*-pyrazolo[3,4-b]pyridin-6-yl]-4-méthyl-*N-*(1-méthyl-1*H-*pyrazol-5-yl)benzamide ;
le 3-fluoro-5-[3-(4-fluorophényl)-1*H-*pyrazolo[4,3-c]pyridin-6-yl]-4-méthyl-*N-*(1-méthyl-1*H-*pyrazol-5-yl)benzamide ;
le 3-[3-(acétylamino)-1*H-*pyrazolo[3,4-b]pyridin-6-yl]-*N-*cyclopropyl-4-méthylbenzamide ;
le *N-*cyclopropyl-4-méthyl-3-{3-[(2-méthylpropanoyl)amino]-1*H-*pyrazolo[3,4-b]pyridin-6-yl}benzamide ;
le *N-*cyclopropyl-4-méthyl-3-[3-(propanoylamino)-1*H-*pyrazolo[3,4-b]pyridin-6-yl]benzamide ; et
le *N-*(6-{5-[(cyclopropylamino)carbonyl]-2-méthylphényl}-1*H-*pyrazolo[3,4-b]pyridin-3-yl)-2-thiophènecarboxamide ;
ou sel ou solvate pharmaceutiquement acceptable de celui-ci.

9. Composition pharmaceutique comprenant au moins un composé tel que revendiqué dans l'une quelconque des revendications 1 à 8, ou un sel ou solvate pharmaceutiquement acceptable de celui-ci, en association avec un ou plusieurs excipients, diluants et/ou supports pharmaceutiquement acceptables.

10. Composé selon l'une quelconque des revendications 1 à 8, ou sel ou solvate pharmaceutiquement acceptable de celui-ci, à utiliser en thérapie.

11. Composé selon l'une quelconque des revendications 1 à 8, ou sel ou solvate pharmaceutiquement acceptable de celui-ci, à utiliser dans le traitement ou la prophylaxie d'une affection ou d'un état pathologique véhiculé par une activité de kinase p38 ou véhiculé par des cytokines produites par l'activité de kinase p38.

12. Utilisation d'un composé tel que revendiqué dans l'une quelconque des revendications 1 à 8, ou d'un sel ou solvate pharmaceutiquement acceptable de celui-ci, dans la fabrication d'un médicament à utiliser dans le traitement d'une affection ou d'un état pathologique véhiculé par une activité de kinase p38 ou véhiculé par des cytokines produites par l'activité de kinase p38.

13. Procédé de préparation d'un composé de formule (I) tel que revendiqué dans l'une quelconque des revendications 1 à 8, ou d'un sel ou solvate pharmaceutiquement acceptable de celui-ci, qui comprend
(a) la réaction d'un composé de formule (II) 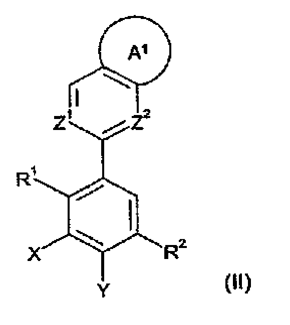 dans laquelle R¹, R², X, Y, Z¹ et Z² sont tels que définis dans la revendication 1 et A¹ est un cycle hétéroaryle à 5 chaînons condensé non substitué contenant un ou deux hétéroatomes indépendamment choisis parmi l'oxygène et l'azote avec un dérivé halogénure,
en présence d'une base ;
(b) lorsque A est un groupe pyrazolyle condensé, la réaction d'un composé de formule (XI) 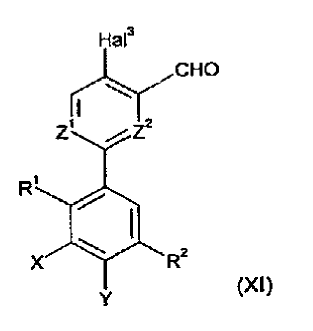 dans laquelle R¹, R², X, Y, Z¹ et Z² sont tels que définis ci-avant et Hal³ est un atome d'halogène, en particulier le chlore, avec un dérivé hydrazine ;
(c) lorsque A est un groupe pyrazolyle condensé substitué par un groupe aryle, la réaction d'un composé de formule (XII) 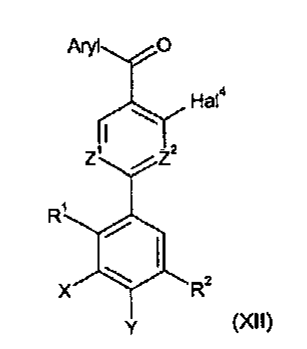 dans laquelle R¹, R², X, Y, Z¹ et Z² sont tels que définis ci-avant et Hal⁴ est un atome d'halogène, en particulier le chlore, avec un dérivé hydrazine ; ou
(d) la modification d'étage final d'un composé de formule (I) tel que défini dans la revendication 1 pour donner un autre composé de formule (I) tel que défini dans la revendication 1.
